(19) Europäisches Patentamt
European Patent Office
Office européen des brevets

(11) EP 1 672 626 A1

(12) # EUROPEAN PATENT APPLICATION
published in accordance with Art. 158(3) EPC

(43) Date of publication:
**21.06.2006 Bulletin 2006/25**

(21) Application number: **04792237.2**

(22) Date of filing: **08.10.2004**

(51) Int Cl.:
***G11B 7/24*** *(1968.09)* ***B41M 5/26*** *(1968.09)*

(86) International application number:
**PCT/JP2004/014969**

(87) International publication number:
**WO 2005/036541 (21.04.2005 Gazette 2005/16)**

(84) Designated Contracting States:
**DE FR GB**

(30) Priority:
**10.10.2003 JP 2003351904**
**10.10.2003 JP 2003351905**
**06.02.2004 JP 2004031199**
**06.02.2004 JP 2004031200**
**30.04.2004 JP 2004136660**
**30.04.2004 JP 2004136659**
**25.06.2004 JP 2004188726**

(71) Applicant: **Mitsui Chemicals, Inc.**
**Tokyo 105-7117 (JP)**

(72) Inventors:
- **Shiozaki, Hiroyoshi,**
**Mitsui Chemicals, Inc.**
**Sodegaura-shi, Chiba 299-0265 (JP)**
- **Miyasato, Masataka,**
**Mitsui Chemicals, Inc.**
**Sodegaura-shi, Chiba 299-0265 (JP)**
- **Ishida, Tsutomu,**
**Mitsui Chemicals, Inc.**
**Sodegaura-shi, Chiba 299-0265 (JP)**
- **Ogiso, Akira,**
**Mitsui Chemicals, Inc.**
**Sodegaura-shi, Chiba 299-0265 (JP)**
- **Ueno, Keiji,**
**Mitsui Chemicals, Inc.**
**Sodegaura-shi, Chiba 299-0265 (JP)**
- **Usui, Hideo,**
**Mitsui Chemicals, Inc.**
**Sodegaura-shi, Chiba 299-0265 (JP)**

(74) Representative: **Stuart, Ian Alexander**
**Mewburn Ellis LLP**
**York House**
**23 Kingsway**
**London WC2B 6HP (GB)**

(54) **OPTICAL RECORDING MEDIUM AND COMPOUND USED IN THE OPTICAL RECORDING MEDIUM**

(57) There are provided an optical recording medium which enables recording and playback with high quality using laser of 300 nm to 900 nm and a novel compound.

An optical recording medium wherein the percentage change ($|[a^2 - a^1]/a^1| \times 100$) of the recording layer thickness ($a^2$) at the recorded site of recording layer (A) after recorded with laser light compared with the recording layer thickness ($a^1$) at unrecorded site of said recording layer (A) is less than 25% and the amount of change ($|a^2 - a^1|$) of the recording layer thickness ($a^2$) at recorded site of said recording layer (A) after recorded with laser light compared with the recording layer thickness ($a^1$) at unrecorded site of said recording layer (A) is controlled to be less than 15 nm.

A compound comprising a six-membered ring structure composed of four carbon atoms and two nitrogen atoms and a substituted or unsubstituted amino group bonded.

Fig.2

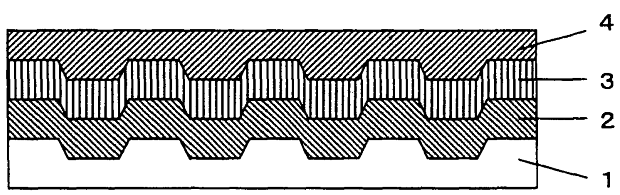


EP 1 672 626 A1

## Description

TECHNICAL FIELD

The present invention relates to an optical recording medium using an organic compound as material for a recording layer, and specifically it relates to a recordable optical recording medium capable of high-density recording, wherein recording/playback can be carried out with a blue-type laser such as a blue-violet laser, which is a kind of visible light laser, and to a novel compound. In addition, the invention relates to material for the recording layer that enables manufacturing of recording layer by coating method such as industrially favored spin-coating method.

BACKGROUND ART

As an optical recording medium compatible with the compact disc (hereafter abbreviated as CD) standard, CD-R (CD-Recordable) has been widely spread. The memory capacity of CD-R is about 680 MB, but there has been increasing demand for recording media with higher density and larger capacity, as the amount of information greatly increases.

A means for achieving higher density of recording media includes using a shorter-wavelength laser for recording/playback and downsizing of beam spot by increasing the numerical aperture (N.A.) of an object lens. And, as a short-wavelength laser used for an optical disc system, there has been put to practical use a red laser of 500 nm to 700 nm, furthermore around 630 nm to 690 nm, specifically 680 nm, 670 nm, 660 nm, 650 nm, 635 nm or the like. Thus, owing to shortening of wavelength of semiconductor lasers, increase in numerical aperture of an object lens, data compression technique and the like, it has become possible to manufacture an optical recording medium on which moving pictures and massive information can be recorded. Optical recording media proposed so far include magneto-optic recording medium, phase-change optical recording medium, chalcogen oxide optical recording medium, organic dye optical recording medium and the like. Among these, organic dye optical recording medium seems advantageous in that it is cheap and easily processed. In such circumstances, it is a recordable digital versatile disc (hereafter abbreviated as DVD-R) that has been developed as an optical recording medium wherein the recording density is higher than that of CD, moving pictures can be recorded and playbacked with quality comparable with TV, playback can be carried out on a widely spreading commercially available DVD video player or DVD-ROM player, and recording can be carried out with a red semiconductor laser having an wavelength of 630 nm to 690 nm. DVD-R is a once-recordable optical recording medium with a recording capacity of 3. 9 GB or 4.7 GB. Particularly very recently, DVD- R medium with recording capacity of 4.7 GB per side has come to be supplied in market. Such DVD-R medium also adopts a stacked structure comprising a recording layer which contains cyanine dye, azo dye or the like and a reflection layer, and is configured as disc with two sheets of substrate of 0. 6 mm in thickness adhered together. Presently, for an optical disc with good recording characteristics and satisfying the above-mentioned recording capacity, development of media capable of high-speed recording has actively been pursued.

Predictably, in the future, higher recording density will be desired and the amount of information will reach 15-30 GB per disc. As a means for achieving such recording density, it is inevitable to use shorter-wavelength lasers. Therefore, dyes with good recording characteristics in a wavelength range of 300 nm to 500 nm are desired as organic dyes used for optical recording media in the future.

For an optical recording medium with recording density higher than that of DVD-R comprising a recording layer having an organic dye, there is disclosed in Patent Document 1 that a recording density corresponding to a recording capacity of 8 GB or higher was attained by using a laser having an wavelength of 680 nm or less. According to the proposal in thisdocument, a large recording capacity of 8 GB or more is attained by converging laser light shorter than 680 nm through light transmission layer of 10 μm to 177 μm in thickness with an object lens with a high value of the numerical aperture of 0.7 or higher.

Meanwhile, in these years, as blue lasers having an wavelength of 390 nm to 430 nm, there have been developed a 410 nm-laser using GaN-based material and an SHG laser of 425 nm that combines a semiconductor laser and an optical waveguide element. Dyes sensitive for blue semiconductor lasers corresponding to these lasers are currently being developed.

Moreover, since the beginning of 1999, after a blue-violet GaN semiconductor laser having an wavelength of 400 nm to 410 nm was offered on a trial base (Nichia Chemical Industry), investigation has been begun for a medium (hereafter abbreviated as HD-DVD-R medium) with higher recording density exceeding 15 GB in one side, wherein the image quality is comparable with that of HDTV (high definition television) broadcast and moving pictures of about 2 hr can be recorded. On an HD-DVD-R medium with such high density, moving pictures of quality comparable with the current broadcast of about 6 hr can be recorded, and therefore it attracts attention as new recording medium replacing home-use VTR.

In such circumstances, the unified standard for next generation high-density optical discs "Blu-ray Disc" has

been formulated and announced by nine companies in Japan, Europe and Korea (February 19, 2002). According to this standard, combination of a blue-violet laser and a lens having high numerical aperture of N.A. = 0.85 enables recording and playback repetitively of image data with a maximum of 27 GB on one side of a disc of 12 cm in diameter. By using a recorder compatible with the same standard, HDTV image of 2 hr or longer can be recorded on one disc. This corresponds to a recording period of 13 hr or more for currently broadcasted NTSC-type image data.

The disc compatible with this standard is 1.2 mm in thickness, wherein laser beam is focused on its recording film formed through an optical transmission layer of about 100 μm in thickness, and three types having recording capacities of 23.3, 25 and 27 GB are proposed. Preceding the above-mentioned standard, applicability of organic dyes to recording media which are used with a blue-violet laser and a lens having high numerical aperture of N.A. = 0.85 is described in the some documents. As a recent specific case, for example, there has been reported an optical recording medium on which recording is carried out at recording mark site through change in refractive index of an organic dye itself accompanying its thermal decomposition and pit formation (Oplus E, vol. 25, No. 6, p.652-657, issued in June 2003).

As organic dyes capable of recording with a blue laser of 400 nm to 500 nm, there have been proposed so far, in addition to cyanine dye compounds described in Patent Document 2, porphyrin dyes, polyene dyes, azo dyes, dicyanovinylphenyl compounds, coumarin compounds, pyrimidine compounds, naphthalocyanine compounds, 5-membered heterocyclic compounds, bisazole compounds, aminopyridine compounds, bispyridinium compounds, oxonol compounds, styryl compounds, aminobutadiene compounds, metal chelate compounds, quinone or quinodimethane compounds, hydrazone compounds, triazine compounds, carbostyryl or naphthyridine compounds, fused heterocyclic compounds, stilbene compounds and the like.

Furthermore, there have been proposed an optical recording medium described in Patent Document 3, which contains two layers of a recording layer containing organic dyes such as porphyrin dyes or cyanine dyes as the major component and a metallic reflection layer containing silver as the major component; an optical recording medium whose configuration is itself inventive described in Patent Document 4, wherein recording can be carried out at two wavelength regions by comprising a blue-sensitive dye layer containing cyanine dye sensitive to a blue laser and a red-sensitive dye layer; an optical recording medium containing indigoid dyes described in Patent Document 5, wherein recording can be carried out at two wavelength regions by mixing two kinds of dyes, that is, a dye for a blue laser and a dye for a red laser; an optical recording medium containing a cyanoethene dye described in Patent Document 6; and an optical recording medium containing a squarilium dye described in Patent Document 7.

As examples wherein recording is carried out on organic dye layers with blue-region light of 400 nm to 500 nm, there has been disclosed a proposal that, by mixing with a molecular compound and polymer that coordinates to central metal of a porphyrin compound or with a polymer having a moiety that coordinates to the central metal in its side chain, the Soret band of said porphyrin compound is shifted to the longer wavelength to cause sensitivity to a 488 nm Ar laser, and the film can be formed by spin-coating method to reduce production cost (Patent Documents 8, 9). According to examination by the present inventors, the polyene dye (Patent Documents 10, 11) has low photostability and some technique such as blending a quencher is necessary to put it into practical use.

[Patent Document 1] JP-A H10-302310
[Patent Document 2] WO 01/44374
[Patent Document 3] JP-A H11-53758
[Patent Document 4] JP-A H11-203729
[Patent Document 5] JP-A H11-78239
[Patent Document 6] JP-A H11-105423
[Patent Document 7] JP-A H11-110815
[Patent Document 8] JP-A H7-304256
[Patent Document 9] JP-A H7-304257
[Patent Document 10] JP-A H4-78576
[Patent Document 11] JP-A H4-89279

## DISCLOSURE OF THE INVENTION

In recent circumstances, as a blue-violet semiconductor laser of 400 nm to 410 nm is promised to be put into practical use, large capacity recordable optical recording media have been actively developed. Particularly it is desired to develop dyes having high photoresistance and excellent high-speed recording characteristics.

As a matter of fact, however, the above-mentioned optical recording media are not fully adapted to laser light of 400 nm to 410 nm. Namely, for optical recording media containing the above-mentioned organic dyes, we found problems such that read-out of signals is not satisfactorily carried out because the carrier-to-noise ratio (C/N) is not necessarily at a satisfactory level on playback of recorded signals. It is urgently desired to conquer this problem and to develop an optical recording medium capable of recording and playback at high density with laser light of 400 nm to 410 nm.

As described above, although large-capacity optical recording media using said laser have been enthusiastically

developed and dyes having high lightresistance and excellent high-speed recording characteristics in particular is desired to be developed, the properties of above-mentioned dye compounds are still unsatisfactory as a recording material capable of recording/playback with laser light of said wavelength region, and there is room for improvement at present. Further, in manufacturing of media by coating methods such as spin-coating method by which recording films are easily formed, high solubility of dye compound to a solvent used in coating should be taken into account as one of favorable properties. Also, in general, since increase in recording capacity requires high-density recording, it is inevitable to increase the numerical aperture of an object lens for collimating optical beam used for recording and to use a shorter-wavelength laser in the optical system. However, the minimal beam dimension of collimated optical beam is determined by its diffraction limit. Furthermore, it is important that the recording laser power is as low as possible for high-speed recording characteristic. Namely, it is important to develop highly sensitive dye which enables forming of good recording marks with a recording laser of low energy.

Since recording takes place when the beam intensity exceeds a certain threshold, the recording site smaller than the collimated beam spot is obtained, as shown in Fig. 1(a). Although the beam intensity in neighborhood of this recording site is at the foot of the peak, currently used short-wavelength light promotes photochemical reaction of recording layer even in the neighborhood of the recording site. Particularly, light in the wavelength region of the above-mentioned blue-violet laser readily induces photochemical reactions of organic compounds, which causes a problem that a boundary between recorded site and unrecorded site (an edge) is deteriorated on recording and the signal characteristics become worse. Namely, as shown in Fig. 1(b), a recording information that should be recorded according to a square-wave form [solid line in Fig. 1(b)] becomes a broad waveform [broken line in Fig. 1(b)] due to deterioration of the edge. Furthermore, if playback is carried out with the same blue-violet laser wavelength as that used for recording, irradiation with even feeble light such as playback light promotes photochemical reactions, causing a problem that deterioration is enhanced on every playback. In above-mentioned JP H7-304256 and JP H7-304257, different wavelengths are used for recording and playback; in reality, the playback light has a wavelength longer than that of recording light as countermeasure to the above problem, and consequently demand for high density is not fully satisfied. Moreover, use of different wavelengths for recording and playback requires individual devices for recording and playback or a device equipped with two optical systems and their control unit. Such complication may restrict applicability as an optical recording medium, increase the device size, increase cost or cause poor capability for general use. Furthermore, for optical recording media such as CD-R, wherein on/off of record is switched at a definitive thermal threshold corresponding to change in physical properties such as melting point, sublimation point, phase-transition point or thermal decomposition point of the organic dye film. On the other hand, presence of light-deterioration mode caused by excitation with a blue-violet laser makes the contrast vague. Particularly in a high density recording system wherein fine recording site smaller than the optical beam dimension must be formed, this light-deterioration mode may significantly lower the quality of recorded signal.

Furthermore, on formation of recorded site, it has been revealed that, when excessive deformation is caused by using a recording method based on thermal deformation, namely, recording method by pit-formation, or when contrast has become vague because of the above-mentioned photo-deterioration, unwanted effects appear in radial direction of the optical disc, that is, one track invades or obscures its neighboring recording (or recorded) track, which significantly lowers the quality of recorded signal in the neighboring track. Here, in optical recording media, in particular, wherein high-density and high-speed recording is required, high quality of recorded signals at recorded sites in a plurality of tracks (hereafter abbreviated as multi-track) is essential. In other words, it is necessary to maintain high signal quality in a whole circle of disc. To meet this requirement, when one track is recorded and its neighboring track is recorded after that, the signal quality in the earlier recorded track needs to be kept good without deterioration and the signal quality in the later recorded track also needs to be kept good without deterioration. Accordingly, on formation of recorded site, it is inevitable to develop a recording mode wherein the signal quality in the neighboring track is kept good by suppressing excessive deformation and photo-deterioration mode, and the recorded site thus formed is clearly recognized. For this purpose, it is immediately desired to develop a recording material having high light-resistance, wherein recording can be satisfactorily carried out at a low recording laser power to avoid excessive deformation and contrast is not obscured due to light-deterioration.

In addition, the intensity of signal to be read is important for reliable read-out of the signal, and this point should be fully considered.

An object of the present invention is to provide an optical recording medium containing an optical recording layer suitable to ultra-high density recording on which recording and playback can be well carried out with a laser of 300 nm to 900 nm, particularly a laser of 390 nm to 430 nm, more particularly a blue-violet laser having a wavelength selected from 400 nm to 410 nm. Furthermore, an object of the invention is to provide a novel compound that can be preferably used for said optical recording medium.

Another object of the invention is to provide a recording material with which said recording layer can be produced by coating methods such as industrially favored spin-coating method.

The inventors of the present invention pursued zealous studies to solve the problems described above and

accomplished the present invention. Namely, the present invention relates to:

1. An optical recording medium containing at least one recording layer (A) capable of recording and playback with a laser light, wherein said recording layer (A) contains at least one kind of organic compound, wherein the percentage change ($|[a^2 - a^1]/a^1| \times 100$) of the recording layer thickness ($a^2$) at the recorded site of said recording layer (A) after recorded with a laser light compared with the recording layer thickness ($a^1$) at unrecorded site of said recording layer (A) is less than 25% and the amount of change ($|a^2 - a^1|$) of the recording layer thickness ($a^2$) at recorded site of said recording layer (A) after recorded with laser light compared with the recording layer thickness ($a^1$) at unrecorded site of said recording layer (A) is less than 15 nm.
2. The optical recording medium according to the above-mentioned item 1, wherein said recording layer (A) can be formed by a coating method.
3. The optical recording medium according to the above-mentioned item 2, wherein the organic compound is organic compound (B) that comprises a six-membered ring system composed of four carbon atoms and two nitrogen atoms and a substituted or unsubstituted amino group bonded.
4. The optical recording medium according to any of the above-mentioned items 1 to 3, wherein the recording laser power is 6 mW or lower.
5. The optical recording medium according to the above-mentioned item 3, wherein one of tautomeric forms of organic compound (B) is represented by general formula (0):

[Formula 1]

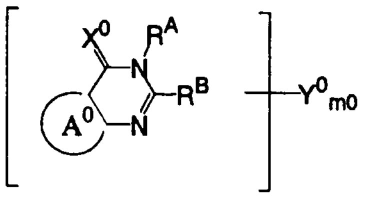

(0)

(wherein ring $A^0$ represents a substituted or unsubstituted carbocyclic aromatic ring or a substituted or unsubstituted heterocyclic aromatic ring; $R^A$ and $R^B$ represent a hydrogen atom or a substituent; $X^0$ represents a divalent substituent; $Y^0$ represents a substituted or unsubstituted amino group; and $m^0$ represents the number of $Y^0$).
6. The optical recording medium according to the above-mentioned item 5, wherein one of the tautomeric forms of organic compound (B) is represented by general formula (1):

[Formula 2]

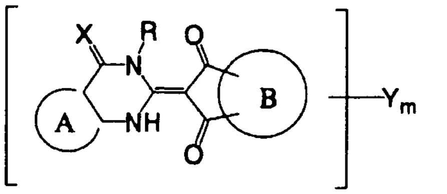

(1)

(wherein ring A and ring B represent a substituted or unsubstituted carbocyclic aromatic ring or a substituted or unsubstituted heterocyclic aromatic ring; R represents a hydrogen atom or a substituent; X represents a divalent substituent; Y represents a substituted or unsubstituted amino group; and m represents the number of Y).
7. The optical recording medium according to the above-mentioned item 6, wherein one of the tautomeric forms of organic compound (B) is represented by general formula (2):

[Formula 3]

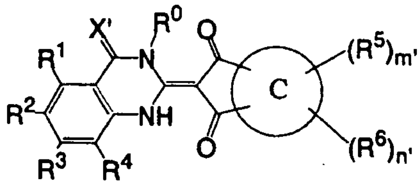

(2)

(wherein ring C represents a substituted or unsubstituted carbocyclic aromatic ring or a substituted or unsubstituted heterocyclic aromatic ring; X' represents a divalent substituent, each of $R^0$-$R^6$ represents independently a hydrogen atom or a substituent; m' represents the number of $R^5$; n' represents the number of $R^6$; at least one group selected from $R^1$-$R^4$ is a substituted or unsubstituted amino group; for a combination among $R^1$-$R^4$ and a combination of $R^5$ and $R^6$, each substituent within each combination may independently bond via a linkage group to form a ring system together with the atom to which it bonds; and each of m' and n' represents 0 or an integer of 1 or more).

8. The optical recording medium according to the above-mentioned item 7, wherein one of the tautomeric forms of organic compound (B) is represented by general formula (3):

[Formula 4]

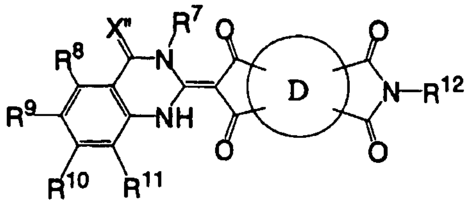

(3)

(wherein ring D represents a substituted or unsubstituted carbocyclic aromatic ring or a substituted or unsubstituted heterocyclic aromatic ring; X" represents a divalent substituent, each of $R^7$-$R^{12}$ represents independently a hydrogen atom or a substituent; at least one group selected from $R^8$-$R^{11}$ is a substituted or unsubstituted amino group; and each of $R^8$-$R^{11}$ may independently bond via a linkage group to form a ring system together with the carbon atom to which it bonds).

9. The optical recording medium according to the above-mentioned item 8, wherein one of the tautomeric forms of organic compound (B) is represented by general formula (4):

[Formula 5]

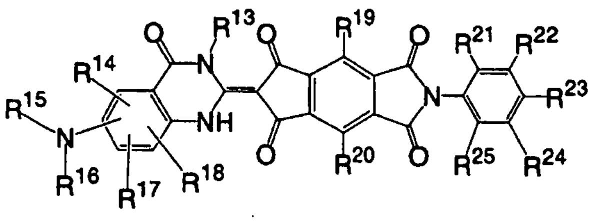

(4)

(wherein each of $R^{13}$-$R^{25}$ represents independently a hydrogen atom or a substituent; and for a combination among $R^{14}$-$R^{18}$ and a combination among $R^{21}$-$R^{25}$, each substituent within each combination may independently bond via a linkage group to form a ring system together with the carbon and/or nitrogen atom to which it bonds).

10. The optical recording medium according to the above-mentioned item 9, wherein one of the tautomeric forms

of organic compound (B) is represented by general formula (5):

[Formula 6]

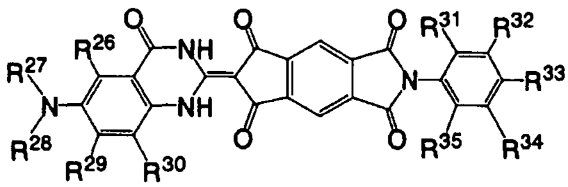

(5)

(wherein each of $R^{26}$-$R^{35}$ represents independently a hydrogen atom or a substituent; and for a combination among $R^{26}$-$R^{30}$ and a combination among $R^{31}$-$R^{35}$, each substituent within each combination may independently bond via a linkage group to form a ring system together with the carbon and/or nitrogen atom to which it bonds).

11. The optical recording medium according to the above-mentioned item 10, wherein at least one group among $R^{31}$-$R^{35}$ is a substituted alkoxy group having a heterocyclic residue containing at least one heteroatom.

12. The optical recording medium according to the above-mentioned item 10, wherein the atoms constituting each substituent represented by $R^{26}$-$R^{35}$ are selected from a carbon atom, a hydrogen atom, a nitrogen atom, a sulfur atom, and an oxygen atom.

13. The optical recording medium according to the above-mentioned item 1, wherein the recording layer (A) contains at least one kind of organic compound that absorbs the laser light and has a temperature at which the color changes by heat.

14. The optical recording medium according to the above-mentioned item 1, wherein the recording layer (A) contains at least one kind of organic compound that absorbs the laser light and forms a crystalline state exothermically when an amorphous state is heated.

15. The optical recording medium according to the above-mentioned item 1, wherein said recording layer (A) contains at least one kind of organic compound that exhibits maximum absorption wavelength ($\lambda max^2$) in the recording layer (A) after recording that is different from that ($\lambda max^1$) before recording in said recording layer (A) by irradiation with recording laser light.

16. The optical recording medium according to the above-mentioned item 1, wherein said recording layer (A) contains at least one kind of organic compound that, upon irradiation with a laser light with a recording/playback wavelength of $\lambda 0$, exhibits a refraction index n2 and an extinction coefficient k2 that are different from the refraction index n1 and the extinction coefficient k1 of said recording layer (A) at $\lambda 0$ before recording.

17. The optical recording medium according to the above-mentioned item 1, wherein the absorption maximum wavelength of the organic compound in solution is shifted to the shorter wavelength when it forms a thin film state of the recording layer.

18. A compound represented by general formula (5).

19. A quinazolin-4-one compound represented by general formula (6) having a disubstituted amino group at any of positions 5-8 in the quinazoline -4- ring:

[Formula 7]

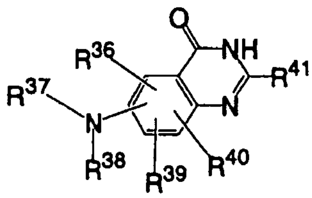

(6)

(wherein each of $R^{36}$-$R^{91}$ represents independently a hydrogen atom or a substituent).

20. A production method of the quinazolin-4-one compound represented by general formula (6) comprising reaction of a compound represented by the following general formula (7) and a compound represented by general formula (8) and/or general formula (9):

[Formula 8]

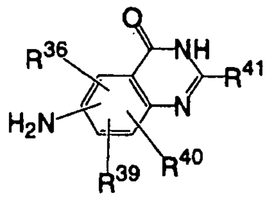

(7)

$R^{37}$-$Z^1$ (8)

$R^{38}$-$Z^2$ (9)

(wherein $R^{36}$-$R^{41}$ represent the same group as $R^{36}$-$R^{41}$ in formula (6); and $Z^1$ and $Z^2$ represent a leaving group).

21. A composition comprising at least one kind of compound represented by general formula (5).

22. An optical recording medium comprising a recording layer (A) capable of recording/playback with a laser light on a substrate,

wherein the recording layer (A) contains at least one kind of organic compound, wherein recording can be carried out by heat generated on irradiation of said recording layer (A) with recording laser light of 6 mW or lower and/or by the laser light without giving mechanical deformation to the substrate.

According to the present invention, by including at least one kind of specific organic compound relating to the present invention in recording layer (A) capable of recording and playback with laser light, it becomes possible to provide an optical recording medium containing a recording layer capable of high quality recording and playback of signals by reducing cross-write from neighboring tracks by using a low power laser of 300 nm to 900 nm, particularly laser of 390 nm to 430 nm, which attracts much attention for high-density optical recording medium, further specifically a blue-violet laser of 400 nm to 410 nm. In addition, it becomes possible to provide a recording material with which said recording layer can be produced by a coating method such as industrially favored spin-coating method.

BRIEF DESCRIPTION OF THE DRAWINGS

[Fig 1]
A conceptual view illustrating the subject of the present invention
[Fig 2]
A schematic view presenting an example of configuration for the optical recording medium of the present invention
[Fig 3]
A schematic view presenting another example of configuration for the optical recording medium of the present invention
[Fig 4]
A schematic view presenting still another example of configuration for the optical recording medium of the present invention
[Fig 5]
A schematic view presenting another example of configuration for the optical recording medium of the present invention
[Fig 6]
A schematic view presenting still another example of configuration for the optical recording medium of the present invention
[Fig 7]
A schematic view presenting another example of configuration for the optical recording medium of the present invention

BEST MODE FOR CARRYING OUT THE INVENTION

The present invention relates to an optical recording medium containing a specific organic compound (B)

relating to the present invention in its recording layer (A). The invention relates to a novel optical recording medium capable of recording and playback with laser light having a wavelength selected from 300 nm to 900 nm, particularly 390 nm to 430 nm or further specifically 400 nm to 410 nm, and novel compounds used for the optical recording medium relating to the present invention.

The optical recording medium relating to the present invention means an optical medium on which information can be recorded and played back. Here, as a preferred embodiment, the optical recording medium of the present invention comprising recording layer (A) and, if necessary, a reflection layer on a substrate will be explained. In the following, there will be explained an optical recording medium in the form of a discus-like optical disc, comprising a guide groove, for example, on a supporting substrate, a recording layer which can preferably be formed by a coating method and which has at least one kind of organic compound relating to the present invention as a recording material on the groove and a reflection layer, wherein recording/playback of signals are carried out by irradiation with laser light of 300 nm to 900 nm. However, the optical recording medium of the present invention is not limited to such shape or configuration but also includes those with various shapes such as card-type or sheet-type and those without a reflection layer, and furthermore it can be applied to recording/playback with a shorter-wavelength laser, which will be developed in the future.

The optical recording medium of the present invention has, for example, either a four-layer configuration wherein substrate 1, recording layer 2, reflection layer 3 and protection layer 4 are successively stacked as shown in Fig. 2 or a laminated configuration shown in Fig. 3. Namely, on substrate 1 is formed recording layer 2, on which is adhered reflection layer 3, on which is further adhered protection layer 4 through adhesion layer 5. Here, there may be another layer on or under recording layer 2, and there may be another layer on reflection layer 3. Furthermore, another configuration has substrate 1, reflection layer 3, recording layer 2 and protection layer 4 stacked in this order, wherein recording/playback is carried out from the protection layer side as shown in Fig. 4. The medium may also have a configuration wherein the thickness of the light transmission layer is specified by the numerical aperture N.A. of the optical system and wavelength $\lambda$ of the laser as disclosed in JP H10-326435.

The optical recording medium of the present invention may, if necessary, have a configuration comprising two or more recording layers as disclosed in JP H11-203729. The optical recording medium used for the configuration comprising two or more kinds of recording layers may have two or more kinds of recording layers (A) relating to the present invention, or it may contain one recording layer (A) of the present invention and at least one other recording layer (A'). The recording layer (A') includes a recording layer containing at least one above-mentioned known organic dye or a known inorganic recording layer using inorganic material such as metal or metalloid. Further, the medium may have two or more kinds of recording layers (A'). Recording layer (A) and recording layer (A') may be adjacent or separated by an intermediate layer. Further, when there are two or more kinds of layers each as recording layer (A) and recording layer (A') respectively, the positional relationship of each layer is not particularly limited. As the intermediate layer, which is located for separation, a transparent resin layer can be used to provide a separation distance, and it may appropriately comprise a reflection layer, a reflection enhancing layer or an adhesion layer. Between each recording layer and the substrate or between each recording layer and the protection layer, there may be a reflection layer, a reflection enhancing layer or an adhesion layer if necessary.

Further, the examples where the present invention is applied to optical disc include embodiments wherein substrate 11, recording layer 12, reflection layer 13 and protection layer 14 are stacked in this order and furthermore dummy substrate 15 is adhered onto protection layer 14, which also serves as an adhere layer as shown in Fig. 5. There may be configuration which lacks substrate 15. There may be another layer between substrate 11 and recording layer 12, between recording layer 12 and reflection layer 13, between reflection layer 13 and protection layer 14 or between protection layer 14 and dummy layer 15. In the optical disc shown in Fig. 5, recording/playback is carried out from the side of substrate 11.

Also, as another embodiment, there may be a configuration disclosed in JP H10-302310, for example, as shown in Fig. 6, wherein, on supporting substrate 11' having a guide groove, reflection layer 13' and recording layer 12' having an organic dye as a main component are formed in this order, light transmission layer 15' is formed on recording layer 12' through arbitrarily formed transparent protection layer 14', and recording/playback of information is carried out from the side of light transmission layer 15'. In addition, conversely, a guide groove may be formed on the side of light transmission layer 15', on which are stacked transparent protection layer 14', recording layer 12' and reflection layer 13', and supporting substrate 11' is adhered thereto.

Or as still another embodiment, there may be a configuration disclosed in JP 2002-175645, for example as shown in Fig. 7, wherein recording layer 22 is formed on supporting substrate 21 having a guide groove, on recording layer 22 is formed dielectric layer 40 containing successively stacked nitride layer 23 and oxide layer 24, on dielectric layer 40 is formed light transmission layer 25, if necessary through adhesive agent. Here information is recorded/reproduced from the side of light transmission layer 25. In addition, conversely, the guide groove may be formed on the side of light transmission layer 25, on which are formed dielectric layer 40 containing successively stacked oxide layer 24 and nitride layer 23, and recording layer 22, and supporting substrate 21 is adhered thereto. In this way, Organic compound (B) relating to the present invention can be applied to such an optical recording medium wherein suitable

initial reflectance is attained, because optical enhancement effect due to multiple reflections is obtained by forming a dielectric layer on an information recording layer without using a reflection layer.

The optical recording medium of the present invention is an optical recording medium containing at least one recording layer (A) capable of recording and playback with laser light, wherein said recording layer (A) contains at least one kind of organic compound (B), wherein the percentage change ($|[a^2 - a^1]/a^1 \times 100$) of the recording layer thickness ($a^2$) at the recorded site of said recording layer (A) after recorded with laser light compared with the recording layer thickness ($a^1$) at unrecorded site of said recording layer (A) is less than 25% and the amount of change ($|a^2 - a^1|$) of the recording layer thickness ($a^2$) at recorded site of said recording layer (A) after recorded with laser light compared with the recording layer thickness ($a^1$) at unrecorded site of said recording layer (A) is less than 15 nm.

In the present invention recording layer (A) is provided on a substrate. Said recording layer (A) is a recording layer which contains at least one kind of organic compound (B) relating to the present invention, wherein the percentage change ($|[a^2 - a^1]/a^1| \times 100$) of the recording layer thickness ($a^2$) at the recorded site of said recording layer (A) after recorded with laser light compared with the recording layer thickness ($a^1$) at unrecorded site of said recording layer (A) can be controlled to be less than 25% and the amount of change ($|a^2 - a^1|$) of the recording layer thickness ($a^2$) at recorded site of said recording layer (A) after recorded with laser light compared with the recording layer thickness ($a^1$) at unrecorded site of said recording layer (A) can be controlled to be less than 15 nm.

Here, the recording site in the present invention is a recording mark site wherein pit-formation or bump due to thermal deformation is controlled, and refers a site wherein optical change has occurred substantially without deformation. The percentage change ($| [a^2 - a^1] /a^1 | \times 100$) of the recording layer thickness ($a^2$) at the recorded site of said recording layer (A) after recorded with laser light compared with the recording layer thickness ($a^1$) at unrecorded site of said recording layer (A) is less than 25%, preferably less than 10%, more preferably less than 5%, further preferably less than 2%, and still further preferably less than 1%.

Further, the amount of change ($|a^2 - a^1|$) of the recording layer thickness ($a^2$) at recorded site of said recording layer (A) after recorded with laser light compared with the recording layer thickness ($a^1$) at unrecorded site of said recording layer (A) is less than 15 nm, preferably less than 10 nm, more preferably less than 5 nm, further preferably less than 2 nm, and still further preferably less than 1 nm.

In the present invention, it is preferred that optical change takes place without deformation at the recorded site when recording is carried out on recording layer (A) of the present invention with laser light and that playback can be carried out with playback laser light.

Said recording layer (A) relating to the present invention may be formed by any method unless the above-mentioned regulation is lost, but it is formed preferably by a coating method.

In said recording layer (A), which contains at least one kind of organic compound (B) as a recording material, inorganic compounds such as $SiO_2$ may be mixed or bonded if necessary. A preferred organic compound in the present invention includes an organic compound that has a six-membered ring structure consisting of four carbon atoms and two nitrogen atoms and has a substituted or unsubstituted amino group.

Organic compound (B) relating to the present invention is recording dye that, in contrast to dye precursor, alone enables recording without co-use of color developer and light absorber, and has sufficient absorbance at the wavelength of laser concerned, and be able to form a site with altered refractivity in recording layer (A), that is mentioned recording site, which change refractive index (n) and extinction coefficient (k) chemical change accompanying photo-thermal conversion , upon irradiation with a laser of a given energy. Furthermore, use of organic compound (B) relating to the present invention provides an optical recording medium having excellent multi-track recording characteristics.

The optical recording medium of the present invention can be carried out recording/playback with a laser having a wavelength selected from 300 nm to 900 nm. In particular, the optical recording medium is obtained good signal quality for a recording and playback laser having wavelength selected from 390 nm to 430 nm, furthermore from 400 nm to 410 nm. Moreover, the optical recording medium of the present invention is capable of recording by optical change without substantial deformation, when a recording layer (A) is single-layer that is formed by a composition wherein organic compound (B) relating to the present invention constitutes the major component, further, constitutes substantially 100%.

An organic compound used for organic compound (B) relating to the present invention can satisfy optical constants (refractive index n and extinction coefficient k) required for the recording layer at the above-mentioned wavelength of laser light by arbitrarily selecting absorption wavelength with absorption coefficient maintained through selection of substituents. Further, organic compound (B) relating to the present invention can also increase the intensity of read-out signals. Moreover, the organic compound used for organic compound (B) relating to the present invention can be used for recording layer (A) relating to the present invention which is prepared by a coating method in good coating shape. Thus, this compound is extremely valuable for using in optical recording media with excellent multi-track recording characteristics, particularly useful for recordable optical recording media, wherein the recording mark shape is formed based on optical change with a low recording laser power, without depending substantially on the deformation described above.

The detail of the present investigation will be explained below.

In the optical recording medium of the present invention, recording layers (A) containing at least one kind of organic compound are formed on a substrate, and at least one of said recording layers can form recording site that undergoes optical change not depending substantially on deformation. Preferably said recording layer (A) can be formed by a coating method.

The optical recording medium of the present invention is an optical recording medium containing at least one kind of organic compound which absorbs said laser light and exothermically forms on said recording layer (A) a crystalline state on heating in its amorphous state.

In the optical recording medium of the present invention, recording layer (A) relating to the present invention preferably contains at least one kind of organic compound which absorbs the laser light and has a temperature at which its color changes by heat (hereafter referred as color-change temperature), more preferably an organic compound which has at least one color-change temperature at 400°C or less, especially preferably an organic compound which has at least one color-change temperature at 400°C or less and no melting point at 400°C or less, as the recording dye. Here the color-change temperature in the present invention means a temperature range wherein the color of organic compound relating to the present invention changes before and after heating. The melting point in the present invention means a temperature range wherein the organic compound relating to the present invention turns from a solid state to a liquid state. The organic compound relating to the present invention is preferably an organic compound having at least one color-change temperature at 400°C or less, more preferably an organic compound that has at least one color-change temperature at 400°C or less and no melting point at 400°C or less, and it is not particularly limited as long it is soluble in a solvent used in coating. It can be used singly or in a combination of two or more as long as it satisfies the physical properties specified above. The color-change temperature range is preferably 200 to 400°C, more preferably 200 to 300°C.

As the organic compound relating to the present invention, may be used any organic compound that satisfies the physical properties specified above and has a color-change temperature, preferably an organic compound having a color-change temperature at 400°C or less, more preferably an organic compound having at least one color-change temperature at 400°C or less and no melting point at 400°C or less, without particular limitation.

The optical recording medium of the present invention is an optical recording medium containing, in recording layer (A), at least one kind of organic compound which gives said recording layer (A) after recording an maximum absorption wavelength ($\lambda max^2$) different from the maximum absorption wavelength ($\lambda max^1$) of said recording layer (A') before recording.

In the optical recording medium of the present invention, recording layer (A) relating to the present invention comprises, as the recording dye relating to the present invention, at least one kind of organic compound which exothermically forms a crystalline state on heating in its amorphous state, more preferably an organic compound of which an exothermic peak temperature at which it exothermically forms a crystalline state on heating in its amorphous state is 200°C or higher, more preferably an organic compound which exothermically forms a crystalline state on heating in its amorphous state and decomposes on heating in its crystalline state without showing a melting point. Here the amorphous state in the present invention means a solid state wherein molecules of the organic compound of the present invention are aggregated without forming crystals in which the molecules are gathered with regular spatial arrangement. The crystalline state in the present invention means a solid state having a crystal structure and includes perfect single crystals and a collection of microcrystals. The above-mentioned crystalline state may contain partially amorphous state. The melting point in the present invention means the temperature range in which the organic compound relating to the present invention turns from a solid state to a liquid state. Particularly, the recording layer preferably contains at least one kind of organic compound whose most intense absorption maximum in a wavelength range of 300 nm to 900 nm is at less than 400 nm, as the recording dye relating to the present invention.

The organic compound relating to the present invention is not particularly limited and can be used singly or in a combination of two or more, as long as it is an organic compound which exothermically forms a crystalline state on heating in its amorphous state, more preferably an organic compound which has an exothermic peak temperature at 200°C or higher at which it exothermically forms a crystalline state on heating in its amorphous state, more preferably an organic compound which exothermically forms a crystalline state on heating in its amorphous state and decomposes on heating in its crystalline state without showing a melting point, and it is soluble in a solvent used in coating.

The organic compound relating to the present invention is an organic compound which absorbs laser light and exothermically forms a crystalline state on heating in its amorphous state, more preferably an organic compound which has an exothermic peak temperature at 200°C or higher at which it exothermically forms a crystalline state on heating in its amorphous state, more preferably an organic compound which exothermically forms a crystalline state on heating in its amorphous state and decomposes on heating in its crystalline state without showing a melting point. It is not particularly limited as long as it satisfies the physical properties specified above.

Further, the optical recording medium of the present invention is an optical recording medium containing, in said recording layer (A), at least one kind of organic compound which, upon irradiation with the recording laser light, gives said recording layer (A) after recording an maximum absorption wavelength ($\lambda max^2$) different from the maximum

absorption wavelength ($\lambda max^1$) of said recording layer (A) before recording.

In the optical recording medium of the present invention, the recording layer (A) relating to the present invention is characterized, after recording by irradiation with the recording laser light, preferably by developing an absorption maximum at a wavelength ($\lambda max^2$) different from the maximum absorption wavelength ($\lambda max^1$) of said recording layer (A) before recording. The specific examples include a recording layer wherein its maximum absorption wavelength ($\lambda max^1$) lies at a wavelength shorter than the recording/playback wavelength ($\lambda 0$) ($\lambda max^1 < \lambda 0$) and the absorbance at $\lambda 0$ increases due to the change in maximum absorption wavelength to $\lambda max^2$ by irradiation with the recording laser light, or a recording medium wherein its maximum absorption wavelength ($\lambda max^1$) lies at a wavelength longer than the recording/playback wavelength ($\lambda 0$) ($\lambda max^1 > \lambda 0$) and the absorbance at $\lambda 0$ increases due to the change in maximum absorption wavelength to $\lambda max^2$ by irradiation with the recording laser light.

The organic compound relating to the present invention is preferably an organic compound whose maximum absorption wavelength ($\lambda max^1$) lies at a wavelength shorter than the recording/playback wavelength ($\lambda 0$) ($\lambda max^1 < \lambda 0$) and which, due to the change of maximum absorption wavelength to $\lambda max^2$ increases the absorbance of recording layer (A) at $\lambda 0$. More preferably, it is an organic compound which can shift the maximum absorption wavelength of the recording layer (A), which lies at a wavelength shorter than the playback wavelength, by 5 nm to 100 nm to the longer-wavelength side after recording, and which can be soluble in a solvent used on coating. It is not particularly limited and may be used singly or in a combination of two or more, as long as it satisfies the physical properties specified above. The amount of wavelength shift is preferably 10 nm to 50 nm, or more preferably 20 nm to 40 nm.

The maximum absorption wavelength in the present invention means a wavelength at which the absorbance shows a maximum in the absorption spectrum of said recording film (A).

The organic compound relating to the present invention is an organic compound which, by irradiation with the recording laser light, exhibits an maximum absorption wavelength ($\lambda max^2$) different from the maximum absorption wavelength ($\lambda max^1$) of said recording layer (A) before recording, preferably an organic compound whose maximum absorption wavelength ($\lambda max^1$) lies at a wavelength shorter than recording/playback wavelength ($\lambda 0$) ($\lambda max^1 < \lambda 0$) and which increases the absorbance of said recording layer (A) due to the change of maximum absorption wavelength to $\lambda max^2$ on irradiation with the recording laser light, more preferably an organic compound which can shift the maximum absorption wavelength of said recording layer (A), which lies at a wavelength shorter than the playback wavelength ($\lambda 0$) before recording, to the longer-wavelength side by 5 nm to 100 nm after recording. It is not particularly limited as long as it satisfies the physical properties specified above.

The optical recording medium of the present invention is an optical recording medium containing, in said recording layer (A), at least one kind of organic compound whichprovide refractive index n2 and extinction coefficient k2 that is different from refractive index n1 and extinction coefficient k1, respectively, at $\lambda 0$ of the recording layer (A) before recording, after recording by irradiation with the recording laser light having recording/ playback wavelength $\lambda 0$.

In the optical recording medium of the present invention, the recording layer (A) relating to the present invention is characterized, after irradiation with the recording laser light, preferably by attaining refractive index n2 and extinction coefficient k2 that is different from refractive index n1 and extinction coefficient k1, respectively, at the recording/playback wavelength $\lambda 0$ of said recording layer (A) before recording. The specific examples include a recording layer in which the refractive index decreases ($n2 < n1$) and the extinction coefficient increases ($k2 > k1$) by irradiation with recording laser.

The refractive index n and extinction coefficient k in the present invention correspond to the real part and imaginary part, respectively, of the complex index of refraction ($N = n - jk$) of said recording film (A).

Here is explained the method for determination of the complex index of refraction after recording.

It is necessary to determine the complex index of refraction of recording film (A) before laser irradiation in advance by a graph method, successive approximation method or the like. In this case, the substrate for preparing recording film (A) is not necessary to have a guide groove and it may be quartz glass or polycarbonate if the substrate surface is flat.

Next, to determine the complex index of refraction after laser irradiation, an apparatus for evaluating optical recording media is actually used, the tracking servo is made to follow the groove in the recording film (A), which was coated on the substrate having the guide groove to configure an optical recording medium, and both groove and land are irradiated with continuous recording laser light.

Then, dummy substrate and reflection film are removed by exfoliation, and the laser-irradiated part of recording film (A) and the substrate was cut into an appropriate piece, and the absorbance is measured in a suitable wavelength range considering the recording/playback wavelength ($\lambda 0$) on a spectrophotometer (For example, in the case of $\lambda 0$ = 405 nm, the wavelength range is 300 nm to 500 nm).

Since the absorption coefficient $\alpha$ can be obtained by dividing this absorbance by the thickness of dye film d, k can be determined by relation $\alpha = 4\pi k/\lambda$. The value of n can be determined by Kramers-Kronig conversion equation of k.

[Equation 1]

[Kramers-Kronig conversion equation]

$$n(\omega) - 1 = \frac{2}{\pi} P \int_0^{\infty} \frac{\omega' \cdot k(\omega')}{(\omega')^2 - \omega^2} d\omega'$$

P: Cauchy's principal integration
(cited from "Handbook of Optical Properties", Asakura-shoten, 1986, third impression)

The organic compound (B) relating to the present invention is more preferably an organic compound which can give said recording layer (A) a characteristic in which the refractive index n2 after recording is lower than the refractive index n1 at λ0 before recording (Δn = n2 - n1 <0) and the extinction coefficient k2 after recording is higher than the extinction coefficient k1 before recording (Δk = k2 - k1 > 0) on irradiation with the recording laser having the recording/playback wavelength λ0. Further preferably, it is an organic compound which can decrease the refractive index of recording layer (A) by 0.01 to 1.0 (Δn = -0.01 to -1.0) and increase its extinction coefficient by 0.005 to 0.5 (Δk = +0.005 to +0.5) after recording. It is not particularly limited and may be used singly or in a combination of two or more. Still more preferably, the organic compound (B) includes an organic compound which can decrease the refractive index by 0.02 to 0.08 (Δn = -0.02 to -0.08) and increase the extinction coefficient by 0.01 to 0.10 (Δk = +0. 01 to +0.10) after recording.

The organic compound relating to the present invention is an organic compound which can give said recording layer (A) a characteristic in which the refractive index n2 and extinction coefficient k2 after recording are different from the refractive index n1 and extinction coefficient k1 at λ0 before recording on irradiation with the recording laser having the recording/playback wavelength λ0, preferably an organic compound which can give said recording layer (A) a characteristic in which the refractive index n2 after recording is smaller than the refractive index before recording n1 (Δn = n2 - n1 < 0) and the extinction coefficient k2 after recording at λ0 is higher than the extinction coefficient k1 before recording (Δk = k2 - k1 >0) on irradiation with the recording laser having the recording/playback wavelength λ0, more preferably an organic compound which can decrease the refractive index of recording layer (A) by 0.01 to 1.0 (Δn = -0.01 to -1.0) after recording and increase its extinction coefficient by 0.005 to 0.5 (Δk = +0.05 to +0.5) after recording, and still more preferably an organic compound which can decrease the refractive index by 0. 02 to 0.08 (Δn = -0.02 to -0.08) after recording and increase the extinction coefficient by 0.01 to 0.10 (Δk = +0.01 to +0.10) after recording. It is not particularly limited as long as it satisfies the physical properties specified above.

The optical recording medium of the present invention is an optical recording medium wherein the maximum absorption wavelength of the organic compound in solution state is shifted to the shorter-wavelength if a recording layer becomes in state of a thin film.

The organic compound relating to the present invention used in recording layer (A) relating to the present invention includes a compound whose maximum absorption wavelength in solution is shifted to the shorter-wavelength if a recording layer becomes in state of a thin film. The solvent for preparing solution of said compound is not limited as long as the absorbance can be measured for the resultant solution. For example, the solvent includes alcoholic solvents such as methanol, ethanol, isopropyl alcohol, allyl alcohol, methyl cellosolve, ethyl cellosolve, tetrafluoropropanol, octafluoropentanol and the like; aliphatic or alicyclic hydrocarbon solvents such as hexane, heptane, octane, decane, cyclohexane, methylcyclohexane, ethylcyclohexane, dimethylcyclohexane and the like; aromatic hydrocarbon solvents such as toluene, xylene, benzene, t-butylbenzene, trimethylbenzene and the like; halogenated hydrocarbon solvents such as carbon tetrachloride, chloroform, tetrachloroethane, dibromomethane and the like; ether-type solvents such as diethyl ether, dibutyl ether, diisopropyl ether, dioxane and the like; ketone-type solvents such as acetone, 3-hydroxy-3-methyl-2-butanone, diisopropyl ketone and the like; ester-type solvents such as ethyl acetate, methyl lactate and the like; water and the like. The preferred solvents include halogenated hydrocarbon solvents such as carbon tetrachloride, chloroform, tetrachloroethane, dibromomethane and the like; alcoholic solvents such as tetrafluoropropanol, octafluoropentanol and the like, chloroform.

The amount of shift to the shorter-wavelength is preferably 5 nm or more, more preferably 10 nm or more, furthermore preferably 20 nm or more, when a recording layer becomes in state of a thin film.

The optical recording medium of the present invention has recording layer (A) capable of recording/playback with laser light on a substrate, wherein recording layer (A) contains at least one kind of organic compound, and is characterized by capability of recording without causing mechanical deformation of said substrate by laser light and/or heat generated by irradiation of said recording layer (A) to recording laser light with a power lower than 6 mW.

Thus, the optical recording medium of the present invention has very excellent feature in which it is free from small bump or pit observed in conventional optical recording media and there is substantially no deformation in the

substrate.

The preferred examples of the organic compound relating to the present invention, more specifically include organic compounds described below.

THe preferred organic compounds used for recording layer (A) relating to the present invention include a recording layer containing at least one kind of organic compound (B) which has a six-membered ring structure consisting of four carbon atoms and two nitrogen atoms and has a substituted or unsubstituted amino group.

Further, recording layer (A) composing the optical recording medium of the present invention is preferably a composition relating to the present invention, which comprises substantially one or more kind(s) of organic compound (B) relating to the present invention.

The content of organic compound (B) relating to the present invention in the composition of the present invention is, it can be arbitrarily selected from a range wherein recording and playback can be carried out, usually 30% by mass or more, preferably 60% by mass or more, and more preferably substantially 100% by mass.

Recording layer (A) of the present invention, besides organic compound (B) relating to the present invention, may contain another compound that has an absorption maximum at wavelength of 290 nm to 690 nm and high refractive index in 300 nm to 900 nm. Furthermore, if necessary, it may contain additives such as quencher, accelerator for thermal decomposition of compounds, ultraviolet ray absorber, adhesive agent, endothermic compound or endothermic decomposable compound and polymer for increasing solubility. Alternatively, compounds having such effects can be introduced as substituents into organic compound (B) relating to the present invention. The mixing ratio of these compounds in the composition of the present invention is usually 40% by mass or less.

Specific examples of preferred quencher include metal complexes of acetylacetonate compounds, bisdithiol compounds such as bisdithio-a-diketone compounds and bisphenyldithiol compounds, thiocatechonal compounds, salicylaldehyde oxime compounds, thiobisphenolate compounds, formazane compounds or the like. Further, amine compounds are also preferred.

The accelerator for thermal decomposition of compounds is not particularly limited as long as acceleration of thermal decomposition of compounds can be confirmed by means of thermogravimetric analysis (TG analysis) or the like. For example, the accelerator for thermal decomposition of compounds includes metal compound such as metallic anti-knocking agents, metallocene compounds and acetylacetonato-metal complexes.

The endothermic compound or endothermically decomposable compound includes the compound disclosed in JP H10-291366 or compounds having the substituents described therein.

Each kind of the above-mentioned quenchers, accelerators for thermal decomposition of compounds, and endothermic compound or endothermically decomposable compounds can be used singly or as a mixture of two or more kinds of them, if necessary.

Furthermore, additives such as binders, leveling agents and antifoaming agents can be added if necessary. The preferred binders include polyvinylalcohol, polyvinylpyrrolidone, nitrocellulose, cellulose acetate, ketone resin, acrylic resin, polystyrene resin, urethane resin, polyvinylbutyral, polycarbonate, polyolefin and the like.

The coating method for preparing recording layer (A) includes, for example, spin-coating method, spray method, casting method, slide method, curtain method, extrusion method, wire method, gravure method, spread method, roller-coat method, knife method, dipping method and the like. Spin-coating method is simple and preferred.

In coating method such as spin-coating method, is used coating solution in which compound (B) relating to the present invention is dissolved or dispersed in a solvent at a content of 1 to 40% by mass, preferably 3 to 30% by mass. Here, it is preferred to choose a solvent that causes no damage to the substrate. The solvent used in coating method includes alcoholic solvents such as methanol, ethanol, isopropyl alcohol, allyl alcohol, methyl cellosolve, ethyl cellosolve, tetrafluoropropanol, octafluoropentanol and the like; aliphatic hydrocarbon solvents or alicyclic hydrocarbon solvents such as hexane, heptane, octane, decane, cyclohexane, methylcyclohexane, ethylcyclohexane, dimethylcyclohexane and the like; aromatic hydrocarbon solvents such as toluene, xylene, benzene, t-butylbenzene, trimethylbenzene and the like; halogenated hydrocarbon solvents such as carbon tetrachloride, chloroform, tetrachloroethane, dibromoethane and the like; ether-type solvents such as diethyl ether, dibutyl ether, diisopropylether, dioxane and the like; ketone-type solvents such as acetone, 3-hydroxy-3-methyl-2-butanone, diisopropyl ketone and the like; ester-type solvents such as ethyl acetate, methyl lactate and the like; water and the like. These can be used singly or as a mixture of two or more. Solvents with boiling point of 150°C or lower under atmospheric pressure are preferred for production of optical recording medium of the present invention because of rapid drying after coating. Further preferred are alcoholic solvents, especially fluorinated alcohols, with boiling point of 150°C or lower under atmospheric pressure. Specifically, the solvent includes tetrafluoropropanol and octafluoropentanol as preferred examples.

It is preferred that organic compound (B) relating to the present invention is dissolved in the above-mentioned fluorinated alcohol usually at a content of 1 by mass or more, preferably 10% by mass or more, further preferably 20% by mass or more.

The six-membered ring structures consisting of four carbon atoms and two nitrogen atoms include 1,2-diaza-ring structure wherein two neighboring nitrogen atoms bond, 1,3-diaza-ring structure wherein two nitrogen atoms bond

though one carbon atom and 1,4-diaza-ring structure wherein two nitrogen atoms bond though two carbon atoms. 1,3-Diaza-ring structure is preferred. Neighboring atoms may bond via either a single bond or a double bond.

The preferred substituents in substituted amino group bonded to organic compound (B) relating to the present invention include a halogen atom, a nitro group, a cyano group, a hydroxyl group, a carboxyl group, a substituted or unsubstituted alkyl group, a substituted or unsubstituted aralkyl group, substituted or unsubstituted aryl group, a substituted or unsubstituted metallocenyl group, a substituted or unsubstituted alkenyl group, a substituted or unsubstituted alkoxy group, a substituted or unsubstituted aralkyloxy group, a substituted or unsubstituted aryloxy group, a substituted or unsubstituted alkenyloxy group, a substituted or unsubstituted alkylthio group, a substituted or unsubstituted aralkylthio group, a substituted or unsubstituted arylthio group, a substituted or unsubstituted alkenylthio group, a substituted or unsubstituted acyl group, a substituted or unsubstituted acyloxy group, a substituted or unsubstituted alkoxycarbonyl group, a substituted or unsubstituted aralkyloxycarbonyl group, a substituted or unsubstituted aryloxycarbonyl group, a substituted or unsubstituted alkenyloxycarbonyl group, a substituted or unsubstituted amino group or a group containing a metallocenyl group.

The specific examples of the substituent in the substituted amino group include halogen atoms such as a fluorine atom, a chlorine atom, a bromine atom and an iodine atom and the like; a nitro group, a cyano group, a hydroxyl group and a carboxyl group.

The specific examples of the substituted or unsubstituted alkyl group as the substituent in the substituted amino group include:

unsubstituted alkyl groups such as a methyl group, an ethyl group, an n-propyl group, an isopropyl group, an n-butyl group, an isobutyl group, a sec-butyl group, a tert-butyl group, an n-pentyl group, an isopentyl group, a 2-methylbutyl group, a 1-methylbutyl group, a neopentyl group, a 1,2-dimethylpropyl group, a 1,1-dimethylpropyl group, a cyclopentyl group, an n-hexyl group, a 4-methylpentyl group, a 3-methylpentyl group, a 2-methylpentyl group, a 1-methylpentyl group, a 3,3-dimethylbutyl group, a 2,3-dimethylbutyl group, a 1,3-dimethylbutyl group, a 2,2-dimethylbutyl group, a 1,2-dimethylbutyl group, a 1,1-dimethybutyl group, a 2-ethylbutyl group, a 1-ethylbutyl group, a 1,2,2-trimethylbutyl group, a 1,1,2-trimethylbutyl group, a 1-ethyl-2-methylpropyl group, a cyclohexyl group, an n-heptyl group, a 2-methylhexylgroup, a 3-methylhexyl group, a 4-methylhexyl group, a 5-methylhexyl group, a 2, 4-dimethylpentyl group, an n-octyl group, a 2-ethylhexyl group, a 2,5-dimethylhexyl group, a 2,5,5-trimethylpentyl group, a 2,4-dimethylhexyl group, a 2,2,4-trimethylpentyl group, a 3, 5, 5-trimethylhexyl group, an n-nonyl group, an n-decyl group, a 4-ethyloctyl group, a 4-ethyl-4,5-methylhexyl group, an n-undecyl group, an n-dodecyl group, a 1,3,5,7-tetraethyloctyl group, a 4-butyloctyl group, a 6,6-diethyloctyl group, an n-tridecyl group, a 6-methyl-4-butyloctyl group, an n-tetradecyl group, an n-pentadecyl group, a 3, 5-dimethylheptyl group, a 2,6-dimethylheptyl group, a 2,4-dimethylheptyl group, a 2,2,5,5-tetramethylhexyl group, a 1-cyclopentyl-2,2-dimethylpropyl group, a 1-cyclohexyl-2,2-dimethylpropyl group and the like;

alkyl groups substituted with halogen atom(s) such as a chloromethyl group, a1-chloroethyl group, a 2-chloroethyl group, a 2-bromoethyl group, a 2-iodoethyl group, a dichloromethyl group, a fluoromethyl group, a trifluoromethyl group, a pentafluoroethyl group, a 2,2,2-trifluoroethyl group, a 2,2,2-trichloroethyl group, a 1,1,1,3,3,3-hexafluoro-2-propyl group, a nonafluorobutyl group, a perfluorodecyl group and the like;

alkyl groups substituted with a hydroxyl group such as a hydroxymethyl group, a 2-hydroxyethyl group, a 3-hydroxypropyl group, a 4-hydroxybutyl group, a 2-hydroxy-3-methoxypropyl group, a 2-hydroxy-3-chloropropyl group, a 2-hydroxy-3-ethoxypropyl group, a 3-butyloxy-2-hydroxypropyl group, a 2-hydroxy-3-cyclohexyloxypropyl group, a 2-hydroxypropyl group, a 2-hydroxybutyl group, a 4-hydroxydecalyl group and the like;

alkyl groups substituted with a hydroxyalkoxy group such as a hydroxymethothymethyl group, a hydroxyethoxyethyl group, a 2-(2'-hydroxy-1'-methylethoxy)-1-methylethyl group, a 2-(3'-fluoro-2'-hydroxypropyloxy)ethyl group, a 2-(3'-chloro-2'-hydroxypropyloxy)ethyl group, a hydroxybutyloxycyclohexyl group and the like;

alkyl groups substituted with a hydroxyalkoxyalkoxy group such as a hydroxymethoxymethoxymethyl group, a hydroxyethoxyethoxyethyl group, a [2'-(2'-hydroxy-1'-methylethoxy)-1'-methylethoxylethoxyethyl group, a [2'-(2'-fluoro-1'-hydroxyethoxy)-1'-methylethoxy]ethoxyethyl group, a [2'-(2'-chloro-1'-hydroxyethoxy)-1'-methylethoxy] ethoxyethyl group and the like;

alkyl groups substituted with a cyano group such as a cyanomethyl group, a 2-cyanoethyl group, a 3-cyanopropyl group, a 4-cyanobutyl group, a 2-cyano-3-methoxypropyl group, a 2-cyano-3-chloropropyl group, a 2-cyano-3-ethoxypropyl group, a 3-butyloxy-2-cyanopropyl group, a 2-cyano-3-cyclohexylpropyl group, a 2-cyanopropyl group, a 2-cyanobutyl group and the like;

alkyl groups substituted with an alkoxy group such as a methoxymethyl group, an ethoxymethyl group, an n-propyloxymethyl group, an n-butyloxymethyl group, a methoxyethyl group, an ethoxyethyl group, an n-propyloxyethyl group, an n-butyloxyethyl group, an n-hexyloxyethyl group, a (4-methylpentyloxy)ethyl group, a (1,3-dimethylbutyloxy)ethyl group, a (2-ethylhexyloxy)ethyl group, an n-octyloxyethyl group, a (3,5,5-trimethylhexyloxy)ethyl group, a (2-methyl-1-isopropylpropyloxy)ethyl group, a (3-methyl-1-isopropylbutyloxy)ethyl group, a 2-ethoxy-1-methylethyl

group, a 3-methoxybutyl group, a (3,3,3-trifluoropropyloxy)ethyl group, a (3,3,3-trichloropropyloxy)ethyl group and the like;

alkyl groups substituted with an alkoxyalkoxy group such as a methoxymethoxymethyl group, a methoxyethoxyethyl group, an ethoxyethoxyethyl group, an n-propyloxyethoxyethyl group, an n-butyloxyethoxyethyl group, a cyclohexyloxyethoxyethyl group, a decalyloxypropyloxyethoxy group, a (1,2-dimethylpropyloxy)ethoxyethyl group, a (3-methyl-1-isobutylbutyloxy)ethoxyethyl group, a (2-methoxy-1-methylethoxy)ethyl group, a (2-butyloxy-1-methylethoxy) ethyl group, 2-(2'-ethoxy-1'-methylethoxy)-1-methylethyl group, a (3,3,3-trifluoropropyloxy)ethoxyethyl group, a (3,3,3-trichloropropyloxy)ethoxyethyl group and the like;

alkyl groups substituted with an alkoxyalkoxyalkoxy group such as a methoxymethoxymethoxymethyl group, a methoxyethoxyethoxyethyl group, an ethoxyethoxyethoxyethyl group, an n-butyloxyethoxyethoxyethyl group, a cyclohexyloxyethoxyethoxyethyl group, an n-propyloxypropyloxypropyloxyethyl group, a (2,2,2-trifluoroethoxy)ethoxyethoxyethyl group, a (2,2,2-trichloroethoxy)ethoxyethoxyethyl group and the like;

alkyl groups substituted with an acyl group such as a formylmethyl group, a 2-oxobutyl group, a 3-oxobutyl group, a 4-oxobutyl group, a 2,6-dioxocyclohexan-1-yl group, a 2-oxo-5-tert-butylcyclohexan-1-yl group and the like;

alkyl groups substituted with an acyloxy group such as a formyloxymethyl group, an acetoxyethyl group, an n-propionyloxyethyl group, an n-butanoyloxyethyl group, a valeryloxyethyl group, a (2-ethylhexanoyloxy)ethyl group, a (3,5,5-trimethylhexanoyloxy)ethyl group, a (3,5,5-trimethylhexanoyloxy)hexyl group, a (3-fluorobutyryloxy)ethyl group, a (3-chlorobutyryloxy)ethyl group and the like;

alkyl groups substituted with an acyloxyalkoxy group such as a formyloxymethoxymethyl group, an acetoxyethoxyethyl group, an n-propionyloxyethoxyethyl group, a valeryloxyethoxyethyl group, a (2-ethylhexanoyloxy)ethoxyethyl group, a (3,5,5-trimethylhexanoyloxy)oxybutyloxyethyl group, a (3,5,5-trimethylhexanoyloxy)ethoxyethyl group, a (2-fluoropropionyloxy)ethoxyethyl group, a (2-chloropropionyloxy)ethoxyethyl group and the like;

alkyl groups substituted with an acyloxyalkoxyalkoxy group such as an acetoxymethoxymethoxymethyl group, an acetoxyethoxyethoxyethyl group, an n-propionyloxyethoxyethoxyethyl group, a valeryloxyethoxyethoxyethyl group, a (2-ethylhexanoyloxy)ethoxyethoxyethyl group, a (3,5,5-trimethylhexanoyloxy)ethoxyethoxyethyl group, a (2-fluoropropionyloxy)ethoxyethoxyethyl group, a (2-chloropropionyloxy)ethoxyethoxyethyl group and the like;

alkyl groups substituted with an alkoxycarbonyl group such as a methoxycarbonylmethyl group, an ethoxycarbonylmethyl group, an n-butyloxycarbonylmethyl group, a methoxycarbonylethyl group, an ethoxycarbonylethyl group, an n-butyloxycarbonylethyl group, a (4-ethylcyclohexyloxycarbonyl)cyclohexyl group, a (2,2,3,3-tetrafluoropropyloxycarbonyl)methyl group, a (2,2,3,3-tetrachloropropyloxycarbonyl)methyl group and the like;

alkyl groups substituted with an aryloxycarbonyl group such as a phenyloxycarbonylmethyl group, a (2-methylphenyloxycarbonyl)methyl group, a (3-methylphenyloxycarbonyl)methyl group, a (4-methylphenyloxycarbonyl)methyl group, a (4-tert-butylphenyloxycarbonyl)methyl group, a phenyloxycarbonylethyl group, a (4-tert-butylphenyloxycarbonyl)ethyl group, a (1-naphthyloxycarbonyl)methyl group, a (2-naphthyloxycarbonyl)methyl group, a (2-phenylphenyloxycarbonyl)ethyl group, a (3-phenylphenyloxycarbonyl)ethyl group, a (4-phenylphenyloxycarbonyl)ethyl group and the like;

alkyl groups substituted with an aralkyloxycarbonyl group such as a benzyloxycarbonylmethyl group, a benzyloxycarbonylethyl group, a phenethyloxycarbonylmethyl group, a (4-cyclohexyloxybenzyloxycarbonyl)methyl group and the like;

alkyl groups substituted with an alkenyloxycarbonyl group such as a vinyloxycarbonylmethyl group, a vinyloxycarbonylethyl group, an allyloxycarbonylmethyl group, a cyclopentadienyloxycarbonylmethyl group, an octenoxycarbonylmethyl group and the like;

alkyl groups substituted with an alkoxycarbonyloxy group such as a methoxycarbonyloxymethyl group, a methoxycarbonyloxyethyl group, an ethoxycarbonyloxyethyl group, a butyloxycarbonyloxyethyl group, a (2,2,2-trifluoroethoxycarbonyloxy)ethyl group, (2,2,2-trichloroethoxycarbonyloxy)ethyl group and the like;

alkyl groups substituted with an alkoxyalkoxycarbonyloxy group such as a methoxymethoxycarbonyloxymethyl group, a methoxyethoxycarbonyloxyethyl group, an ethoxyethoxycarbonyloxyethyl group, an n-butyloxyethoxycarbonyloxyethyl group, a (2,2,2-trifluoroethoxy)ethoxycarbonyloxyethyl group, a (2,2,2-trichloroethoxy)ethoxycarbonyloxyethyl group and the like;

alkyl groups substituted with a dialkylamino group such as a dimethylaminomethyl group, a diethylaminomethyl group, a di-n-butylaminomethyl group, a di-n-hexylaminomethyl group, a di-n-octylaminomethyl group, a di-n-decylaminomethyl group, an N-isoamyl-N-methylaminomethyl group, a piperidinomethyl group, a di(methoxymethyl) aminomethyl group, a di(methoxyethyl)aminomethyl group, a di(ethoxymethyl)aminomethyl group, a di(ethoxyethyl) aminomethyl group, a (di-n-propyloxyethyl)aminomethyl group, a di(n-butyloxyethyl)aminomethyl group, a bis(2-cyclohexyloxyethyl)aminomethyl group, a dimethylaminoethyl group, a diethylaminoethyl group, a di-n-butylaminoethyl group, a di-n-hexylaminoethyl group, a di-n-octylaminoethyl group, a di-n-decylaminoethyl group, N-isoamyl-N-methylaminoethyl group, a piperidinoethyl group, a di(methoxymethyl)aminoethyl group, a di(methoxyethyl)aminoethyl group, a di(ethoxymethyl)aminoethyl group, a di(ethoxyethyl)aminoethyl group, a di(n-propyloxyethyl)aminoethyl group, a di-(n-butyloxyethyl)aminoethyl group, a bis(2-cyclohexyloxyethyl)aminoethyl group, a dimethylaminopropyl group, a diethylaminopropyl group, a di-n-butylaminopropyl group, a di-n-hexylaminopropyl group, a di-n-octylaminopropyl group, a di-n-decylaminopropyl group, an N-isoamyl-N-methylaminopropyl group, a piperidinopropyl group, a di(methoxymethyl)aminopropyl group, a di(methoxyethyl)aminopropyl group, a di(ethoxymethyl)aminopropyl group, a di(ethoxyethyl)aminopropyl group, a di(n-propyloxyethyl)aminopropyl group, a di(n-butyloxyethyl) aminopropyl group, a bis(2-cyclohexyloxyethyl)aminopropyl group, a dimethylaminobutyl group, a diethylaminobutyl group, a di-n-butylaminobutyl group, a di-n-hexylaminobutyl group, a di-n-octylaminobutyl group, a di-n-decylaminobutyl group, an N-isoamyl-N-methylaminobutyl group, a piperidinobutyl group, a di(methoxymethyl)aminobutyl group, a di(methoxyethyl)aminobutyl group, a di(ethoxymethyl)aminobutyl group, a di(ethoxyethyl)aminobutyl group, a di(n-propyloxyethyl)aminobutyl group, a di(n-butyloxyethyl)aminobutyl group, a bis(2-cyclohexyloxyethyl)amino butyl group and the like;

alkyl groups substituted with an acylamino group such as an acetylaminomethyl group, an acetylaminoethyl group, an n-propionylaminoethyl group, a n-butanoylaminoethyl group, a cyclohexylcarbonylaminoethyl group, a 4-methylcyclohexylcarbonylaminoethyl group, a succiniminoethyl group and the like;

alkyl groups substituted with an alkylsulfoneamino group such as a methylsulfoneaminomethyl group, a methylsulfoneaminoethyl group, an ethylsulfoneaminoethyl group, an n-propylsulfoneaminoethyl group, an n-octylsulfoneaminoethyl group and the like;

alkyl groups substituted with an alkylsulfonyl group such as a methylsulfonylmethyl group, an ethylsulfonylmethyl group, a butylsulfonylmethyl group, a methylsulfonylethyl group, an ethylsulfonylethyl group, an n-butylsulfonylethyl group, a 2-ethylhexylsulfonylethyl group, a 2,2,3,3-tetrafluoropropylsulfonylmethyl group, a 2,2,3,3-tetrachloropropylsulfonylmethyl group and the like;

alkyl groups substituted with an arylsulfonyl group such as a phenylsulfonylmethyl group, a phenylsulfonylethyl group, a phenylsulfonylpropyl group, a phenylsulfonylbutyl group, a 2-methylphenylsulfonylmethyl group, a 3-methylphenylsulfonylmethyl group, a 4-methylphenylsulfonylmethyl group, a 4-methylphenylsulfonylethyl group, a 4-methylphenylsulfonylpropyl group, a 4-methylphenylsulfonylbutyl group, a 2,4-dimethylphenylsulfonylmethyl group, a 2,6-dimethylphenylsulfonylmethyl group, a 2,4-dimethylphenylsulfonylethyl group, a 2,4-dimethylphenylsulfonylpropyl group, a 2,4-dimethylphenylsulfonylbutyl group and the like;

alkyl groups substituted with a heterocyclic group such as a thiadiazolinomethyl group, a pyrrolinomethyl group, a pyrrolidinomethyl group, a pyrazolinomethyl group, an imidazolidinomethyl group, an oxazolyl group, a triazolinomethyl group, a morpholinomethyl group, an indolinomethyl group, a benzimidazolinomethyl group, a carbazolinomethyl group and the like; and the like.

The substituted or unsubstituted aralkyl group as the substituent in the substituted amino group is an aralkyl group optionally substituted with the above-mentioned alkyl group(s) or an aralkyl group optionally substituted with substituent(s) like those which the above-mentioned alkyl groups may have. The specific examples include a benzyl group, a phenethyl group, an α-methylbenzyl group, an α,α-dimethylbenzyl group, a 1-naphthylmethyl group, a 2-naphthylmethyl group, a furfuryl group, a 2-methylbenzyl group, a 3-methylbenzyl group, a 4-methylbenzyl group, a 4-ethylbenzyl group, a 4-isopropylbenzyl group, a 4-tert-butylbenzyl group, a 4-n-hexylbenzyl group, a 4-n-nonylbenzyl group, a 3,4-dimethylbenzyl group, a 3-methoxybenzyl group, a 4-methoxybenzyl group, a 4-ethoxybenzyl group, a 4-n-butyloxybenzyl group, a 4-n-hexyloxybenzyl group, a 4-n-nonyloxybenzyl group, a 3-fluorobenzyl group, a 4-fluorobenzyl group, a 2-chlorobenzyl group, 4-chlorobenzyl group and the like.

The substituted or unsubstituted aryl group as the substituent in the substituted amino group is an unsubstituted carbocyclic aromatic group or heterocyclic aromatic group, or a carbocyclic aromatic group or heterocyclic aromatic group substituted with the above-mentioned alkyl group(s), or a carbocyclic aromatic group or heterocyclic aromatic group substituted with substituent(s) like those which the above-mentioned alkyl groups may have. The specific examples include substituted or unsubstituted carbocyclic aromatic groups such as a phenyl group, a 4-methylphenyl group, a 3-methylphenyl group, a 2-methylphenyl group, a 4-ethylphenyl group, a 3-ethylphenyl group, a 2-ethylphenyl group, a 4-n-propylphenyl group, a 4-isopropylphenyl group, a 2-isopropylphenyl group, a 4-n-butylphenyl group, a 4-isobutylphenyl group, a 4-sec-butylphenyl group, a 2-sec-butylphenyl group, a 4-tert-butylphenyl group, a 3-tert-butylphenyl group, a 2-tert-butylphenyl group, a 4-n-pentylphenyl group, a 4-isopentylphenyl group, a 4-neopentylphenyl group, a 4-tert-pentylphenyl group, a 4-n-hexylphenyl group, a 4-(2'-ethylbutyl)phenyl group, a 4-n-heptylphenyl group, a 4-n-octylphenyl group, a 4-(2'-ethylhexyl)phenyl group, a 4-n-nonylphenyl group, a 4-n-decylphenyl group, a 4-n-undecylphenyl group, a 4-n-dodecylphenyl group, a 4-n-tetradecylphenyl group, a 4-cyclohexylphenyl group, a 4-(4'-methylcyclohexyl)phenyl group, a 4-(4'-tert-butylcyclohexyl)phenyl group, a 3-cyclohexylphenyl group, a 2-cyclohexylphenyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 3,4,5-trimethylphenyl group, a 2,3,5,6-tetramethylphenyl group, a 2,4-diethylphenyl group, 2,6-diethylphenyl group, a 2,5-diisopropylphenyl group, a 2,6-diisopropylphenyl group, a 2, 6-diisobutylphenyl group, a 2,4-di-tert-butylphenyl group, a 2,5-di-tert-butylphenyl group, a 4,6-di-tert-butyl-2-methylphenyl

group, a 5-tert-butyl-2-methylphenyl group, a 4-tert-butyl-2,6-dimethylphenyl group, a 1-napththyl group, a 2-naphthyl group, a 1,2,3,4-tetrtahydro-5-naphthyl group, a 1,2,3,4-tetrtahydro-6-naphthyl group, a 4-ethyl-1-naphthyl group, a 6-n-butyl-2-naphthyl group, a 5-indanyl group, a 4-methoxyphenyl group, a 3-methoxyphenyl group, a 2-methoxyphenyl group, a 4-ethoxyphenyl group, a 3-ethoxyphenyl group, a 2-ethoxyphenyl group, a 4-n-propyloxyphenyl group, a 3-n-propyloxyphenyl group, a 4-isopropyloxyphenyl group, a 2-isopropyloxyphenyl group, a 4-n-butyloxyphenyl group, a 4-isobutyloxyphenyl group, a 2-sec-butyloxyphenyl group, a 4-n-pentyloxyphenyl group, a 4-isopentyloxyphenyl group, a 2-isopentyloxyphenyl group, a 4-neopentyloxyphenyl group, a 2-neopentyloxyphenyl group, a 4-n-hexyloxyphenyl group, a 4-(2'-ethylbutyl)oxyphenyl group, a 4-n-heptyloxyphenyl group, a 4-n-octyloxyphenyl group, a 4-n-nonyloxyphenyl group, a 4-n-decyloxyphenyl group, a 4-n-undecyloxypheyl group, a 4-n-dodecyloxyphenyl group, a 4-n-tetradecyloxyphenyl group, a 4-cyclohexyloxyphenyl group, a 2-cyclohexyloxyphenyl group, a 2,3-dimethoxyphenyl group, a 2,4-dimethoxyphenyl group, a 2,5-dimethoxyphenyl group, a 3,4-dimethoxyphenyl group, a 3,5-dimethoxyphenyl group, a 3,5-diethoxyphenyl group, a 2-methoxy-4-methylphenyl group, a 2-methoxy-5-methylphenyl group, a 2-methyl-4-methoxyphenyl group, a 3-methyl-4-methoxyphenyl group, a 3-methyl-5-methoxyphenyl group, a 2-methoxy-1-naphthyl group, a 4-methoxy-1-naphthyl group, a 4-n-butyloxy-1-naphthyl group, a 5-ethoxy-1-naphthyl group, a 6-methoxy-2-naphthyl group, a 6-ethoxy-2-naphthyl group, a 6-n-butyloxy-2-naphthyl group, a 6-n-hexyloxy-2-naphthyl group, a 7-methoxy-2-naphthyl group, a 7-n-butyloxy-2-naphthyl group, a 4-phenylphenyl group, a 3-phenylphenyl group, a 2-phenylphenyl group, a 4-(4'-methylphenyl)phenyl group, a 4-(3'-methylphenyl)phenyl group, a 4-(4'-ethylphenyl)phenyl group, a 4-(4'-isopropylphenyl)phenyl group, a 4-(4'-tert-butyllphenyl)phenyl group, a 4-(4'-n-hexylphenyl)phenyl group, a 4-(4'-n-octylphenyl)phenyl group, a 4-(4'-methoxyphenyl)phenyl group, a 4-(4'-n-butyloxyphenyl)phenyl group, a 2-(2'-methoxyphenyl)phenyl group, a 4-(4'-chlorophenyl)phenyl group, 3-methyl-4-phenylphenyl group, a 3-methoxy-4-phenylphenyl group, a 9-phenyl-2-fluorenyl group, a 9,9-diphenyl-2-fluorenyl group, a 9-methyl-9-phenyl-2-fluorenyl group, a 9-ethyl-9-phenyl-2-fluorenyl group, a4-fluorophenyl group, a 3-fluorophenyl group, a2-fluorophenyl group, a 4-chlorophenyl group, a 3-chlorophenyl group, a 2-chlorophenyl group, a 4-bromophenyl group, a 2-bromophenyl group, a 4-trifluoromethylphenyl group, a 2,3-difluorophenyl group, a 2,4-difluorophenyl group, a 2,5-difluorophenyl group, a 2,6-difluorophenyl group, a 3,4-difluorophenyl group, a 3,5-difluorophenyl group, a 2,3-dichlorophenyl group, a 2,4-dichlorophenyl group, a 2,5-dichlorophenyl group, a 3,4-dichlorophenyl group, a 3,5-dichlorophenyl group, a 2,5-dibromophenyl group, a 2,4,6-trichlorophenyl group, a 2-fluoro-4-methylphenyl group, a 2-fluoro-5-methylphenyl group, a 3-fluoro-2-methylphenyl group, a 3-fluoro-4-methylphenyl group, a 2-methyl-4-fluorophenyl group, a 2-methyl-5-fluorophenyl group, a 3-methyl-4-fluorophenyl group, a 2-chloro-4-methylphenyl group , a 2-chloro-5-methylphenyl group , a 2-chloro-6-methylphenyl group, a 3-chloro-4-methylphenyl group, a 2-methyl-3-chlorophenyl group, a 2-methyl-4-chlorophenyl group, a 3-methyl-4-chlorophenyl group, a 2-chloro-4,6-dimethylphenyl group, 2,4-dichloro-1-naphthyl group, 1,6-dichloro-2-naphthyl group, a 2-methoxy-4-fluorophenyl group, a 3-methoxy-4-fluorophenyl group, a 2-fluoro-4-methoxyphenyl group, a 2-fluoro-4-ethoxyphenyl group, a 2-fluoro-6-methoxyphenyl group, a 3-fluoro-4-methoxyphenyl group, a 3-fluoro-4-ethoxyphenyl group, 2-chloro-4-methoxyphenyl group, a 3-chloro-4-methoxyphenyl group, a 2-methoxy-5-chlorophenyl group, a 3-methoxy-4-chlorophenyl group, a 3-methoxy-6-chlorophenyl group, a 5-chloro-2,4-dimethoxyphenyl group, a 2-hydroxyphenyl group, a 3-hydroxyphenyl group, a 4-hydroxyphenyl group, a 2-nitrophenyl group, a 3-nitrophenyl group, a 4-nitrophenyl group, a 2-cyanophenyl group, a 3-cyanophenyl group, a 4-cyanophenyl group, a 2-methyl-5-nitrophenyl group, a 3,5-dinitrophenyl group, a 2-hydroxy-4-nitrophenyl group and the like; and substituted or unsubstituted heterocyclic aromatic groups such as a 4-pyridyl group, a 3-pyridyl group, a 2-pyridyl group, a 4-methyl-2-pyridyl group, a 5-methyl-2-pyridyl group, a 6-methyl-2-pyridyl group, a 4,6-dimethyl-2-pyridyl group, a 4-methyl-5-nitro-2-pyridyl group, a 3-hydroxy-2-pyridyl group, a 6-fluoro-3-pyridyl group, a 6-methoxy-3-pyridyl group, a 6-methoxy-2-pyridyl group, a 2-pyrimidyl group, a 3-pyrimidyl group, a 4-pyrimidyl group, a 2,6-dimethyl-4-pyrimidyl group, a 4-quinolyl group, a 3-quinolyl group, a 4-methyl-2-quinolyl group, a 3-furyl group, a 2-furyl group, a 3-thienyl group, a 2-thienyl group, a 4-methyl-3-thienyl group, a 5-methyl-2-thienyl group, a 3-methyl-2-thienyl group, a 2-oxazolyl group, a 2-thiazolyl group, a 2-thiadiazolyl group, a 2-benzoxazolyl group, a 2-benzothiazolyl group, 2-benzimidazolyl group and the like.

The substituted or unsubstituted metallocenyl group as the substituent in the substituted amino group is an unsubstituted metallocenyl group, a metallocenyl group substituted with the above-mentioned alkyl group(s) or a metallocenyl group substituted with substituent(s) like those which the above-mentioned alkyl groups may have. The specific examples include substituted or unsubstituted metallocenyl groups such as a ferrocenyl group, a cobaltocenyl group, a nickelocenyl group, a dichlorotitanocenyl group, a trichlorotitaniumcyclopentadienyl group, a bis(trifluoromethanesulfonato)titanocenyl group, a dichlorozirconocenyl group, a dimethylzirconocenyl group, a diethoxyzirconocenyl group, a bis(cyclopentadienyl)chromium group, a bis(cyclopentadienyl)dichloromolybdenum group, a bis(cyclopentadienyl) dichlorohafnium group, a bis(cyclopentadienyl)dichloroniobium group, a bis(cyclopentadienyl) ruthenium group, a bis (cyclopentadienyl)vanadium group, a bis(cyclopentadienyl)dichlorovanadium group, an octamethylferrocenyl group, an octamethylcobaltocenyl group, an octamethylnickelocenyl group and the like.

The substituted or unsubstituted alkenyl group as the substituent in the substituted amino group is an alkenyl group optionally substituted with the above-mentioned alkyl group(s) or an alkenyl group optionally substituted with substituent (s) like those which the above-mentioned alkyl groups may have. The specific examples include a vinyl

group, a propenyl group, a 1-butenyl group, an isobutenyl group, a 1-pentenyl group, a 2-pentenyl group, a 2-methyl-l-butenyl group, a 3-methyl-1-butenyl group, a 2-methyl-2-butenyl group, a 2,2-dicyanovinyl group, a 2-cyano-2-methylcarboxyvinyl group, a 2-cyano-2-methylsulfonevinyl group, a styryl group, a 4-phenyl-2-butenyl group and the like.

The substituted or unsubstituted alkoxy group as the substituent in the substituted amino group is an alkoxy group optionally substituted with substituent (s) like those which the above-mentioned alkyl groups may have. The specific examples include linear, branched or cyclic unsubstituted alkoxy groups such as a methoxy group, an ethoxy group, an n-propyloxy group, an isopropyloxy group, an n-butyloxy group, an isobutyloxy group, a tert-butyloxy group, a sec-butyloxy group, an n-pentyloxy group, an isopentyloxy group, a tert-pentyloxy group, a sec-pentyloxy group, a cyclopentyloxy group, an n-hexyloxy group, a 1-methylpentyloxy group, a 2-methylpentyloxy group, a 3-methylpentyloxy group, a 4-methylpentyloxy group, a 1,1-dimethylbutyloxy group, a 1,2-dimethylbutyloxy group, a 1,3-dimethylbutyloxy group, a 2,3-dimethylbutyloxy group, a 1,1,2-trimethylpropyloxy group, a 1,2,2-trimethylpropyloxy group, a 1-ethylbutyloxy group, a 2-ethylbutyloxy group, a 1-ethyl-2-methylpropyloxy group, a cyclohexyloxy group, a methylcyclopentyloxy group, an n-heptyloxy group, a 1-methylhexyloxy group, a 2-methylhexyloxy group, a 3-methylhexyloxy group, a 4-methylhexyloxy group, a 5-methylhexyloxy group, a 1,1-dimethylpentyloxy group, a 1.,2-dimethylpentyloxy group, a 1,3-dimethylpentyloxy group, a 1,4-dimethylpentyloxy group, a 2,2-dimethylpentyloxy group, a 2,3-dimethylpentyloxy group, a 2,4-dimethylpentyloxy group, a 3,3-dimethylpentyloxy group, a 3,4-dimethylpentyloxy group, a 1-ethylpentyloxy group, a 2-ethylpentyloxy group, a 3-ethylpentyloxy group, a 1,1,2-trimethylbutyloxy group, a 1,1,3-trimethylbutyloxy group, a 1,2,3-trimethylbutyloxy group, a 1,2,2-trimethylbutyloxy group, a 1,3,3-trimethylbutyloxy group, a 2,3,3-trimethylbutyloxy group, a 1-ethyl-1-methylbutyloxy group, a 1-ethyl-2-methylbutyloxy group, a 1-ethyl-3-methylbutyloxy group, a 2-ethyl-1-methylbutyloxy group, a 2-ethyl-3-methylbutyloxy group, a 1-n-propylbutyloxy group, a 1-isopropylbutyloxy group, a 1-isopropyl-2-methylpropyloxy group, a methylcyclohexyloxy group, an n-octyloxy group, a 1-methylheptyloxy group, a 2-methylheptyloxy group, a 3-methylheptyloxy group, a 4-methylheptyloxy group, a 5-methylheptyloxy group, a 6-methylheptyloxy group, a 1,1-dimethylhexyloxy group, a 1,2-dimethylhexyloxy group, a 1,3-dimethylhexyloxy group, a 1,4-dimethylhexyloxy group, a 1,5-dimethylhexyloxy group, a 2,2-dimethylhexyloxy group, a 2,3-dimethylhexyloxy group, a 2,4-dimethylhexyloxy group, a 2,5-dimethylhexyloxy group, a 3,3-dimethylhexyloxy group, a 3,4-dimethylhexyloxy group, a 3,5-dimethylhexyloxy group, a 4,4-dimethylhexyloxy group, a 4,5-dimethylhexyloxy group, a 1-ethylhexyloxy group, a 2-ethylhexyloxy group, a 3-ethylhexyloxy group, a 4-ethylhexyloxy group, a 1-n-propylpentyloxy group, a 2-n-propylpentyloxy group, a 1-isopropylpentyloxy group, a 2-isopropylpentyloxy group, a 1-ethyl-1-methylpentyloxy group, a 1-ethyl-2-methylpentyloxy group, a 1-ethyl-3-methylpentyloxy group, a 1-ethyl-4-methylpentyloxy group, a 2-ethyl-1-methylpentyloxy group, a 2-ethyl-2-methylpentyloxy group, a 2-ethyl-3-methylpentyloxy group, a 2-ethyl-4-methylpentyloxy group, a 3-ethyl-1-methylpentyloxy group, a 3-ethyl-2-methylpentyloxy group, a 3-ethyl-3-methylpentyloxy group, a 3-ethyl-4-methylpentyloxy group, a 1,1,2-trimethylpentyloxy group, a 1,1,3-trimethylpentyloxy group, a 1,1,4-trimethylpentyloxy group, a 1,2,2-trimethylpentyloxy group, a 1,2,3-trimethylpentyloxy group, a 1,2,4-trimethylpentyloxy group, a 1,3,4-trimethylpentyloxy group, a 2,2,3-trimethylpentyloxy group, a 2,2,4-trimethylpentyloxy group, a 2,3,4-trimethylpentyloxy group, a 1,3,3-trimethylpentyloxy group, a 2,3,3-trimethylpentyloxy group, a 3,3,4-trimethylpentyloxy group, a 1,4,4-trimethylpentyloxy group, a 2,4,4-trimethylpentyloxy group, a 3,4,4-trimethylpentyloxy group, a 1-n-butylbutyloxy group, a 1-isobutylbutyloxy group, a 1-sec-butylbutyloxy group, a 1-tert-butylbutyloxy group, a 2-tert-butylbutyloxy group, a 1-n-propyl-1-methylbutyloxy group, a 1-n-propyl-2-methylbutyloxy group, a 1-n-propyl-3-methylbutyloxy group, a 1-isopropyl-1-methylbutyloxy group, a 1-isopropyl-2-methylbutyloxy group, a 1-isopropyl-3-methylbutyloxy group, a 1,1-diethylbutyloxy group, a 1,2-diethylbutyloxy group, a 1-ethyl-1,2-dimethylbutyloxy group, a 1-ethyl-1,3-dimethylbutyloxy group, a 1-ethyl-2,3-dimethylbutyloxy group, a 2-ethyl-1,1-dimethylbutyloxy group, a 2-ethyl-1,2-dimethylbutyloxy group, a 2-ethyl-1,3-dimethylbutyloxy group, a 2-ethyl-2,3-dimethylbutyloxy group, a 1,1,3,3-tetramethylbutyloxy group, a 1,2-dimethylcyclohexyloxy group, a 1,3-dimethylcyclohexyloxy group, a 1, 4-dimethylcyclohexyloxy group, an ethylcyclohexyloxy group, an n-nonyloxy group, a 3,5,5-trimethylhexyloxy group, an n-decyloxy group, an n-undecyloxy group, an n-dodecyloxy group, a 1-adamantyloxy group, an n-pentadecyloxy group and the like;

alkoxy groups substituted with an alkoxy group such as a methoxymethoxy group, a methoxyethoxy group, an ethoxyethoxy group, an n-propyloxyethoxy group, an isopropyloxyethoxy group, an n-butyloxyethoxy group, an isobutyloxyethoxy group, a tert-butyloxyethoxy group, a sec-butyloxyethoxy group, an n-pentyloxyethoxy group, an isopentyloxyethoxy group, a tert-pentyloxyethoxy group, a sec-pentyloxyethoxy group, a cyclopentyloxyethoxy group, an n-hexyloxyethoxy group, an ethylcyclohexyloxyethoxy group, an n-nonyloxyethoxy group, a (3,5,5-trimethylhexyloxy)ethoxy group, a (3,5,5-trimethylhexyloxy)butyloxy group, an n-decyloxyethoxy group, an n-undecyloxyethoxy group, an n-dodecyloxyethoxy group, a 3-methoxypropyloxy group, a 3-ethoxypropyloxy group, a 3-(n-propyloxy)propyloxy group, a 2-isopropyloxypropyloxy group, a 2-methoxybutyloxy group, a 2-ethoxybutyloxy group, a 2-(n-propyloxy)butyloxy group, a 4-isopropyloxybutyloxy group, a decalyloxyethoxy group, an adamantyloxyethoxy group and the like;

alkoxy groups substituted with an alkoxyalkoxy group such as a methoxymethoxymethoxy group, an ethoxymethoxymethoxy group, a propyloxymethoxymethoxy group, a butyloxymethoxymethoxy group, a methoxyethoxymethoxy group, an ethoxyethoxymethoxy group, a propyloxyethoxymethoxy group, a butyloxyethoxymethoxy group, a methoxypropyloxymethoxy group, an ethoxypropyloxymethoxy group, a propyloxypropyloxymethoxy group, a butyloxypropyloxymethoxy group, a methoxybutyloxymethoxy group, an ethoxybutyloxymethoxy group, a propyloxybutyloxymethoxy group, a butyloxybutyloxymethoxy group, a methoxymethoxyethoxy group, an ethoxymethoxyethoxy group, a propyloxymethoxyethoxy group, a butyloxymethoxyethoxy group, a methoxyethoxyethoxy group, an ethoxyethoxyethoxy group, a propyloxyethoxyethoxy group, a butyloxyethoxyethoxy group, a methoxypropyloxyethoxy group, an ethoxypropyloxyethoxy group, a propyloxypropyloxyethoxy group, a butyloxypropyloxyethoxy group, a methoxybutyloxyethoxy group, an ethoxybutyloxyethoxy group, a propyloxybutyloxyethoxy group, a butyloxybutyloxyethoxy group, a methoxymethoxypropyloxy group, an ethoxymethoxypropyloxy group, a propyloxymethoxypropyloxy group, a butyloxymethoxypropyloxy group, a methoxyethoxypropyloxy group, an ethoxyethoxypropyloxy group, a propyloxyethoxypropyloxy group, a butyloxyethoxypropyloxy group, a methoxypropyloxypropyloxy group, an ethoxypropyloxypropyloxy group, a propyloxypropyloxypropyloxy group, a butyloxypropyloxypropyloxy group, a methoxybutyloxypropyloxy group, an ethoxybutyloxypropyloxy group, a propyloxybutyloxypropyloxy group, a butyloxybutyloxypropyloxy group, a methoxymethoxybutyloxy group, an ethoxymethoxybutyloxy group, a propyloxymethoxybutyloxy group, a butyloxymethoxybutyloxy group, a methoxyethoxybutyloxy group, an ethoxyethoxybutyloxy group, a propyloxyethoxybutyloxy group, a butyloxyethoxybutyloxy group, a methoxypropyloxybutyloxy group, an ethoxypropyloxybutyloxy group, a propyloxypropyloxybutyloxy group, a butyloxypropyloxybutyloxy group, a methoxybutyloxybutyloxy group, an ethoxybutyloxybutyloxy group, a propyloxybutyloxybutyloxy group, a butyloxybutyloxybutyloxy group, a (4-ethylcyclohexyloxy)ethoxyethoxy group, a (2-ethyl-1-hexyloxy)ethoxypropyloxy group, a [4-(3,5,5-trimethylhexyloxy)butyloxy]ethoxy group and the like;

alkoxy groups substituted with an alkoxycarbonyl group such as a methoxycarbonylmethoxy group, an ethoxycarbonylmethoxy group, an n-propyloxycarbonylmethoxy group, an isopropyloxycarbonylmethoxy group, a (4'-ethylcyclohexyloxy)carbonylmethoxy group and the like;

alkoxy groups substituted with an acyl group such as an acetylmethoxy group, an ethylcarbonylmethoxy group, an n-octylcarbonylmethoxy group and a phenacyloxy group;

alkoxy groups substituted with an acyloxy group such as an acetyloxymethoxy group, an acetyloxyethoxy group, an acetyloxyhexyloxy group, an n-butanoyloxycyclohexyloxy group and the like;

alkoxy groups substituted with an alkylamino group such as a methylaminomethoxy group, a 2-methylaminoethoxy group, a 2-(2-methylaminoethoxy)ethoxy group, a 4-methylaminobutyloxy group, a 1-methylaminopropan-2-yloxy group, a 3-methylaminopropyloxy group, a 2-methylamino-2-methylpropyloxy group, a 2-ethylaminoethoxy group, a 2-(2-ethylaminoethoxy)ethoxy group, a 3-ethylaminopropyloxy group, a 1-ethylaminopropyloxy group, a 2-isopropylaminoethoxy group, a 2-(n-butylamino)ethoxy group, a 3-(n-hexylamino)propyloxy group, 4-(cyclohexylamino)butyloxy group and the like;

alkoxy groups substituted with an alkylaminoalkoxy group such as a methylaminomethoxymethoxy group, a methylaminoethoxyethoxy group, a methylaminoethoxypropyloxy group, an ethylaminoethoxypropyloxy group, a 4-(2'-isobutylaminopropyloxy)butyloxy group and the like;

alkoxy groups substituted with a dialkylamino group such as a dimethylaminomethoxy group, a 2-dimethylaminoethoxy group, a 2-(2-dimethylaminoethoxy)ethoxy group, a 4-dimethylaminobutyloxy group, a 1-dimethylaminopropan-2-yloxy group, a 3-dimethylaminopropyloxy group, a 2-dimethylamino-2-methylpropyloxy group, a 2-diethylaminoethoxy group, a 2-(2-diethylaminoethoxy)ethoxy group, a 3-diethylaminopropyloxy group, a 1-diethylaminopropyloxy group, a 2-diisopropylaminoethoxy group, a 2-(di-n-butylamino)ethoxy group, a 2-piperidylethoxy group, a 3-(di-n-hexylamino)propyloxy group and the like;

alkoxy groups substituted with a dialkylaminoalkoxy group such as a dimethylaminomethoxymethoxy group, a dimethylaminoethoxyethoxy group, a dimethylaminoethoxypropyloxy group, a diethylaminoethoxypropyloxy group, a 4-(2'-diisobutylaminopropyloxy)butyloxy group and the like; and

alkoxy groups substituted with a alkylthio group such as a methylthiomethoxy group, a 2-methylthioethoxy group, a 2-ethylthioethoxy group, a 2-n-propylthioethoxy group, a 2-isopropylthioethoxy group, a 2-n-butylthioethoxy group, a 2-isobutylthioethoxy group, a (3,5,5-trimethylhexylthio)hexyloxy group and the like.

The substituted or unsubstituted aralkyloxy group as the substituent in the substituted amino group is an aralkyloxy group optionally substituted with the above-mentioned alkyl group(s) or an aralkyloxy group optionally substituted with substituent (s) like those which the above-mentioned alkyl groups may have. The specific examples include a benzyloxy group, a 4-nitrobenzyloxy group, a 4-cyanobenzyloxy group, a 4-hydroxybenzyloxy group, a 2-methylbenzyloxy group, a 3-methylbenzyloxy group, a 4-methylbenzyloxy group, a 4-trifluoromethylbenzyloxy group, a 1-naphthylmethoxy group, a 2-naphthylmethoxy group, a 4-cyano-1-naphthylmethoxy group, a 4-hydroxy-1-naphthylmethoxy group, a 6-hydroxy-2-naphthylmethoxy group, a 4-methyl-1-naphthylmethoxy group, a 6-methyl-2-naphthylmethoxy group, a 4-trifluromethyl-1-naphthylmethoxy group, a fluoren-9-ylethoxy group and the like.

The substituted or unsubstituted aryloxy group as the substituent in the substituted amino group is an aryloxy

group optionally substituted with the above-mentioned alkyl group(s) or an aryloxy group optionally substituted with substituent(s) like those which the above-mentioned alkyl groups may have. The specific examples include substituted or unsubstituted carbocyclic aryloxy groups such as a phenoxy group, a 4-methylphenoxy group, a 3-methylphenoxy group, a 2-methylphenoxy group, a 4-ethylphenoxy group, a 3-ethylpehnoxy group, a 2-ethylphenoxy group, a 4-n-propylphenoxy group, a 4-isopropylphenoxy group, a 2-isopropylphenoxy group, a 4-n-butylphenoxy group, a 4-isobutylphenoxy group, a 4-sec-butylphenoxy group, a 2-sec-butylphenoxy group, a 4-tert-butylphenoxy group, a 3-tert-butylphenoxy group, a 2-tert-butylphenoxy group, a 4-n-pentylphenoxy group, a 4-isopentylphenoxy group, a 4-neopentylphenoxy group, a 4-tert-pentylphenoxy group, a 4-n-hexylphenoxy group, a 4-(2'-ethylbutyl)phenoxy group, a 4-n-heptylphenoxy group, a 4-n-octylphenoxy group, a 4-(2'-ethylhexyl)phenoxy group, a 4-n-nonylphenoxy group, a 4-n-decylphenoxy group, a 4-n-undecylphenoxy group, a 4-n-dodecylphenoxy group, a 4-n-tetradecylphenoxy group, a 4-cyclohexylphenoxy group, a 4-(4'-methylcyclohexyl)phenoxy group, a 4-(4'-tert-butylcyclohexyl)phenoxy group, a 3-cyclohexylphenoxy group, a 2-cyclohexylphenoxy group, a 2,3-dimethylphenoxy group, a 2,4-dimethylphenoxy group, a 2,5-dimethylphenoxy group, a 2,6-dimethylphenoxy group, a 3,4-dimethylphenoxy group, a 3,5-dimethylphenoxy group, a 3,4,5-trimethylphenoxy group, a 2,3,5,6-tetramethylphenoxy group, a 2,4-diethylphenoxy group, a 2,6-diethylphenoxy group, a 2,5-diisopropylphenoxy group, a 2, 6-diisopropylphenoxy group, a 2,6-diisobutylphenoxy group, a 2,4-di-tert-butylphenoxy group, a 2,5-di-tert-butylphenoxy group, a 4,6-di-tert-butyl-2-methylphenoxy group, a 5-tert-butyl-2-methylphenoxy group, a 4-tert-butyl-2,6-dimethylphenoxy group, a 1-naphthyloxy group, a 2-naphthyloxy group, a 1,2,3,4-tetrahydro-5-naphthyloxy group, a 1,2,3,4-tetrahydro-6-naphthyloxy group, a 4-ethyl-1-naphthyloxy group, a 6-n-butyl-2-naphthyloxy group, a 5-indanyloxy group, a 4-methoxyphenoxy group, a 3-methoxyphenoxy group, a 2-methoxyphenoxy group, a 4-ethoxyphenoxy group, a 3-ethoxyphenoxy group, a 2-ethoxyphenoxy group, a 4-n-propyloxyphenoxy group, a 3-n-propyloxyphenoxy group, a 4-isopropyloxyphenoxy group, a 2-isopropyloxyphenoxy group, a 4-n-butyloxyphenoxy group, a 4-isobutyloxyphenoxy group, a 2-sec-butyloxyphenoxy group, a 4-n-pentyloxyphenoxy group, a 4-isopentyloxyphenoxy group, a 2-isopentyloxyphenoxy group, a 4-neopentyloxyphenoxy group, a 2-neopentyloxyphenoxy group, a 4-n-hexyloxyphenoxy group, a 4-(2'-ethylbutyl)oxyphenoxy group, a 4-n-heptyloxyphenoxy group, a 4-n-octyloxyphenoxy group, a 4-n-nonyloxyphenoxy group, a 4-n-decyloxyphenoxy group, a 4-n-undecyloxyphenoxy group, a 4-n-dodecyloxyphenoxy group, a 4-n-tetradecyloxyphenoxy group, a 4-cyclohexyloxyphenoxy group, a 2-cyclohexyloxyphenoxy group, a 2,3-dimethoxyphenoxy group, a 2,4-dimethoxyphenoxy group, a 2,5-dimethoxyphenoxy group, a 3,4-dimethoxyphenoxy group, a 3,5-dimethoxyphenoxy group, a 3,5-diethoxyphenoxy group, a 2-methoxy-4-methylphenoxy group, a 2-methoxy-5-methylphenoxy group, a 2-methyl-4-methoxyphenoxy group, a 3-methyl-4-methoxyphenoxy group, a 3-methyl-5-methoxyphenoxy group, a 2-methoxy-1-naphthyloxy group, a 4-methoxy-1-naphthyloxy group, a 4-n-butyloxy-1-naphthyloxy group, a 5-ethoxy-1-naphthyloxy group, a 6-methoxy-2-naphthyloxy group, a 6-ethoxy-2-naphthyloxy group, a 6-n-butyloxy-2-naphthyloxy group, a 6-n-hexyloxy-2-naphthyloxy group, a 7-methoxy-2-naphthyloxy group, a 7-n-butyloxy-2-naphthyloxy group, a 4-phenylphenoxy group, a 3-phenylphenoxy group, a 2-phenylphenoxy group, a 4-(4'-methylphenyl)phenoxy group, a 4-(3'-methylphenyl)phenoxy group, a 4-(4'-ethylphenyl)phenoxy group, a 4-(4'-isopropylphenyl)phenoxy group, a 4-(4'-tert-butylphenyl)phenoxy group, a 4-(4'-n-hexylphenyl)phenoxy group, a 4-(4'-n-octylphenyl)phenoxy group, a 4-(4'-methoxyphenyl)phenoxy group, a 4-(4'-n-butyloxyphenyl)phenoxy group, a 2-(2'-methoxyphenyl)phenoxy group, 4-(4'-chlorophenyl)phenoxy group, a 3-methyl-4-phenylphenoxy group, a 3-methoxy-4-phenylphenoxy group, a 9-phenyl-2-fluorenyloxy group, a 9,9-diphenyl-2-fluorenyloxy group, a 9-methyl-9-phenyl-2-fluorenyloxy group, a 9-ethyl-9-phenyl-2-fluorenyloxy group, a 4-fluorophenoxy group, a 3-fluorophenoxy group, a 2-fluorophenoxy group, a 4-chlorophenoxy group, a 3-chlorophenoxy group, a 2-chlorophenoxy group, a 4-bromophenoxy group, a 2-bromophenoxy group, a 4-trifluoromethylphenoxy group, a 2,3-difluorophenoxy group, a 2,4-difluorophenoxy group, a 2,5-difluorophenoxy group, a 2,6-difluorophenoxy group, a 3,4-difluorophenoxy group, a 3,5-difluorophenoxy group, a 2,3-dichlorophenoxy group, a 2,4-dichlorophenoxy group, a 2,5-dichlorophenoxy group, a 3,4-dichlorophenoxy group, a 3,5-dichlorophenoxy group, a 2,5-dibromophenoxy group, a 2,4,6-trichlorophenoxy group, a 2-fluoro-4-methylphenoxy group, a 2-fluoro-5-methylphenoxy group, a 3-fluoro-2-methylphenoxy group, a 3-fluoro-4-methylphenoxy group, a 2-methyl-4-fluorophenoxy group, a 2-methyl-5-fluorophenoxy group, a 3-methyl-4-fluorophenoxy group, a 2-chloro-4-methylphenoxy group, a 2-chloro-5-methylphenoxy group, a 2-chloro-6-methylphenoxy group, a 3-chloro-4-methylphenoxy group, a 2-methyl-3-chlorophenoxy group, a 2-methyl-4-chlorophenoxy group, a 3-methyl-4-chlorophenoxy group, a 2-chloro-4,6-dimethylphenoxy group, a 2,4-dichloro-1-naphthyloxy group, a 1,6-dichloro-2-naphthyloxy group, a 2-methoxy-4-fluorophenoxy group, a 3-methoxy-4-fluorophenoxy group, a 2-fluoro-4-methoxyphenoxy group, a 2-fluoro-4-ethoxyphenoxy group, a 2-fluoro-6-methoxyphenoxy group, a 3-fluoro-4-methoxyphenoxy group, a 3-fluoro-4-ethoxyphenoxy group, a 2-chloro-4-methoxyphenoxy group, a 3-chloro-4-methoxyphenoxy group, a 2-methoxy-5-chlorophenoxy group, a 3-methoxy-4-chlorophenoxy group, a 3-methoxy-6-chlorophenoxy group, a 5-chloro-2,4-dimethoxyphenoxy group, a 2-hydroxyphenoxy group, a 3-hydroxyphenoxy group, a 4-hydroxyphenoxy group, a 2-nitrophenoxy group, a 3-nitrophenoxy group, a 4-nitrophenoxy group, a 2-cyanophenoxy group, a 3-cyanophenoxy group, a 4-cyanophenoxy group, a 2-methyl-5-nitrophenoxy group, a 3, 5-dinitrophenoxy group, a 2-hydroxy-4-nitrophenoxy group and the like;

substituted or unsubstituted heterocyclic aryloxy groups such as a 4-pyridyloxy group, a 3-pyridyloxy group, a 2-pyridyloxy group, a 4-methyl-2-pyridyloxy group, a 5-methyl-2-pyridyloxy group, a 6-methyl-2-pyridyloxy group, a 4,6-dimethyl-2-pyridyloxy group, a 4-methyl-5-nitro-2-pyridyloxy group, a 3-hydroxy-2-pyridyloxy group, a 6-fluoro-3-pyridyloxy group, a 6-methoxy-3-pyridyloxy group, a 6-methoxy-2-pyridyloxy group, a 2-pyrimidyloxy group, a 3-pyrimidyloxy group, a 4-pyrimidyloxy group, a 2,6-dimethyl-4-pyrimidyloxy group, a 4-quinolyloxy group, a 3-quinolyloxy group, a 4-methyl-2-quinolyloxy group, a 3-furyloxy group, a 2-furyloxy group, a 3-thienyloxy group, a 2-thienyloxy group, a 4-methyl-3-thienyloxy group, a 5-methyl-2-thienyloxy group, a 3-methyl-2-thienyloxy group, a 2-oxazolyloxy group, a 2-thiazolyloxy group, a 2-thiadiazolyloxy group, a 2-benzoxazolyloxy group, a 2-benzothiazolyloxy group, a 2-benzimidazolyloxy group and the like;
and the like.

The substituted or unsubstituted alkenyloxy group as the substituent in the substituted amino group is an alkenyloxy group optionally substituted with the above-mentioned alkyl group(s) or an alkenyloxy group optionally substituted with substituent (s) like those which the above-mentioned alkyl groups may have. The specific examples include a vinyloxy group, a propenyloxy group, a 1-butenyloxy group, an isobutenyloxy group, a 1-pentenyloxy group, a 2-pentenyloxy group, a 2-methyl-1-butenyloxy group, a 3-methyl-1-butenyloxy group, a 2-methyl-2-butenyloxy group, a cyclopentadienyloxy group, a 2,2-dicyanovinyloxy group, a 2-cyano-2-methylcarboxyvinyloxy group, a 2-cyano-2-methylsulfonevinyloxy group, a styryloxy group, a 4-phenyl-2-butenyloxy group, cinnamyloxy group and the like.

The substituted or unsubstituted alkylthio group as the substituent in the substituted amino group is an alkylthio group optionally substituted with substituent (s) like those which the above-mentioned alkyl groups may have. The specific examples include a methylthio group, an ethylthio group, an n-propylthio group, an isopropylthio group, an n-butylthio group, an isobutylthio group, a sec-butylthio group, a tert-butylthio group, an n-pentylthio group, an isopentylthio group, a neopentylthio group, a 2-methylbutylthio group, a methylcarboxyethylthio group, a 2-ethylhexylthio group, a 3,5,5-trimethylhexylthio group, a decalylthio group and the like.

The substituted or unsubstituted aralkylthio group as the substituent in the substituted amino group is an aralkylthio group optionally substituted with the above-mentioned alkyl group(s) or an aralkylthio group optionally substituted with substituent (s) like those which the above-mentioned alkyl groups may have. The specific examples include a benzylthio group, a 4-cyanobenzylthio group, a 4-hydroxybenzylthio group, a 2-methylbenzylthio group, a 3-methylbenzylthio group, a 4-methylbenzylthio group, a 4-trifluoromethylbenzylthio group, a 1-naphthylmethylthio group, a 4-nitro-1-naphthylmethylthio group, a 4-cyano-1-naphthylmethylthio group, a 4-hydroxy-1-naphthylmethylthio group, a 4-methyl-1-naphthylmethylthio group, a 4-trifluoromethyl-1-naphthylmethylthio group, a fluoren-9-ylethylthio group and the like.

The substituted or unsubstituted arylthio group as the substituent in the substituted amino group is an arylthio group optionally substituted with the above-mentioned alkyl group(s) or an arylthio group optionally substituted with substituent (s) like those which the above-mentioned alkyl groups may have. The specific examples include a phenylthio group, a 4-mehylphenylthio group, a 2-methoxyphenylthio group, a 4-tert-butylphenylthio group, a naphthylthio group and the like.

The substituted or unsubstituted alkenylthio group as the substituent in the substituted amino group is an alkenylthio group optionally substituted with the above-mentioned alkyl group(s) or an alkenylthio group optionally substituted with substituent (s) like those which the above-mentioned alkyl groups may have. The specific examples include a vinylthio group, a propenylthio group, a 1-butenylthio group, an isobutenylthio group, a 1-pentenylthio group, a 2-pentenylthio group, a 2-methyl-1-butenylthio group, a 3-methyl-1-butenylthio group, a 2-methyl-2-butenylthio group, a cyclopentadienylthio group, a 2,2-dicyanovinylthio group, a 2-cyano-2-methylcarboxyvinylthio group, a 2-cyano-2-methylsulfonevinylthio group, a styrylthio group, a 4-phenyl-2-butenylthio group, a cinnamyloxy group and the like.

The substituted or unsubstituted acyl group as the substituent in the substituted amino group is an acyl group optionally substituted with the above-mentioned alkyl group(s) or an acyl group optionally substituted with substituent (s) like those which the above-mentioned alkyl groups may have. The specific examples include a formyl group, a methylcarbonyl group, an ethylcarbonyl group, an n-propylcarbonyl group, an isopropylcarbonyl group, an n-butylcarbonyl group, an isobutylcarbonyl group, a sec-butylcarbonyl group, a tert-butylcarbonyl group, an n-pentylcarbonyl group, an isopentylcarbonyl group, a neopentylcarbonyl group, a 2-methylbutylcarbonyl group, a benzoyl group, a 2-methylbenzoyl group, a 3-methylbenzoyl group, a 4-methylbenzoyl group, a 4-ethylbenzoyl group, a 4-n-propylbenzoyl group, a 4-tert-butylbenzoyl group, a 4-nitrobenzylcarbonyl group, a 3-n-butoxy-2-naphthoyl group, a cinnamoyl group and the like.

The substituted or unsubstituted acyloxy group as the substituent in the substituted amino group is an acyloxy group optionally substituted with the above-mentioned alkyl group(s) or an acyloxy group optionally substituted with substituent(s) like those which the above-mentioned alkyl groups may have. The specific examples include a formyloxy group, a methylcarbonyloxy group, an ethylcarbonyloxy group, an n-propylcarbonyloxy group, an isopropylcarbonyloxy group, an n-butylcarbonyloxy group, an isobutylcarbonyloxy group, a sec-butylcarbonyloxy group, a tert-butylcarbonyloxy group, an n-pentylcarbonyloxy group, an isopentylcarbonyloxy group, a neopentylcarbonyloxy group, a 2-methylbutylcarbonyloxy group, a benzoyloxy group, a 2-methylbenzoyloxy group, a 3-methylbenzoyloxy group, a 4-methylbenzoyloxy group, a 4-ethylbenzoyloxy group, 4-n-propylbenzoyloxy group, a 4-tert-butylbenzoyloxy group, a 4-nitrobenzylcarbonyloxy group, 3-n-butoxy-2-naphthoyloxy group, a cinnamoyloxy group and the like.

The substituted or unsubstituted alkoxycarbonyl group as the substituent in the substituted amino group is an alkoxycarbonyl group optionally substituted with substituent(s) like those which the above-mentioned alkyl groups may have. The specific examples include alkoxylcarbonyl groups such as a methoxycarbonyl group, an ethoxycarbonyl group, an n-propyloxycarbonyl group, an isopropyloxycarbonyl group, an n-butyloxycarbonyl group, an isobutyloxycarbonyl group, a sec-butyloxycarbonyl group, a tert-butyloxycarbonyl group, an n-pentyloxycarbonyl group, an isopentyloxycarbonyl group, a neopentyloxycarbonyl group, a 2-ethylhexyloxycarbonyl group, a 3,5,5-trimethylhexyloxycarbonyl group, a decalyloxycarbonyl group, a cyclohexyloxycarbonyl group, a 2-chloroethoxycarbonyl group, a hydroxymethoxycarbonyl group, a 2-hydroxyethoxycarbonyl group and the like;

alkoxycarbonyl groups substituted with an alkoxy group such as a methoxymethoxycarbonyl group, a methoxyethoxycarbonyl group, an ethoxyethoxycarbonyl group, an n-propyloxyethoxycarbonyl group, an n-butyloxyethoxycarbonyl group, an n-pentyloxyethoxycarbonyl group, an n-hexyloxyethoxyethyl group, an n-butyloxybutyloxycarbonyl group, an n-hexyloxybutyloxycarbonyl group, a hydroxymethoxymethoxycarbonyl group, a hydroxyethoxyethoxycarbonyl group and the like;
alkoxycarbonyl groups substituted with an alkoxyalkoxy group such as a methoxymethoxymethoxycarbonyl group, a methoxyethoxyethoxycarbonyl group, an ethoxyethoxyethoxycarbonyl group, an n-propyloxyethoxyethoxycarbonyl group, an n-butyloxyethoxyethoxycarbonyl group, an n-pentyloxyethoxyethoxycarbonyl group, an n-hexyloxyethoxyethoxycarbonyl group and the like;
and the like.

The substituted or unsubstituted aralkyloxycarbonyl group as the substituent in the substituted amino group is an aralkyloxycarbonyl group optionally substituted with the above-mentioned alkyl group(s) or an aralkyloxycarbonyl group optionally substituted with substituent(s) like those which the above-mentioned alkyl groups may have. The specific examples include a benzyloxycarbonyl group, a 4-nitrobenzyloxycarbonyl group, a 4-cyanobenzyloxycarbonyl group, a 4-hydroxybenzyloxycarbonyl group, a 2-methylbenzyloxycarbonyl group, a 3-methylbenzyloxycarbonyl group, a 4-methylbenzyloxycarbonyl group, a 4-trifluoromethylbenzyloxycarbonyl group, a 1-naphthylmethoxycarbonyl group, a 2-naphthylmethoxycarbonyl group, a 4-cyano-1-naphthylmethoxycarbonyl group, a 4-hydroxy-1-naphthylmethoxycarbonyl group, a 6-hydroxy-2-naphthylmethoxycarbonyl group, a 4-methyl-1-naphthylmethoxycarbonyl group, a 6-methyl-2-naphthylmethoxycarbonyl group, a 4-trifluoromethyl-1-naphthyloxymethoxycarbonyl group, a fluoren-9-ylethoxycarbonyl group and the like.

The substituted or unsubstituted aryloxycarbonyl group as the substituent in the substituted amino group is an aryloxycarbonyl group optionally substituted with the above-mentioned alkyl group(s) or an aryloxycarbonyl group optionally substituted with substituent (s) like those which the above-mentioned alkyl groups may have. The specific examples include a phenyloxycarbonyl group, a 2-methylphenyloxycarbonyl group, a 4-methylphenyloxycarbonyl group, a 4-tert-butylphenyloxycarbonyl group, a 2-methoxyphenyloxycarbonyl group, a 4-isopropylphenyloxycarbonyl group, a naphthyloxycarbonyl group and the like.

The substituted or unsubstituted alkenyloxycarbonyl group as the substituent in the substituted amino group is an alkenyloxycarbonyl group optionally substituted with the above-mentioned alkyl group(s) or an alkenyloxycarbonyl group optionally substituted with substituent (s) like those which the above-mentioned alkyl groups may have. The specific examples include a vinyloxycarbonyl group, a propenyloxycarbonyl group, a 1-butenyloxycarbonyl group, an isobutenyloxycarbonyl group, a 1-pentenyloxycarbonyl group, a 2-pentenyloxycarbonyl group, a cyclopentadienyloxycarbonyl group, a 2-methyl-l-butenyloxycarbonyl group, a 3-methyl-l-butenyloxycarbonyl group, a 2-methyl-2-butenyloxycarbonyl group, a 2,2-dicyanovinyloxycarbonyl group, a 2-cyano-2-methylcarboxyvinyloxycarbonyl group, a 2-cyano-2-methylsulfonevinyloxycarbonyl group, a styryloxycarbonyl group, a 4-phenyl-2-butenyloxycarbonyl group and the like.

The substituted or unsubstituted amino group as the substituent in the substituted amino group is an amino group optionally substituted with the above-mentioned alkyl group(s) or an alkylamino group optionally substituted with substituent (s) like those which the above-mentioned alkyl groups may have. The specific examples include, monoalkylamino groups such as an amino group a methylamino group, an ethylamino group, a propylamino group, a butylamino group, a pentylamino group, a hexylamino group, a heptylamino group, an octylamino group, a 2-ethylhexylamino group, a cyclohexylamino group, a 3,5,5-trimethylhexylamino group, a nonylamino group, a decylamino group and the like, dialkylamino groups such as a dimethylamino group, a diethylamino group, a methylethylamino group, a dibutylamino group, a piperidino group, a morpholino group, a di(acetyloxyethyl)amino group, a di(propionyloxyethyl) amino group and the like; aralkylamino groups optionally substituted with the above-mentioned alkyl group(s) or aralkylamino groups optionally substituted with substituent (s) like those which the above-mentioned alkyl groups may have, for example, monoaralkylamino groups such as a benzylamino group, a phenethylamino group, a 3-phenylpropylamino group, a 4-ethylbenzylamino group, a 4-isopropylbenzylamino group and the like, and diaralkylamino groups such as a dibenzylamino group, a diphenethylamino group, a bis(4-ethylbenzyl)amino group, a bis(4-isopropylbenzyl) amino group and the like; arylamino groups optionally substituted with the above-mentioned alkyl group(s) or arylamino groups optionally substituted with substituent(s) like those which the above-mentioned alkyl groups may have, for example, monoarylamino groups such as a phenylamino group, a 1-naphthylamino group, a 2-naphthylamino group, a 2-methylphenylamino group, a 3-methylphenylamino group, a 4-methylphenylamino group, a 2,4-dimethylphenylamino group, a 2,6-dimethylphenylamino group, a 4-ethylphenylamino group, a 4-isopropylphenylamino group, a 4-methoxyphenylamino group, a 4-chlorophenylamino group, a 4-acetylphenylamio group, a 4-methoxycarbonylphenylamino group, 4-ethoxycarbonylphenylamino group, 4-propyloxycarbonylphenylamino group and the like, and diarylamino groups such as an N,N-diphenylamino group, an N, N-di(3-methylphenyl)amino group, an N,N-di(4-methylphenyl)amino group, an N,N-di(4-ethylphenyl)amino group, an N,N-di(4-tert-butylphenyl)amino group, an N,N-di(4-n-hexylphenyl)amino group, an N,N-di(4-methoxyphenyl)amino group, an N,N-di(4-ethoxyphenyl)amino group, an N,N-di(4-n-butyloxyphenyl)amino group, an N,N-di(4-n-hexyloxyphenyl)amino group, an N,N-di(1-naphthyl)amino group, an N,N-di(2-naphthyl)amino group, an N-phenyl-N-(3-methylphenyl)amino group, an N-phenyl-N-(4-methylphenyl)amino group, an N-phenyl-N-(4-n-octylphenyl)amino group, an N-phenyl-N-(4-methoxyphenyl)amino group, an N-phenyl-N-(4-ethoxyphenyl)amino group, an N-phenyl-N-(4-n-hexyloxyphenyl)amino group, an N-phenyl-N-(4-fluorophenyl)amino group, an N-phenyl-N-(1-naphthyl)amino group, an N-phenyl-N-(2-naphthyl)amino group, an N-phenyl-N-(4-phenylphenyl)amino group and the like;
and the like.

The group having a metallocenyl residue as the substituent in the substituted amino group is a group containing a linkage group and a substituted or unsubstituted metallocenyl group, and it is preferably a group represented by the following general formula (a):

[Formula 9]

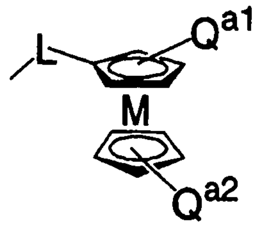

(a)

(wherein L represents a linkage group, $Q^{a1}$ and $Q^{a2}$ each represent independently a hydrogen atom, a halogen atom, a substituted or unsubstituted alkyl group, a substituted or unsubstituted alkoxy group, a substituted or unsubstituted aryl group or a substituted or unsubstituted phosphino group, and M represents a divalent transition metal.)

In the group represented by general formula (a), as examples of the halogen atom, the substituted or unsubstituted alkyl group, the substituted or unsubstituted alkoxy group or the substituted or unsubstituted aryl group represented by $Q^{a1}$ or $Q^{a2}$ includes those like the above-mentioned substituted or unsubstituted alkyl groups, the above-mentioned substituted or unsubstituted alkoxy groups and the above-mentioned substituted or unsubstituted aryl groups.

Further, the substituted or unsubstituted phosphino group represented by $Q^{a1}$ or $Q^{a2}$ is a phosphino group optionally substituted with substituent(s) like those which the above-mentioned alkyl groups may have. The specific examples include dialkylphosphino groups such as a dimethylphosphino group, a diethylphosphino group, a dipropylphosphino group, a dibutylphosphino group, a dipentylphosphino group, a dihexylphosphino group and the like; an alkylarylphosphino groups such as a P-methyl-P-phenylphosphino group and the like; diarylphosphino groups such as a diphenylphosphino group, a ditolylphosphino group, a dimesitylphosphino group, a di(t-butylphenyl)phosphino group, a phenyl-3,5-xylylphosphino group and the like; and the like.

In the group represented by general formula (a), M represents a divalent transition metal. The specific examples include Fe, Co, Ni, Ru, Os, Mn, Cr, W, V, Sc, Y, La, Ce, Pr, Nd, Sm, Gd, Er, Tm, Yb and the like. Fe is particularly preferred.

In the group represented by general formula (a), the linkage group represented by L is a group wherein atoms selected from a carbon atom, a heteroatom such as a nitrogen atom, an oxygen atom, a sulfur atom, a phosphorus atom, a metal atom, a metalloid atom and a hydrogen atom, are properly combined and bonded through one or more bonds properly selected from a single bond, a double bond and/or a triple bond, to form a linkage.

The preferred examples of the linkage group L include a group represented by one of the following formula (b):

[Formula 10]

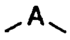 , 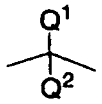 , 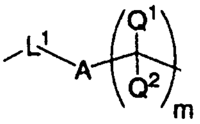 , 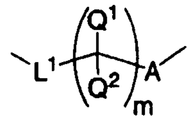 , 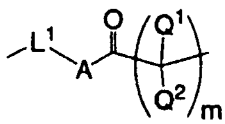

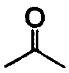 , 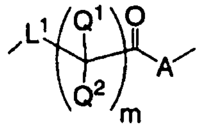 , 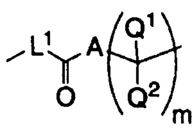 , 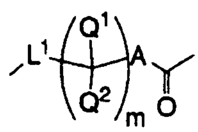 , 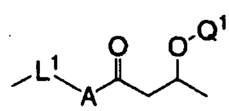 (b)

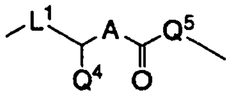 , 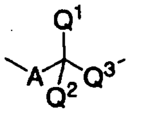 , 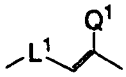 , 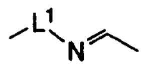

[wherein A represents a group represented by -O-, -S- or -N ($Q^6$) -; $L^1$ represents a single bond, a substituted or unsubstituted divalent aliphatic hydrocarbon group or a substituted or unsubstituted divalent aromatic cyclic group, $Q^1$, $Q^2$ and $Q^6$ each represent independently a hydrogen atom or a substituted or unsubstituted alkyl group, $Q^3$ represents a group represented by -O-$Q^7$-, -C (=O)-O-$Q^7$- or -O-C (=O)-$Q^7$-, $Q^7$ represents a single bond or a substituted or unsubstituted divalent aliphatic hydrocarbon group, $Q^4$ represents a hydrogen atom or a methyl group, $Q^5$ represents a group represented by $-CH_2-$, $-CH_2CH_2-$, -CH=CH-, $-CH_2-C(=O)$ - or $-CH_2CH_2-C(=O)-$, and m is an integer from 0 to 4.]

In the above formula, the substituted or unsubstituted divalent aliphatic hydrocarbon group represented by $L^1$ is a divalent aliphatic hydrocarbon group optionally substituted with the above-mentioned alkyl groups or a divalent aliphatic hydrocarbon group optionally substituted with substituent(s) like those which the above-mentioned alkyl groups may have. The specific examples include alkylene groups such as a methylene group, an ethylene group, a trimethylene group, a tetramethylene group, a pentamethylene group, a cyclopentylene group, a hexamethylene group, a cyclohexylene group, a heptamethylene group, an octamethylene group, a nonamethylene group, a decamethylene group, an undecamethylene group, a dodecamethylene group, a tridecamethylene group, a tetradecamethylene group, a pentadecamethylene group and the like,
alkenylene groups such as a vinylene group, a propenylene group, a 1-butenylene group, a 1-pentenylene group, a 2-pentenylene group, a decanylene group and the like; and the like.

In the above formula, the substituted or unsubstituted divalent aromatic cyclic group represented by $L^1$ is a divalent aromatic cyclic group optionally substituted with the above-mentioned alkyl group(s) or a divalent aromatic cyclic group optionally substituted with substituent(s) like those which the above-mentioned alkyl groups may have. The specific examples include aromatic hydrocarbon groups such as a phenylene group, a naphthylene group, an indenylene group, an anthracenylene group, a fluorenylene group an azulenylene group, a naphthacenylene group, a chrycenylene group, a pyrenylene group, a perylenylene group and the like;
heterocyclic groups such as a furanylene group, a pyrrolylene group, a 3-pyrrolinylene group, a pyrrolidinylene group, a 1, 3-oxolanylene group, a pyrazolylene group, a 2-pyrazolinylene group, a pyrazolidinylene group, an imidazolylene group, an oxazolylene group, a thiazolylene group, a 1,2,3-oxadiazolylene group, a 1,2,3-triazolylene group, a 1,2,4-triazolylene group, a 1,3,4-thiadiazolylene group, a 4H-pyranylene group, a pyridinylene group, a piperidinylene group, a dioxanylene group, a morpholinylene group, a pyridazinylene group, a pyrimidinylene group, a pyrazinylene group, a piperadinylene group, a triazinylene group, a benzofuranylene group, an indolylene group, a thionaphthenylene group, a benzimidazolylene group, a benzothiazolylene group, a purinylene group, a quiolinylene group, an isoquinolylene group, a coumarinylene group, a cinnolinylene group, a quinoxalinylene group, a dibenzofuranylene group, a carbazolylene group, a phenanthrolinylene group, a phenothiazinylene group, a flavonylene group, perimidylene group and the like; and the like.

The examples of the substituted or unsubstituted alkyl groups represented by $Q^1$, $Q^2$ or $Q^6$ include groups like the above-mentioned substituted or unsubstituted alkyl groups. The specific examples include alkyl groups having 1 to 4 carbon atoms such as a methyl group, an ethyl group, an n-propyl group, an isopropyl group, an n-butyl group, an isobutyl group, a sec-butyl group, a tert-butyl group and the like.

The examples of the substituted or unsubstituted divalent aliphatic hydrocarbon groups represented by $Q^7$ include groups like the above-mentioned substituted or unsubstituted divalent aliphatic hydrocarbon groups. The specific

examples include the above-mentioned alkylene groups having 1 to 4 carbon atoms and alkenylene groups having 2 to 4 carbon atoms such as a vinylene group, a propylenylene group, a 1-butenylene group and the like.

The substituted amino group which bonds to organic compound (B) relating to the present invention may be either a monosubstituted amino group containing one of the above substituents bonded or a disubstituted amino group containing two of the above-mentioned substituents bonded. In the case of disubstituted amino group, the two substituents may bond via a single bond or a linkage group to form a ring structure together with the nitrogen atom to which they bond. The examples of the linkage group include linkage groups like the above-mentioned linkage group L.

The number of six-membered ring structures and that of amino groups which organic compound (B) relating to the present invention contains are not particularly limited and it is usually 1 to 4, preferably 1 to 2, and more preferably one, respectively.

The preferred examples of organic compound (B) relating to the present invention, more specifically include a compound one of whose tautomeric structures is represented by the following general formula (0):

[Formula 11]

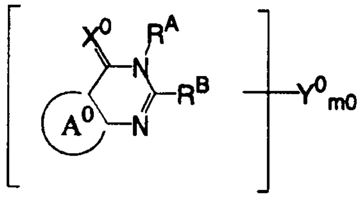

(0)

(wherein ring $A^0$ represents a substituted or unsubstituted carbocyclic aromatic ring or a substituted or unsubstituted heterocyclic aromatic ring; $R^A$ and $R^B$ represent a hydrogen atom or a substituent; $X^0$ represents a divalent substituent; $Y^0$ represents a substituted or unsubstituted amino group; and $m^0$ represents the number of $Y^0$.)

In compounds represented by general formula (0), $A^0$ represents a substituted or unsubstituted carbocyclic aromatic ring or a substituted or unsubstituted heterocyclic aromatic ring.

In compounds represented by general formula (0), $A^0$ is preferably a substituted or unsubstituted carbocyclic aromatic ring having 6-26 carbon atoms or a substituted or unsubstituted heterocyclic aromatic ring having 3-26 carbon atoms. More preferably it is a substituted or unsubstituted carbocyclic aromatic ring having 6-20 carbon atoms or a substituted or unsubstituted heterocyclic aromatic ring having 3-20 carbon atoms.

The specific examples of the aromatic ring represented by A° include carbocyclic aromatic rings such as a substituted or unsubstituted benzene ring, a substituted or unsubstituted naphthalene ring, a substituted or unsubstituted pentalene ring, a substituted or unsubstituted indacene ring, a substituted or unsubstituted azulene ring, a substituted or unsubstituted heptalene ring, a substituted or unsubstituted biphenylene ring, a substituted or unsubstituted phenanthrene ring, a substituted or unsubstituted anthracene ring, a substituted or unsubstituted fluoranthene ring, a substituted or unsubstituted acenaphthylene ring, a substituted or unsubstituted triphenylene ring, a substituted or unsubstituted pyrene ring, a substituted or unsubstituted chrycene ring, a substituted or unsubstituted naphthacene ring, a substituted or unsubstituted pleiadene ring, a substituted or unsubstituted picene ring, a substituted or unsubstituted perylene ring, a substituted or unsubstituted pentaphene ring, a substituted or unsubstituted pentacene ring, a substituted or unsubstituted tetraphenylene ring, a substituted or unsubstituted hexaphene ring, a substituted or unsubstituted hexacene ring, a substituted or unsubstituted rubicene ring, a substituted or unsubstituted coronene ring, a substituted or unsubstituted trinaphthylene ring, a substituted or unsubstituted heptaphene ring, a substituted or unsubstituted heptacene ring, a substituted or unsubstituted pyranthrene ring, a substituted or unsubstituted ovalene ring and the like;

heterocyclic aromatic rings such as a substituted or unsubstituted furan ring, a substituted or unsubstituted thiophene ring, a substituted or unsubstituted pyrrole ring, a substituted or unsubstituted pyrazole ring, a substituted or unsubstituted imidazole ring, a substituted or unsubstituted oxazole ring, a substituted or unsubstituted thiazole ring, a substituted or unsubstituted pyridine ring, a substituted or unsubstituted pyradazine ring, a substituted or unsubstituted pyrimidine ring, a substituted or unsubstituted pyrazine ring, a substituted or unsubstituted quinoline ring, a substituted or unsubstituted isoquinoline ring, a substituted or unsubstituted quinoxaline ring, a substituted or unsubstituted indolizine ring, a substituted or unsubstituted indole ring, a substituted or unsubstituted indazole ring, a substituted or unsubstituted purine ring, a substituted or unsubstituted phthalazine ring, a substituted or unsubstituted naphthyridine ring, a substituted or unsubstituted quinazoline ring, a substituted or unsubstituted cinnoline ring, a substituted or unsubstituted pteridine ring, a substituted or unsubstituted carboline ring, a substituted or unsubstituted phenanthridine ring, a substituted or unsubstituted acridine ring, a substituted or unsubstituted perimidine ring, a substituted or unsubstituted phenathroline ring, a

substituted or unsubstituted phenazine ring, a substituted or unsubstituted furazane ring and the like; and the like.

When ring $A^0$ has substituent(s), the substituent is a halogen atom, a nitro group, a cyano group, a hydroxyl group, a carboxyl group, a substituted or unsubstituted alkyl group, a substituted or unsubstituted aralkyl group, a substituted or unsubstituted aryl group, a substituted or unsubstituted metallocenyl group, a substituted or unsubstituted alkenyl group, a substituted or unsubstituted alkoxy group, a substituted or unsubstituted aralkyloxy group, a substituted or unsubstituted aryloxy group, a substituted or unsubstituted alkenyloxy group, a substituted or unsubstituted alkylthio group, a substituted or unsubstituted aralkylthio group, a substituted or unsubstituted arylthio group, a substituted or unsubstituted alkenylthio group, a substituted or unsubstituted acyl group, a substituted or unsubstituted acyloxy group, a substituted or unsubstituted alkoxycarbonyl group, a substituted or unsubstituted aralkyloxycarbonyl group, a substituted or unsubstituted aryloxycarbonyl group, a substituted or unsubstituted alkenyloxycarbonyl group, a substituted or unsubstituted amino group or a group having a metallocenyl residue, as mentioned above. Specific examples of each substituent are substituents like examples of the above-mentioned substituent in the substituted amino group.

In compounds represented by general formula (0), $R^A$ and $R^B$ represent a hydrogen atom or a substituent. The substituent represented by $R^A$ or $R^B$ is a halogen atom, a nitro group, a cyano group, a hydroxyl group, a carboxyl group, a substituted or unsubstituted alkyl group, a substituted or unsubstituted aralkyl group, a substituted or unsubstituted aryl group, a substituted or unsubstituted metallocenyl group, a substituted or unsubstituted alkenyl group, a substituted or unsubstituted alkoxy group, a substituted or unsubstituted aralkyloxy group, a substituted or unsubstituted aryloxy group, a substituted or unsubstituted alkenyloxy group, a substituted or unsubstituted alkylthio group, a substituted or unsubstituted aralkylthio group, a substituted or unsubstituted arylthio group, a substituted or unsubstituted alkenylthio group, a substituted or unsubstituted acyl group, a substituted or unsubstituted acyloxy group, a substituted or unsubstituted alkoxycarbonyl group, a substituted or unsubstituted aralkyloxycarbonyl group, a substituted or unsubstituted aryloxycarbonyl group, a substituted or unsubstituted alkenyloxycarbonyl group, a substituted or unsubstituted amino group or a group having a metallocenyl residue. Specific examples of each substituent are substituents like examples of the above-mentioned substituent in the substituted amino group.

In compounds represented by general formula (0), $X^0$ represents a divalent substituent. The examples of the divalent substituent represented by $X^0$ include a divalent atoms such as an oxygen atom and a sulfur atom, a substituted or unsubstituted imino group represented as $=N-R^{01}$, [wherein $R^{01}$ is a hydrogen atom or a substituent like examples of the above-mentioned substituent in the substituted amino group].

In compounds represented by general formula (0), $Y^0$ represents a substituted or unsubstituted amino group. The examples of the substituent in the substituted amino group represented by $Y^0$ include substituents like examples of the above-mentioned substituent in the substituted amino group.

In compounds represented by general formula (0), $m^0$ represents the number of $Y^0$, that is, the substituted or unsubstituted amino group represented by $Y^0$. $m^0$, which is an integer of 1 or more, is usually 1 to 4, preferably 1 or 2, more preferably 1. It is preferred that at least one substituted or unsubstituted amino group represented by $Y^0$ bonds to ring $A^0$ in general formula (0) as a substituent.

More preferably $Y^0$ is a substituted or unsubstituted amino group having 1 to 2 carbon atom(s) such as a dimethylamino group, a methylamino group, an ethylamino group and the like. A dimethylamino group is particularly preferred.

When the atom in $R^B$ in general formula (0) of the present invention, which directly bonds to the carbon atom having $R^B$ as a substituent, has hydrogen atom(s) or when $R^A$ in general formula (0) is a hydrogen atom, compounds represented by general formula (0) have tautomeric structures and can exist tautomers.

The further preferred examples of organic compound (B) relating to the present invention include compounds one of whose tautomeric structures is represented by the following general formula (1):

[Formula 12]

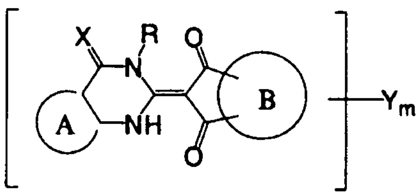

(1)

(wherein ring A and ring B represent a substituted or unsubstituted carbocyclic aromatic ring or a substituted or unsubstituted heterocyclic aromatic ring, R represents a hydrogen atom or a substituent, X represents a divalent substituent, Y represents a substituted or unsubstituted amino group and m represents the number of Y.)

In compounds represented by general formula (1), ring A and ring B each represents independently a substituted or unsubstituted carbocyclic aromatic ring or a substituted or unsubstituted heterocyclic aromatic ring.

The examples of the substituted or unsubstituted carbocyclic aromatic ring or the substituted or unsubstituted heterocyclic aromatic ring represented by ring A or ring B in general formula (1) include examples of the substituted or unsubstituted carbocyclic aromatic ring or substituted or unsubstituted heterocyclic aromatic ring as the above-mentioned ring $A^0$. When the ring has substituent (s), examples of the substituent are like those described for ring $A^0$.

In compounds represented by general formula (1), R represents a hydrogen atom or a substituent. The examples of the substituent represented by R include substituents like the above-mentioned examples of $R^A$ and $R^B$.

In compounds represented by general formula (1), X represents a divalent substituent. The examples of the divalent substituent represented by X include substituents like the .above-mentioned examples of $X^0$.

In compounds represented by general formula (1), Y represents a substituted or unsubstituted amino group. The examples of the substituted or unsubstituted amino group represented by Y include substituents like the above-mentioned examples of $Y^0$. It is preferred that at least one substituted or unsubstituted amino group represented by Y bonds to ring A in general formula (1) as a substituent. Further, more preferably, Y is a substituted or unsubstituted amino group having 1 or 2 carbon atom(s) such as a dimethylamino group, a methylamino group, an ethylamino group and the like. A dimethylamino group is particularly preferred.

In compounds represented by general formula (1), m represents the number of Y. The number of Y represented by m includes numbers described for the above-mentioned examples of $m^0$.

The compound of the present invention represented by general formula (1) has a structure capable of tautomerism and can have tautomers. Specifically, they are represented by the following general formula (1) and general formulae (101) to (103). Although the structure of general formula (1) is represented for convenience in the present invention, one can freely use compounds having structure represented by general formulae (101) to (103) or any mixture of tautomeric structures represented by general formula (1) and general formulae (101) to (103).

[Formula 13]

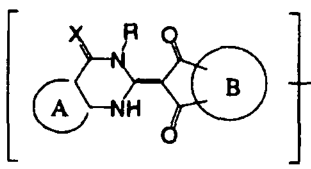 (1) 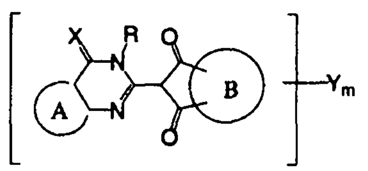 (101)

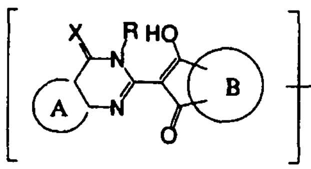 (102) 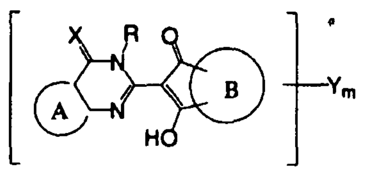 (103)

The compound of the present invention represented by general formula (1), when R is a hydrogen atom, may also exist as tautomeric structures represented by the following general formulae (104) to (106) besides the above-mentioned tautomeric structures.

[Formula 14]

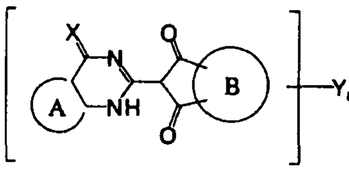 (104)

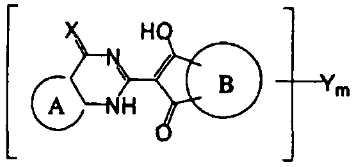 (105)

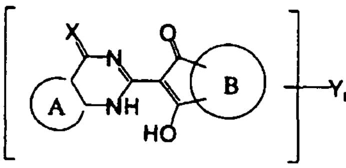 (106)

Furthermore, the compound of the present invention represented by general formula (104) to (106), when X is an oxygen atom or a sulfur atom, may also exist as tautomeric structures represented by the following general formulae (107) to (110) besides the above-mentioned tautomeric structures.

[Formula 15]

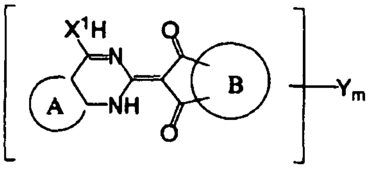 (107)

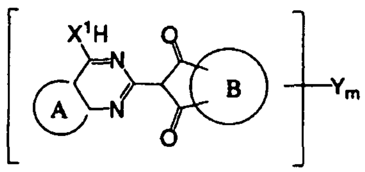 (108)

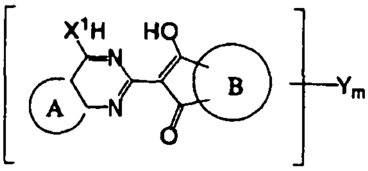 (109)

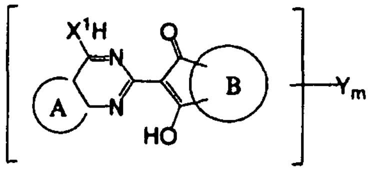 (110)

(wherein X1 represents an oxygen atom or a sulfur atom.)

The further preferred examples of organic compound (B) relating to the present invention include compounds one of whose tautomeric structures is represented by the following general formula (2):

[Formula 16]

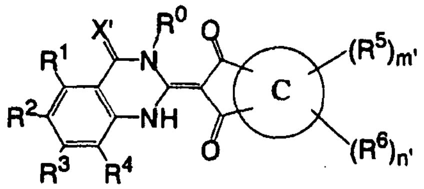 (2)

(wherein ring C represents a substituted or unsubstituted carbocyclic aromatic ring or a substituted or unsubstituted heterocyclic aromatic ring, X' represents a divalent substituent, each of $R^0$-$R^6$ represents independently a hydrogen

atom or a substituent; m' represents the number of $R^5$, n' represents the number of $R^6$; at least one group selected from $R^1$-$R^4$ is a substituted or unsubstituted amino group; for a combination among $R^1$-$R^4$ and a combination of $R^5$ and $R^6$ each substituent within each combination may independently bond via a linkage group to form a ring structure together with the atom to which it bonds; and each of m' and n' represents 0 or an integer of 1 or more.)

In compounds represented by general formula (2), ring C represents a substituted or unsubstituted carbocyclic aromatic ring or a substituted or unsubstituted heterocyclic aromatic ring.

The specific examples of the substituted or unsubstituted carbocyclic aromatic ring or substituted or unsubstituted heterocyclic aromatic ring represented by ring C in general formula (2) include rings like the above-mentioned ring A and ring B.

In compounds represented by general formula (2), X' represents a divalent substituent. The examples of the divalent substituent represented by X' include groups like the above-mentioned divalent substituents represented by X.

In compounds represented by general formula (2), each of $R^0$-$R^6$ represents independently a hydrogen atom or a substituent, m' represents the number of $R^5$, and n' represents the number of $R^6$. Here, at least one group selected from $R^1$-$R^4$ is a substituted or unsubstituted amino group. For a combination among $R^1$-$R^4$ and a combination of $R^5$ and $R^6$, each substituent within each combination may independently bond via a linkage group to form a ring structure together with the atom to which it bonds.

The specific examples of the substituent represented by $R^0$-$R^6$ include substituents like the above-mentioned examples of $R^A$ and $R^B$. Here it is preferred that $R^2$ is a substituted or unsubstituted amino group. The specific examples of the substituted or unsubstituted amino group include the above-mentioned substituted or unsubstituted amino groups. Substituted or unsubstituted amino groups having 1 or 2 carbon atom(s) such as a dimethylamino group, a methylamino group and an ethylamino group are preferred. A dimethylamino group is particularly preferred.

When, for a combination among $R^1$-$R^4$ and a combination of $R^5$ and $R^6$ in general formula (2), each substituent within each combination independently bonds via a linkage group to form a ring structure together with the atom to which it bonds, the examples of the linkage group include groups like the above-mentioned linkage group L.

Among $R^5$, $R^6$, and the ring structure formed by bonding of $R^5$, $R^6$ and the atoms which have $R^5$ or $R^6$ as the substituent via a linkage group, at least one of them preferably contains a heterocyclic residue having at least one heteroatom. Here "having heteroatom(s)" means that, at one or more site in the middle of carbon atom-carbon atom and/or carbon atom-hydrogen atom, one or more of same or different heteroatom(s) exist.

The heteroatom referred here is an atom other than a carbon atom or a hydrogen atom. Preferably the heteroatoms include an oxygen atom, a sulfur atom and a nitrogen atom.

The preferred example of the heterocyclic residue having at least one heteroatom includes a heterocyclic residue having 1 to 4 heteroatoms. As more preferred example includes a heterocyclic residue having 1 to 4 heteroatoms selected from an oxygen atom, a sulfur atom and a nitrogen atom.

The specific examples of the heterocyclic residue having one heteroatom include substituted or unsubstituted oxygen-containing heterocyclic residues whose heterocycle is a furan ring, a dihydrofuran ring, a tetrahydrofuran ring, a pyran ring, a dihydropyran ring, a tetrahydropyran ring or the like;
substituted or unsubstituted nitrogen-containing heterocyclic residues whose heterocycle is a pyrrole ring, a pyrroline ring, a pyrrolidine ring, a pyridine ring, a piperidine ring or the like; and
substituted or unsubstituted sulfur-containing heterocyclic residues whose heterocycle is a thiophene ring, a dihydrothiophene ring, a tetrahydrothiophene ring, a 1,1-dioxotetrahydrothiophene ring or the like.

The specific examples of the heterocyclic residue having two heteroatoms include substituted or unsubstituted oxygen-containing heterocyclic residues whose heterocycle is a 1,2-dioxolane ring, a 1,3-dioxolane ring, a 1,2-dioxane ring, a 1,3-dioxane ring, a 1,4-dioxane ring, a dioxene ring or the like;
substituted or unsubstituted nitrogen-containing heterocyclic residues whose heterocycle is an oxazolidine ring, an oxazole ring, a pyrazole ring, a pyrazoline ring, a pyrazolidine ring, an imidazole ring, a morpholine ring, a pyridazine ring, a pyrimidine ring, a pyrazine ring, a piperazine ring or the like; and
substituted or unsubstituted sulfur-containing heterocyclic residues whose heterocycle is a thiazole ring, a thiazolidine ring, a thiazoline ring or the like.

The specific examples of the heterocyclic residue having three heteroatoms include substituted or unsubstituted oxygen-containing heterocyclic residues whose heterocycle is a trioxane ring or the like;
substituted or unsubstituted nitrogen-containing heterocyclic residues whose heterocycle is a 1,3,5-triazine ring, a 1,2,4-triazine ring, a 1,2,3-triazine ring, a 1,3,5-hexahydrotriazine ring, a 1,2,4-hexahydrotriazine ring, a 1,2,3-hexahydrotriazine ring, an oxadiazole ring or the like; and
substituted or unsubstituted sulfur-containing heterocyclic residues whose heterocycle is a trithiane ring, a thiadiazole ring or the like.

The specific examples of the heterocyclic residue having four heteroatoms include substituted or unsubstituted nitrogen-containing heterocyclic residues whose heterocycle is a tetrazole ring, a tetrazine ring or the like.

In the compound represented by general formula (2), m' represents the number of $R^5$. The examples of the

number of $R^5$ represented by m', which is 0 or an integer of 1 or more include usually 0 to 4, preferably 0 to 2, and more preferably 1.

In the compound represented by general formula (2), n' represents the number of $R^6$. The number of $R^6$ represented by n', which is 0 or an integer of 1 or more, is usually 0 to 4, preferably 0 to 2, and more preferably 1.

The specific examples of tautomeric structures of the compound represented by general formula (2) include structures like those represented by general formulae (101) to (110), and one cam also use any mixture of these.

The further preferred examples of organic compound (B) relating to the present invention include compounds one of whose tautomeric structures is represented by the following general formula (3):

[Formula 17]

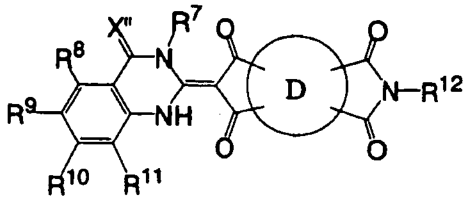

(3)

(wherein ring D represents a substituted or unsubstituted carbocyclic aromatic ring or a substituted or unsubstituted heterocyclic aromatic ring, X" represents a divalent substituent, each of $R^7$-$R^{12}$ represents independently a hydrogen atom or a substituent, at least one group selected from $R^8$-$R^{11}$ is a substituted or unsubstituted amino group, and each of $R^8$-$R^{11}$ may independently bond via a linkage group to form a ring structure together with the carbon atom to which it bonds.)

In the compound represented by general formula (3), ring D represents a substituted or unsubstituted carbocyclic aromatic ring or a substituted or unsubstituted heterocyclic aromatic ring.

The specific examples of the substituted or unsubstituted carbocyclic aromatic ring or the substituted or unsubstituted heterocyclic aromatic ring represented by ring D of general formula (3) include rings like the above-mentioned ring A and ring B.

In the compound represented by general formula (3), X" represents a divalent substituent. The specific examples of the divalent substituent represented by X" include groups like the above-mentioned divalent substituents represented by X.

In the compound represented by general formula (3), each of $R^7$-$R^{12}$ represents independently a hydrogen atom or a substituent, at least one group selected from $R^8$-$R^{11}$ is a substituted or unsubstituted amino group, and each of $R^8$-$R^{11}$ may independently bond via a linkage group to form a ring structure together with the carbon atom to which it bonds. The specific examples of the substituent represented by $R^7$-$R^{12}$ include substituents like the above-mentioned examples of $R^A$ and $R^B$. Here, $R^8$-$R^{11}$, further $R^9$ is preferably a substituted or unsubstituted amino group. The specific examples of the substituted or unsubstituted amino group include the above-mentioned substituted or unsubstituted amino groups. Substituted or unsubstituted amino groups having 1 or 2 carbon atom (s) such as a dimethylamino group, a methylamino group and an ethylamino group are preferred. A dimethylamino group is particularly preferred.

The atoms constituting each substituent represented by $R^7$-$R^{12}$ are preferably selected from a carbon atom, a hydrogen atom, a nitrogen atom, a sulfur atom and an oxygen atom.

When individual substituents within a combination among $R^8$-$R^{11}$ in general formula (3) independently bond via a linkage group to form a ring structure together with the carbon atom to which it bonds, the examples of the linkage group include groups like the above-mentioned linkage group L.

More preferably $R^{12}$ in general formula (3) is a substituent having a heterocyclic residue containing at least one heteroatom. The examples of the substituent include substituents substituted with the above-mentioned heterocyclic residue. Preferably it is a substituted alkoxy group or a substituted amino group substituted with a substituted or unsubstituted heterocyclic residue whose heterocycle is a piperazine ring, a morpholine ring, a dioxane ring, a pyrrolidine ring or the like. The specific examples include an alkoxy group to which a heterocyclic residue bonds such as a 4-methylpiperazinomethoxy group, a 4-methylpiperazinoethoxy group, a 4-methylpiperazinopropoxy group, a 4-methylpiperazinobutoxy group, a 4-ethylpiperazinomethoxy group, a 4-ethylpiperazinoethoxy group, a 4-ethylpiperazinopropoxy group, a 4-ethylpiperazinobutoxy group, a 4-propylpiperazinomethoxy group, a 4-propylpiperazinoethoxy group, a 4-propylpiperazinopropoxy group, a 4-propylpiperazinobutoxy group, a 4-butylpiperazinomethoxy group, a 4-butylpiperazinoethoxy group, a 4-butylpiperazinopropoxy group, a 4-butylpiperazinobutoxy group, a morpholinomethoxy group, a morpholinoethoxy group, a morpholinopropoxy group, a morpholinobutoxy group, a 1,3-dioxan-2-ylmethoxy group, a

1,3-dioxan-2-ylethoxy group, a 1,3-dioxan-2-ypropoxy group, a 1,3-dioxan-2-ylbutoxy group, a 2-oxopyrrolidinomethoxy group, a 2-oxopyrrolidinoethoxy group, a 2-oxopyrrolidinopropoxy group, a 2-oxopyrrolidinobutoxy group and the like; a disubstituted amino group to which heterocyclic residues bond such as a bis(4-methylpiperazinomethyl)amino group, a bis(4-methylpiperazinoethyl)amino group, a bis(4-methylpiperazinopropyl)amino group, a bis(4-methylpiperazinobutyl) amino group, a bis(4-ethylpiperazinomethyl)amino group, a bis(4-ethylpiperazinoethyl)amino group, a bis(4-ethylpiperazinopropyl)amino group, a bis(4-ethylpiperazinobutyl)amino group, a bis(4-propylpiperazinomethyl)amino group, a bis (4-propylpiperazinoethyl)amino group, a bis(4-propylpiperazinopropyl)amino group, a bis(4-propylpiperazinobutyl)amino group, a bis(4-butylpiperazinomethyl)amino group, a bis(4-butylpiperazinoethyl)amino group, a bis(4-butylpiperazinopropyl)amino group, a bis(4-butylpiperazinobutyl)amino group, a bis(4-morpholinomethyl)amino group, a bis(4-morpholinoethyl)amino group, a bis(4-morpholinopropyl)amino group, a bis(4-morpholinobutyl)amino group, a bis(1,3-dioxan-2-ylmethyl)amino group, a bis(1,3-dioxan-2-ylethyl)amino group, a bis(1,3-dioxan-2-ylpropyl)amino group, a bis(1,3-dioxan-2-ylbutyl)amino group, a bis(2-oxopyrrolidinomethyl)amino group, a bis(2-oxopyrrolidinoethyl)amino group, a bis(2-oxopyrrolidinopropyl)amino group, a bis(2-oxopyrrolidinobutyl)amino group and the like;
a monosubstituted amino group to which a heterocyclic residue bonds such as a 4-methylpiperazinomethylamino group, a 4-methylpiperazinoethylamino group, a 4-methylpiperazinopropylamino group, a 4-methylpiperazinobutylamino group, a 4-ethylpiperazinomethylamino group, a 4-ethylpiperazinoethylamino group, a 4-ethylpiperazinopropylamino group, a 4-ethylpiperazinobutylamino group, a 4-propylpiperazinomethylamino group, a 4-propylpiperazinoethylamino group, a 4-propylpiperazinopropylamino group, a 4-propylpiperazinobutylamino group, a 4-butylpiperazinomethylamino group, a 4-butylpiperazinoethylamino group, a 4-butylpiperazinopropylamino group, a 4-butylpiperazinobutylamino group, a 4-morpholinomethylamino group, a 4-morpholinoethylamino group, a 4-morpholinopropylamino group, a 4-morpholinobutylamino group, a 1,3-dioxan-2-ylmethylamino group, a 1,3-dioxan-2-ylethylamino group, a 1,3-dioxan-2-ylpropylamino group, a 1,3-dioxan-2-ylbutylamino group, a 2-oxopyrrolidinomethylamino group, a 2-oxopyrrolidinoethylamino group, a 2-oxopyrrolidinopropylamino group, a 2-oxopyrrolidinobutylamino group and the like; and the like.

The specific examples of the tautomeric form of general formula (3) include structures like those represented by general formulae (101) to (110), and one can also use any mixture of these.

The further preferred examples of organic compound (B) relating to the present invention include compounds represented by the following general formula (4):

[Formula 18]

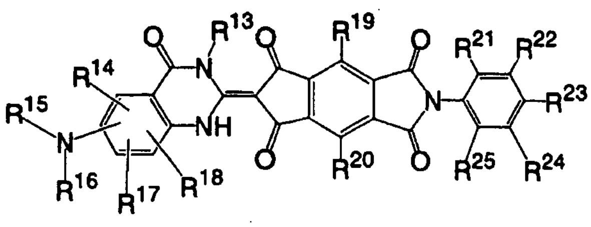

(4)

(wherein each of $R^{13}$-$R^{25}$ represents independently a hydrogen atom or a substituent, and for a combination among $R^{14}$-$R^{18}$ and a combination among $R^{21}$-$R^{25}$, each substituent within each combination may independently bond via a linkage group to form a ring structure together with the carbon and/or nitrogen atom to which it bonds.)

In the compound represented by general formula (4), each of $R^{13}$-$R^{25}$ represents independently a hydrogen atom or a substituent, and for a combination among $R^{14}$-$R^{18}$ and a combination among $R^{21}$-$R^{25}$, each substituent within each combination may independently bond via a linkage group to form a ring structure together with the carbon and/or nitrogen atom to which it bonds. The specific examples of the substituent represented by $R^{13}$-$R^{25}$ include substituents like the above-mentioned examples of $R^A$ and $R^B$. Preferably, at least one of $R^{21}$-$R^{25}$ is a substituent having a heterocyclic residue containing at least one heteroatom. More preferably, the specific examples of the substituent represented by $R^{13}$-$R^{25}$ include a substituted alkoxy group having a heterocyclic residue containing at least one heteroatom. The specific examples include groups like the above-mentioned substituents having a heterocyclic residue containing at least one heteroatom represented by $R^{12}$ in general formula (3).

Further, the atoms constituting each substituent represented by $R^{13}$-$R^{25}$ are preferably selected from a carbon atom, a hydrogen atom, a nitrogen atom, a sulfur atom, and an oxygen atom.

When, for a combination among $R^{14}$-$R^{18}$ and a combination among $R^{21}$-$R^{25}$ in general formula (4), each

substituent within each combination independently bonds via a linkage group to form a ring structure together with the carbon and/or nitrogen atom to which it bonds, the examples of the linkage group include groups like the above-mentioned linkage group L.

The bonding position of the substituted amino group bonding with $R^{15}$ and $R^{16}$ in the compound of general formula (4) is preferably the 6-position in the quinazoline ring. The specific examples of the substituted or unsubstituted amino group include the above-mentioned substituted or unsubstituted amino groups. Substituted or unsubstituted amino groups having 1 or 2 carbon atom(s) such as a dimethylamino group, a methylamino group and an ethylamino group are preferred. A dimethylamino group is particularly preferred.

The specific examples of the tautomeric structures of general formula (4) include structures like those represented by general formulae (101) to (110), and one can also use any mixture of these.

The further preferred examples of organic compound (B) relating to the present invention include compounds represented by the following general formula (5):

[Formula 19]

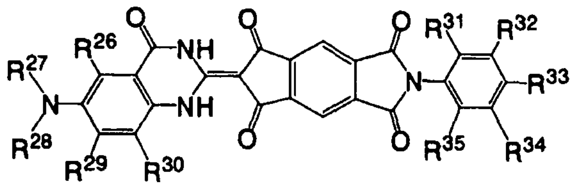

(5)

(wherein each of $R^{26}$-$R^{35}$ represents independently a hydrogen atom or a substituent; and for a combination among $R^{26}$-$R^{30}$ and a combination among $R^{31}$-$R^{35}$, each substituent within each combination may independently bond via a linkage group to form a ring structure together with the carbon and/or nitrogen atom to which it bonds.)

In the compound represented by general formula (5), each of $R^{26}$-$R^{35}$ represents independently a hydrogen atom or a substituent, and for a combination among $R^{26}$-$R^{30}$ and a combination among $R^{31}$-$R^{35}$, each substituent within each combination may independently bond via a linkage group to form a ring structure together with the carbon and/or nitrogen atom to which it bonds. The specific examples of the substituent represented by $R^{26}$-$R^{35}$ include substituents like the above-mentioned examples of $R^A$ and $R^B$. Here, $R^{31}$-$R^{35}$ is preferably a substituent having a heterocyclic residue containing at least one heteroatom. More preferably, it is a substituted alkoxy group having a heterocyclic residue containing at least one heteroatom. The specific examples include groups like the above-mentioned substituted alkoxy group having a heterocyclic residue containing at least one heteroatom represented by $R^{12}$ in general formula (3). The especially preferred examples include alkoxy groups to which a heterocyclic residue bonds such as a 2-oxopyrrolidinomethoxy group, a 2-oxopyrrolidinoethoxy group, a 2-oxopyrrolidinopropoxy group, 2-oxopyrrolidinobutoxy group and the like.

Further, the atoms constituting each substituent represented by $R^{26}$-$R^{35}$ are preferably selected from a carbon atom, a hydrogen atom, a nitrogen atom, a sulfur atom, and an oxygen atom.

When, for a combination among $R^{26}$-$R^{30}$ and a combination among $R^{31}$-$R^{35}$ in general formula (4), each substituent within each combination independently bonds via a linkage group to form a ring structure together with the carbon and/or nitrogen atom to which it bonds, the examples of the linkage group include groups like the above-mentioned linkage group L.

The specific examples of the substituted or unsubstituted amino group include the above-mentioned substituted or unsubstituted amino groups. Substituted or unsubstituted amino groups having 1 or 2 carbon atom(s) such as a dimethylamino group, a methylamino group and an ethylamino group are preferred. A dimethylamino group is particularly preferred.

The specific examples of tautomeric structures of general formula (5) include structures like those represented by general formulae (101) to (110), and one can also use any mixture of these.

As organic compound (B) contained in recording layer (A) relating to the present invention, the above-mentioned compounds of general formulae (0) to (5) can be preferably used. More preferably, it is a compound containing a substituent having a heterocyclic residue containing at least one heteroatom, and further preferably it is a compound containing a substituted alkoxy group having a heterocyclic residue containing at least one heteroatom. The specific examples include substituted alkoxy groups like the above-mentioned substituted alkoxy groups represented by $R^{12}$ in formula (3).

Further, a compound wherein an amino groups substituted with 1 or 2 methyl group(s) bonds to a quinazoline ring at its 6-position is particularly desirable. Substituted or unsubstituted amino groups having 1 or 2 carbon atom(s) such as a dimethylamino group, a methylamino group and an ethylamino group are preferred, and a dimethylamino group is particularly preferred.

In the present invention, a quinazolin-4-one compound having a disubstituted amino group at any one of positions 5-8 in the quinazoline -4- ring represented by general formula (6) is a novel compound suitable as an organic compound for the recording layer in the recording medium of the present invention:

[Formula 20]

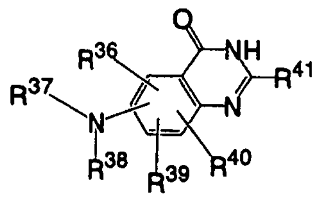

(6)

(wherein each of $R^{36}$-$R^{41}$ represents independently a hydrogen atom or a substituent.)

In the compound represented by general formula (6), each of $R^{36}$-$R^{41}$ represents independently a hydrogen atom or a substituent, and for combinations among $R^{36}$-$R^{40}$ each substituent within each combination may independently bond via a linkage group to form a ring structure together with the carbon and/or nitrogen atom to which it bonds. The specific examples of the substituent represented by $R^{36}$-$R^{41}$ include substituents like the above-mentioned examples of $R^A$ and $R^B$.

When each substituent within each of combinations among $R^{36}$-$R^{40}$ in general formula (6) independently bonds via a linkage group to form a ring structure together with the carbon and/or nitrogen atom to which it bonds, the examples of the linkage group include groups like the above-mentioned linkage group L.

The specific examples of organic compound (B) used for the optical recording medium of the present invention include the following compounds, although the present invention is not limited thereto.

[Table 1]

**Examples of compounds**

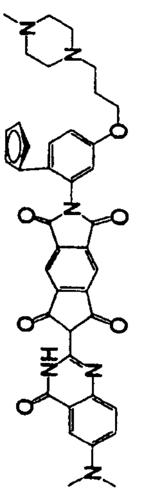

A-1

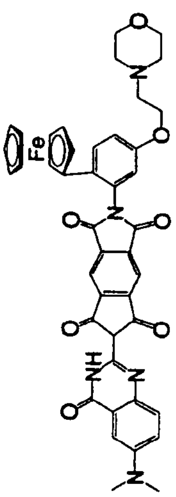

A-2

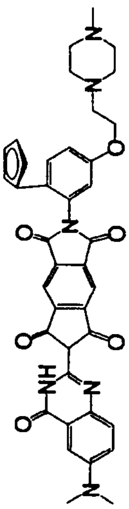

A-3

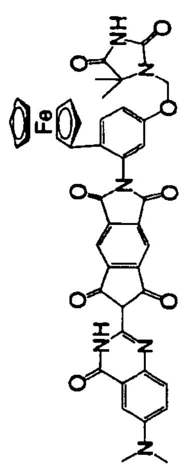

A-4

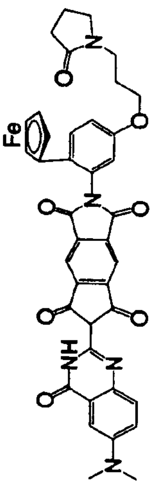

A-5

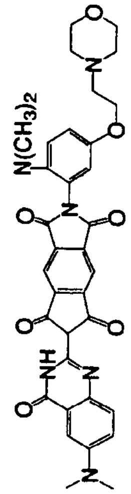

A-6

The compound represented by general formula (0), which is preferable as organic compound (B) relating to the present invention, can be produced, for example, by the following method. Namely, for example, by reaction of a compound represented by the following general formula (10) and a compound represented by the following general formula (11) in the presence or absence of a solvent, with heating if necessary, the compound represented by general formula (0) can be produced. A catalyst may be used in the reaction if necessary.

[Formula 21]

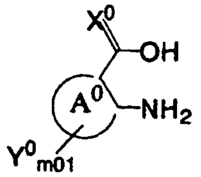 (1 0) 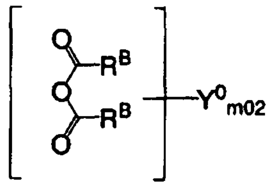 (1 1) 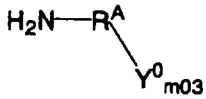 (1 2)

[wherein ring $A^0$, $R^A$, $R^B$, $X^0$ and $Y^0$ mean the same as ring $A^0$, $R^A$, $R^B$, $X^0$ and $Y^0$ in general formula (0), each of m01, m02 and m03 represents independently 0 or an integer of 1 or more, and these integers satisfy the relation with integer m0 in general formula (0), m01 + m2 + m03 = m0.]

The solvent used in reaction includes hydrocarbon solvents such as pentane, hexane, heptane, octane and the like, sulfur-containing solvents such as sulforane and the like, amide-type solvents such as N-methyl-2-pyrrolidinone, 1,3-dimethylimidazolidin-2-one and the like, halogenated aromatic solvents such as 1-chloronaphthalene, 1, 2-dichlorobenzene and the like, aromatic nitro compounds such as nitrobenzene and the like, organic acid solvents such as acetic acid, propionic acid, butyric acid and the like, and ethereal solvents such as tetrahydrofuran, dioxane, dibutyl ether and the like. The catalysts include amines such as isoquinoline, 1,8-diazabicyclo[5.4.0]-7-undecene, 1,5-diazabicyclo[4.3.0]-5-nonene and the like, organic acids such as acetic acid, propionic acid, butyric acid and the like, and metal salts such as palladium acetate, palladium chloride, palladium oxide and the like.

Further, the reaction temperature is not particularly limited as long as the reaction proceeds. It is preferably 30°C or higher, more preferably 50°C or higher, and further preferably 100°C or higher.

As another production method of the compound represented by general formula (0), for example, the compound represented by general formula (0) can be produced by reaction of a compound represented by the following general formula (13) and a compound represented by the following general formula (14) in the presence or absence of a solvent in the presence or absence of a base, with heating if necessary. A catalyst may be used in the reaction if necessary.

[Formula 22]

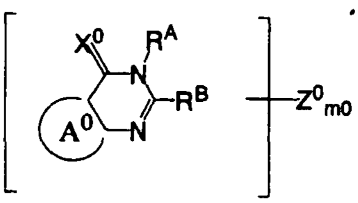 (1 3) $H-Y^0_{m0}$ (1 4)

wherein ring $A^0$, $R^A$, $R^B$, $X^0$, $Y^0$ and $m^0$ mean the same as ring $A^0$, $R^A$, $R^B$, $X^0$, $Y^0$ and $m^0$ in general formula (0) and $Z^0$ represents a leaving group.

The solvents and catalysts used in the reaction include those like the above-mentioned solvents and catalysts. The heating temperature is the above-mentioned heating temperatures.

The bases used in the reaction include metal alcoholate compounds such as sodium methoxide, sodium ethoxide, sodium propoxide, sodium butoxide, potassium methoxide, potassium ethoxide, potassium propoxide, potassium butoxide and the like.

The leaving group represented by $Z^0$ in general formula (13) is not particularly limited. It can be arbitrarily selected from groups which can be eliminated on amination of the compound represented by general formula (13) with the amine compound represented by general formula (14). The specific examples include halogen atoms such as a chlorine atom, a bromine atom and an iodine atom and substituted sulfonyloxy groups such as an arylsolfonyloxy group, an alkylsulfonyloxy group and the like.

The compound represented by general formula (1), which is preferable as organic compound (B) relating to the present invention, can be produced, for example, by the following method. Namely, for example, the compound represented by general formula (1) can be produced by reaction of a compound represented by the following general formula (15) and a compound represented by the following general formula (16) in the presence or absence of a solvent

with heating. A catalyst may be used in the reaction if necessary.

[Formula 23]

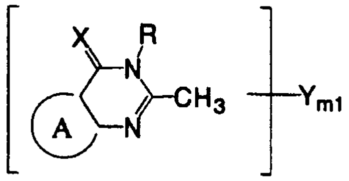 (15) 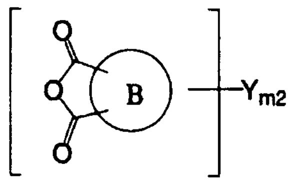 (16)

[wherein ring A, ring B, X and R mean the same as ring A, ring B, X and R in general formula (1), each of m1 and m2 represents independently 0 or an integer of 1 or more, and these integers satisfy the relation with integer m in general formula (1), m1 + m2 = m0.]

A solvent and a catalyst used in the reaction includes those like the above-mentioned solvents and catalysts. The heating temperature is the above-mentioned heating temperatures.

Further, the compound represented by general formula (2) can be produced, for example, from a compound represented by the following general formula (17) and a compound represented by the following general formula (18) by the above-mentioned reaction in the same way.

[Formula 24]

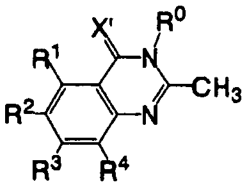 (17) 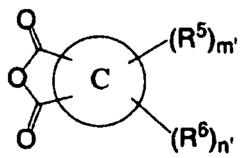 (18)

[wherein ring C, X', $R^0$-$R^6$, m' and n' mean the same as ring C, X', $R^0$-$R^6$, m' and n' in general formula (2).]

Further, the compound represented by general formula (3) can be produced, for example, by a method wherein the above-mentioned reaction of a compound represented by the following general formula (19) and a compound represented by the following general formula (20) is carried out in the same way to obtain the following general formula (21), which is subjected to successive reaction with an amine compound of general formula (22) in the presence or absence of a solvent with heating. A catalyst may be used in the reaction if necessary.

[Formula 25]

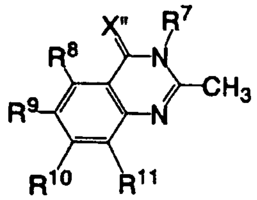 (19)

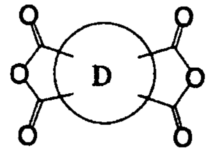 (20)

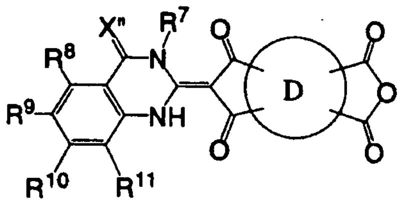 (21)

$H_2N—R^{12}$ (22)

[wherein ring D, X" and $R^7$-$R^{12}$ mean the same as ring D, X" and $R^7$-$R^{12}$ in general formula (3).]

Further, the compound represented by general formula (4) can be produced, for example, from a compound represented by the following general formula (23) and a compound represented by the following general formula (24) by the above-mentioned reaction in the same way to obtain the following general formula (25), which is subjected to successive reaction with an amine compound of general formula (26).

[Formula 26]

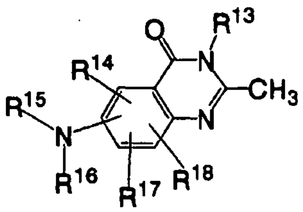 (23)

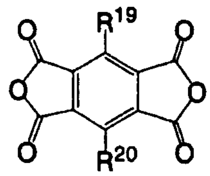 (24)

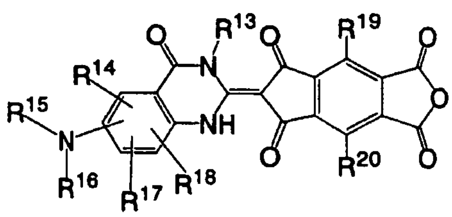 (25)

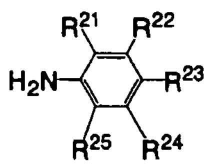 (26)

[wherein $R^{13}$-$R^{25}$ mean the same as $R^{13}$-$R^{25}$ in general formula (4).]

Further, the compound represented by general formula (5) can be produced, for example, from a compound represented by the following general formula (27) and a compound represented by the following general formula (28) by the above-mentioned reaction in the same way to obtain the following general formula (29), which is subjected to successive reaction with an amine compound represented by general formula (30).

[Formula 27]

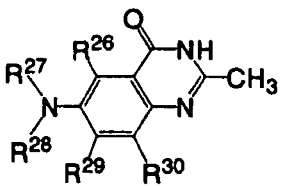 (27)

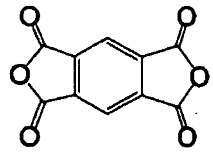 (28)

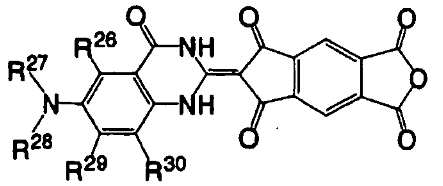 (29)

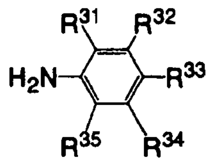 (30)

[wherein $R^{26}$-$R^{35}$ mean the same as $R^{26}$-$R^{35}$ in general formula (4).]

A compound represented by following general formula (27) can be produced according to the method for producing the above-mentioned compound represented by general formula (0). Alternatively, it can be produced according to the following synthetic method as another synthetic method. Namely, the compound represented by general formula (6) can be produced by reaction of a compound represented by the following general formula (7) and a compound represented by the following general formula (8) and/or a compound represented by the following general formula (9) in the presence or absence of a solvent in the presence or absence of base, with heating if necessary.

[Formula 28]

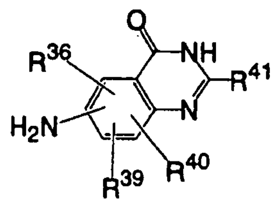 (7)

$R^{37}$- $Z^1$ (8)

$R^{38}$ - $Z^2$ (9)

[wherein $R^{36}$-$R^{41}$ represent the same groups as $R^{36}$-$R^{41}$ in general formula (6), and $Z^1$ and $Z^2$ represent a leaving group.]

The leaving group represented by $Z^1$ and $Z^2$ in general formula (8) and general formula (9) is not particularly limited. It can be arbitrarily selected from groups which can be eliminated on substitution reaction of the compound represented by general formula (7) with the compound represented by general formula (8) and/or general formula (9). The specific examples include halogen atoms such as a chlorine atom, a bromine atom and an iodine atom and substituted sulfonyloxy groups such as an arylsolfonyloxy group, an alkylsulfonyloxy group and the like.

The amount of the compound represented by general formula (8) and/or general formula (9) used in the reaction is preferably 1.0 to 10 mol, more preferably 2.0 to 6.0 mol for 1 mol of the compound represented by general formula (7).

The preferred examples of general formula (8) and general formula (9) include substituted or unsubstituted halogenated compounds. More specifically, the preferred example of general formula (8) and general formula (9) includes an aliphatic hydrocarbon compound substituted with halogen atom(s).

The reaction of the present invention is carried out in the presence or absence of a solvent. The solvent employed is not particularly limited unless it prohibits the reaction. For example, the solvents include alcoholic solvents

such as methanol, ethanol, isopropyl alcohol and the like, amide-type solvents such as N,N-dimethylformamide, N-methylpyrrolidone and the like, urea-type solvents such as 1,3-dimethyl-2-imidazolidinone and the like, sulfoxide-type solvents such as dimethylsulfoxide, sulfolane and the like, and aromatic solvents such as toluene, xylene and the like. Aprotic solvents such as amide-type solvents and sulfoxide-type solvents are preferred. More preferably N,N-dimethylformamide or dimethylsulfoxide is used. These solvents may be used singly or as a mixture of two or more.

The amount of the above-mentioned solvent used in the reaction is preferably 0 to 50 parts by weight more preferably 0 to 30 parts by weight for 1 part by weight of the compound of general formula (7), although it is necessary to adjust depending on homogeneity of the reaction solution and the stirring speed.

The basic compounds used in the reaction of the present invention are selected from various compounds including inorganic base reagents such as potassium carbonate, sodium hydride and the like, alkali metal alcoholates such as potassium t-butoxide and the like, and organic base reagents such as t-butyl lithium and the like. Preferably, the basic compounds include inorganic base reagents, more preferably potassium carbonate and sodium hydride.

The amount of the above-mentioned basic compound used is preferably 0.1 to 5.0 mol, more preferably 0.5 to 2.5 mol for 1 mol of the compound of general formula (7).

The reaction of the present invention is usually carried out by stirring under an air atmosphere or nitrogen atmosphere. The reaction temperature is, usually -20 to 100°C, preferably below 50°C so that formation of a byproduct can be suppressed. Here the byproduct is a compound wherein the substituent is bonded to the imino-NH at the 3-position in the quinazolin-4-one ring. The reaction temperature is more preferably 10 to 50°C, further preferably 10 to 40°C, and especially preferably 15 to 30°C. The reaction pressure is not particularly limited and atmospheric pressure is sufficient. The reaction time is preferably 15 min to 1 day and more preferably 30 min to 15 hr.

The preferred reaction examples of the present invention include a procedure wherein compounds of general formulae (7) to (9), a basic compound and a solvent are mixed and stirred. The order of mixing of the compounds of general formulae (7) to (9), the basic compound and the solvent is not particularly limited. They can be mixed in any order depending on necessity.

The final product, compound of general formula (6), can be isolated and purified after completion of the reaction by conventional methods such as concentration, distillation, recrystallization, column chromatography and the like. Alternatively, it can be obtained through operations such as pouring into a poor solvent, filtration, washing, drying and the like.

In preparation of an optical recording medium shown in Fig. 2 or Fig. 3, when recording layer (A) is formed on the substrate, a layer containing inorganic material or polymer may be formed on the substrate in order to improve solvent-resistance or reflectance of the substrate or recording sensitivity.

The film thickness of recording layer (A) is 10 nm to 1000 nm and preferably 20 nm to 300 nm. If the film thickness of recording layer (A) is less than 10 nm, in some cases recording cannot be carried out due to large thermal diffusion or the recorded signal is distorted and its amplitude becomes small. When the film thickness of recording layer (A) is more over 1000 nm, the reflectivity is reduced and the characteristics of playback signals may be deteriorated.

Next, a reflection layer of preferably 50 nm to 300 nm in thickness is formed on recording layer (A). In order to increase reflectance or adhesion, a reflection enhancing layer or an adhesion layer may be provided between recording layer (A) and the reflection layer. As materials for the reflection layer, one can use materials having sufficiently high reflectance at the wavelength of playback light, for example, metals of Au, Al, Ag, Cu, Ti, Cr, Ni, Pt, Ta and Pd singly or as alloy. Among them, Au, Ag and Al have high reflectance and are suitable materials for the reflection layer. When playback is carried out with blue laser, Al or Ag is suitable. Other than these, the following material may be included. For example, the materials for the reflection layer include metal or metalloid of Mg, Se, Hf, V, Nb, Ru, W, Mn, Re, Fe, Co, Rh, Ir, Zn, Cd, Ga, In, Si, Ge, Te, Pb, Po, Sn and Bi. Material containing Ag or Al as the major component is preferred because it can easily provide a reflection layer with high reflectance. One can also use, as a reflection layer, a multilayer film formed with non-metal material by stacking alternately low refractive index films and those having high refractive index films.

The methods to form the reflection layer include sputtering method, ion-plating method, chemical vapor deposition method, vacuum vapor deposition method and the like. On the substrate or under the reflection layer, an inorganic or organic intermediate layer or an adhesion layer known in the art may be formed to increase reflectance, to improve recording characteristics, to increase stability against playback light or to increase adhesion.

Further, material for the protection layer formed on the reflection layer is not particularly limited as long as it can protect the reflection layer from external force. The inorganic materials include $SiO_2$, $Si_3N_4$, $MgF_2$, AlN, $SnO_2$, $TiO_2$ and the like. The organic materials include thermoplastic resin, thermosetting resin, electron beam curing resin, ultraviolet curing resin and the like. Thermoplastic resin and thermosetting resin can be formed by procedures wherein they are dissolved in an appropriate solvent to prepare a coating liquid and then a layer to be protected is coated with the coating liquid and dried. Ultraviolet curingcuring resin can be formed by procedures wherein a layer to be protected is coated with resin itself or a coating liquid prepared by dissolving the resin in an appropriate solvent and the resin is hardened by irradiation with ultraviolet light. The ultraviolet curing resins include acrylate resins such as urethane acrylate, epoxy

acrylate, polyester acrylate and the like. These materials may be used singly or as a mixture. The protection layer can be formed either as a single layer film or as a multilayer film.

The methods of formation of the protection layer, like those of the recording layer include coating methods such as spin-coating method, casting method and the like, sputtering method, chemical vapor deposition method and the like. Among these, spin-coating method is preferred.

The film thickness of the protection layer is generally 0.1 μm to 100 μm, but it is 3 μm to 30 μm in the present invention, preferably 5 μm to 20 μm.

Label, barcode etc. can be printed on the protection layer.

Moreover, there may be used a method wherein a protection sheet or a substrate is adhered onto the reflection layer surface, a method wherein two sheets of the optical recording medium are adhered together with their reflection layer surfaces facing each other, or the like.

On the mirror plane side of the substrate, ultraviolet curing resin, an inorganic material-based thin film or the like may be formed for protecting the surface or preventing attachment of dust.

When an optical recording medium such as shown in Fig. 4 is prepared, a reflection layer of preferably 1 nm to 300 nm in thickness is formed on the substrate. In order to increase reflectance or to improve adhesion, a reflection enhancing layer or an adhesion layer may be formed between recording layer (A) and the reflection layer. As materials for the reflection layer, one can use materials having sufficiently high reflectance at the wavelength of playback light, for example, Al, Ag, Ni or Pt singly or as alloy. Among these, Ag and Al have high reflectance and are suitable materials for the reflection layer. Other than these, the following material may be included if necessary. For example, the materials for the reflection layer include metal or metalloid of Mg, Se, Hf, V, Nb, Ru, W, Mn, Re, Fe, Co, Rh, Ir, Zn, Cd, Ga, In, Si, Ge, Te, Pb, Po, Sn, Bi, Au, Cu, Ti, Cr, Pd and Ta. A material containing Ag or Al as the major component, with which a reflection layer having high reflectance can be easily obtained, is preferred. One can also use, as a reflection layer, a multilayer film formed with non-metal material by stackig alternately low refractive index films and high refractive index films.

The methods to form the reflection layer include sputtering method, ion-plating method, chemical vapor deposition method, vacuum vapor deposition method and the like. On the reflection layer, an inorganic or organic intermediate layer or an adhesion layer known in the art may be formed to increase reflectance, to improve recording characteristics, to increase stability against playback light or to increase adhesion.

Next, when a film of recording layer (A) is formed on the reflection layer, a layer comprising of inorganic material or polymer may be formed on the substrate in order to improve solvent-resistance or reflectance of the substrate or recording sensitivity.

The preparation methods of recording layer (A) include coating methods such as spin-coating method, spray method, casting method, slide method, curtain method, extrusion method, wire method, gravure method, spread method, roller-coat method, knife method, dipping method and the like. Spin-coating method is simple and preferred.

In coating method such as spin-coating method, one can use a coating liquid prepared by dissolving or dispersing compound (B) relating to the present invention in a solvent at a content of 1 to 40% by mass, preferably 3 to 30% by mass. Here, it is preferred to choose a solvent that causes no damage to the substrate.

The solvent used in coating method includes those like the above-mentioned solvents. These can be used singly or as a mixture of two or more. Solvents with boiling point of 150°C or lower under atmospheric pressure are preferred for production of optical recording medium of the present invention, because solvents are rapidly dried after coating.. Alcoholic solvents, especially fluorinated alcohols, with boiling point of 150°C or lower under atmospheric pressure are furthermore preferred. Specifically, the solvents used in coating method include tetrafluoropropanol and octafluoropentanol as preferred examples.

The film thickness of recording layer (A) is 1 nm to 1000 nm and preferably 5 nm to 300 nm. If the film thickness of recording layer (A) is less than 1 nm, in some cases recording cannot be carried out or the recorded signal is distorted and its amplitude becomes small. When the film thickness is more over 1000 nm, the reflectivity is reduced and the characteristics of playback signals may be deteriorated.

Further, material for the protection layer formed on recording layer (A) is not particularly limited as long as it can protect recording layer (A) from unwanted external effects such as external force and atmosphere. The inorganic materials include $SiO_2$, $Si_3N_4$, $MgF_2$, AlN, $SnO_2$, $TiO_2$ and the like. The organic materials include thermoplastic resin, thermosetting resin, electron beam curing resin, ultraviolet curingcuring resin and the like. Thermoplastic resin, thermosetting resin and the like can be formed by procedures wherein they are dissolved in an appropriate solvent to prepare a coating liquid and then a layer to be protected is coated with the coating liquid and dried. Ultraviolet curing resin can be formed by procedures wherein a layer to be protected is coated with resin itself or a coating liquid prepared by dissolving the resin in an appropriate solvent and the resin is hardened by irradiation with ultraviolet light. The ultraviolet curing resins include acrylate resins such as urethane acrylate, epoxy acrylate, polyester acrylate and the like. These materials may be used singly or as a mixture. The protection layer can be formed either as a single layer film or as a multilayer film.

The forming methods of the protection layer include coating methods such as spin-coating method, casting method and the like, sputtering method, chemical vapor deposition method and the like similar to the case of recording layer (A). Among these, spin-coating method is preferred. In addition, when any solvent which causes no damage to the recording layer cannot be selected, sputtering method, chemical vapor deposition method, vacuum vapor deposition method or the like may be used under conditions similar to those in the case of forming the recording layer. Furthermore, as described in JP H11-273147, the protection layer may be formed through an adhesion layer such as a pressure-sensitive adhesion sheet, a dry photopolymer sheet and the like, or the pressure-sensitive adhesion sheet or the dry photopolymer sheet itself can be used as the protection layer.

Further, when the protection layer is formed on recording layer (A), a layer containing inorganic material or polymer may be formed on recording layer (A) in order to improve solvent-resistance or reflectance of the recording layer or recording sensitivity.

The film thickness of protection layer is usually 0.01 μm to 1000 μm. In some cases it can be 0.1 μm to 100 μm or furthermore 1 μm to 20 μm.

Further, as alternative protection methods, a protection sheet or a reflection layer may be adhered onto the substrate surface, or two sheets of the optical recording medium may be adhered together with their substrate surfaces facing each other, or the like.

On the protection layer side, ultraviolet curing resin, an inorganic material-based thin film or the like may be formed for protecting the surface or preventing attachment of dust.

In the optical recording medium of the present invention, a chassis-type protection unit which protects the disc as used on flexible disc or magneto-optical disc may be provided to protect the whole medium. As material for the protection unit, plastics or metals such as aluminum and the like can be used.

As material for the substrate, any substance which is transparent at the recording wavelength and playback wavelength can be basically used. As material for the supporting substrate, considering also the case in which blue-violet laser is irradiated through substrate 11 as shown in Fig. 5, there can be used transparent material such as polymeric material such as acrylic resin, polyethylene resin, polycarbonate resin, polyolefin resin, epoxy resin and the like or inorganic material such as glass and the like. On the other hand, when laser is irradiated from the side of light transmission layer 15' opposite to substrate 11' as shown in the configuration of Fig. 6, material for the substrate can be selected from a wider variety of substance without limitation by such requirements in optical properties. From the viewpoint of required mechanical properties and productivity of the substrate, is preferred material which can be used for injection molding or cast molding such as acrylic resin, polycarbonate resin, polyolefin resin and the like. Such substrate material may be molded to a substrate in the disc-like form by injection molding method or the like.

Furthermore, a guide groove and/or pre-pit of submicron order may be formed spirally or in a concentric circle on a surface layer of the substrate, if necessary. The guide groove and pre-pit are preferably provided at formation of the substrate. They can be provided by molding with a stamper or by thermal transfer method using a photopolymer. The guide groove and/or pre-pit may be formed in light transmission layer 15' in Fig. 6, and these can be provided guide groove and pre-pit in the same way. The pitch and depth of the guide groove are preferably selected from a range of 0.25 to 0.80 μm for pitch and a range of 20 nm to 150 nm for depth in the case of HD-DVD-R to be recorded with the density higher than that of DVD.

When it is used as an optical disc, the optical recording medium of the present invention may usually be a discus shape of about 1.2 mm in thickness and 80 mm to 120 mm in diameter. There may be a hole of about 15 mm in diameter in the center.

On the optical recording medium of the present invention, recording and playback can be carried out at the recording laser wavelength and playback laser wavelength selected from 300 nm to 900 nm. Particularly, good C/N ratio, high stability against playback light and high quality signal characteristics are attained at the recording laser wavelength and playback laser wavelength selected from 390 nm to 430 nm, furthermore 400 nm to 410 nm.

Laser of 300 nm to 900 nm referred in the present invention is not particularly limited. The lasers of 300 nm to 900 nm include dye laser wherein the wavelength can be selected from a wide range in visible region, gas laser such as nitrogen laser (337 nm) and the like, ion-laser such as helium-cadmium laser (445 nm), argon ion laser (457 nm or 488 nm) and the like, GaN laser (400 nm to 410 nm), laser which generates the second harmonics (430 nm) of infrared laser based on Cr-doped $LiSnAlF_6$ (860 nm), He-Ne laser (633 nm), as well as semiconductor laser such as visible semiconductor laser of 415 nm, 425 nm, 602 nm, 612 nm, 635 nm, 647 nm, 650 nm, 660 nm, 670 nm, 680 nm, 780 nm or 830 nm, and the like. In the present invention, the above-mentioned laser can be appropriately selected depending on the wavelength at which the recording layer, on which recording and playback are carried out, is sensitive. It is preferred that high-density recording and playback each utilize one wavelength or a plurality of wavelengths selected from the above-mentioned laser, further preferably, laser having wavelength of 430 nm or less, in particular, laser having wavelength of 410 nm or less.

The recording laser power in the present invention is not particularly limited. The recording laser power is preferred a lower power. The preferred example of recording laser power for recording layer (A) relating to the present

invention includes usually 10 mW or less, preferably 8 mW or less and further preferably 6 mW or less. The playback laser power is not particularly limited. The playback laser power is preferred a lower power. The preferred example of playback laser power for recording layer (A) relating to the present invention is usually 2 mW or less, preferably 1 mW or less and further preferably 0.7 mW or less.

In the optical recording medium of the present invention, recording can be carried out with recording laser power of 5mW or less, the substrate is not deformed, and the quality is excellent both for recording and playback. Accordingly, recording and playback can be carried out with low recording laser power and it is excellent in multi-track recording characteristics. Furthermore, the degree of modulation is high and high-quality signal can be obtained.

Examples of the present invention are explained below, but the present invention is not limited to these examples. Further, melting points were determined on a melting point apparatus (BUCHI; B-540) with a temperature raising speed of 20°C/min, and exothermic peaks were measured on a differential scanning thermal analyzer (Shimadzu; DSC-50). Absorption spectra were recorded on a spectrophotometer (Shimadzu; UV-1600). Surfaces of recording layers were observed with a scanning electron microscope (SEM) (Hitachi Hi-Technologies; S-4500) or a scanning probe microscope (AFM) (Digital Instruments; NanoScope™ III). NMR spectra were recorded on an FT-NMR spectrometer (JEOL; GSX270), and mass spectra were obtained on a liquid chromatography-mass spectrometer (Shimadzu; LCMS-QP8000α) or an FD-MS mass spectrometer (JEOL; SX-102A).

[Production example 1]

Synthesis of compound A-1

A solution prepared by adding pyromellitic anhydride (16.9 parts) to tetrahydrothiophene-1,1-dioxide (170 parts) was heated to 200°C. Then a compound represented by the following formula (B-1) (15.7 parts) was added to the solution and the mixture was stirred at the same temperature for 10 hr. After completion of the reaction, the mixture was cooled below 40°C and poured into toluene (850 parts). The precipitate was washed with toluene and dried to obtain a compound represented by the following formula (a-1) (28.0 parts; yield 91%). Next, a solution prepared by mixing the compound represented by the following formula (a-1) (7.7 parts), 2-ferrocenyl-5-[3-(4-methylpiperazin-1-yl)propoxy]aniline (8.2 parts), 1-methyl-2-pyrrolidone (300 parts) and toluene (30 parts) was stirred at 150°C for 11 hr. After completion of the reaction, the mixture was cooled below 40°C and poured into methanol (2000 parts), and the precipitate was filtered. The precipitate collected was dissolved in chloroform and purified by column chromatography on silica-gel (eluting solvent: chloroform/methanol = 9/1). The resultant solid material was washed with methanol and dried to obtain a compound represented by the following formula (A-1) (5.7 parts; yield 37%).

[Formula 29]

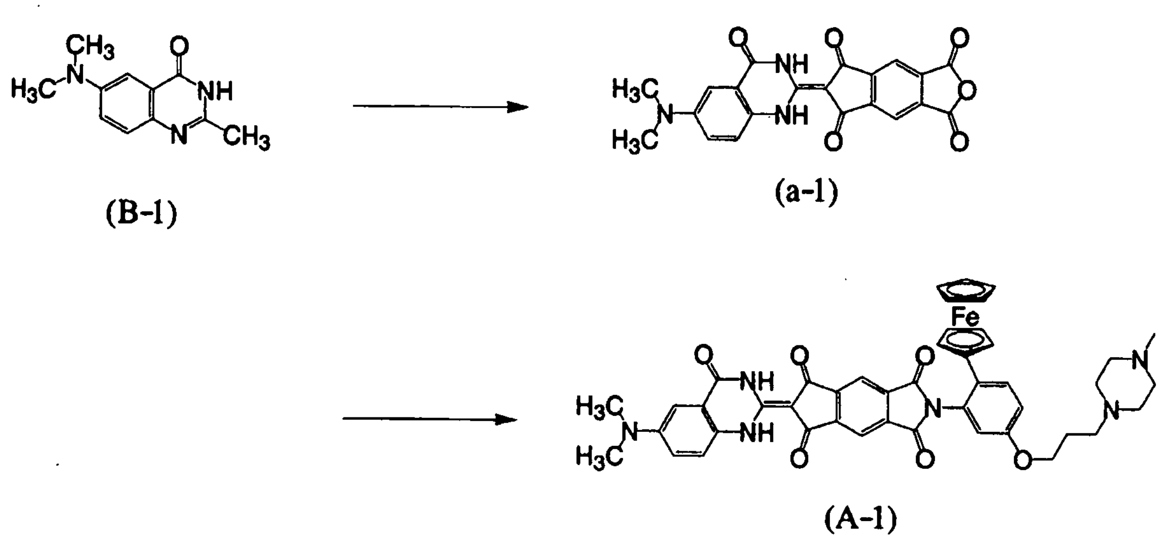

(B-1)

(a-1)

(A-1)

The physical properties of compound (A-1) are as follows:

- MS (m/z): 819

- Color-change temperature: < 390°C
- Melting point: > 400°C
- Exothermic peak temperature on exothermic transition from an amorphous state to a crystalline state: 233°C
- Maximum Absorption wavelength in a range of 300 nm to 900 nm in chloroform solution: 389.5 nm
- Refractive index at 405 nm in thin film, n1: 1.83
- Extinction coefficient at 405 nm in thin film, k1: 0.18

[Production example 2]

Synthesis of compound A-2

A solution prepared by mixing the compound represented by the above formula (a-1) (6.0 parts), 2-ferrocenyl-5-[2-(N-morpholyl)ethoxy]aniline (6.0 parts), 1-methyl-2-pyrrolidone (300 parts) and toluene (30 parts) was stirred at 150°C for 11 hr. After completion of the reaction, the mixture was cooled below 40°C and poured into methanol (1000 parts), and the precipitate was filtered. The precipitate collected was dissolved in chloroform and purified by column chromatography on silica-gel (eluting solvent: chloroform/methanol = 95/5). The resultant solid material was washed with methanol and dried to obtain a compound represented by formula (A-2) (2.6 parts; yield 22%).

The physical properties of compound (A-2) are as follows:

- MS (m/z): 792
- Color-change temperature: < 300°C
- Melting point: > 400°C
- Exothermic peak temperature on exothermic transition from an amorphous state to a crystalline state: 245°C
- Maximum absorption wavelength in a range of 300 nm to 900 nm in chloroform solution: 394.5 nm
- Refractive index at 405 nm in thin film, n1: 1.80
- Extinction coefficient at 405 nm in thin film, k1: 0.20

[Example 1-1]

Compound A-1 (0.2 g) was dissolved in 2,2,3,3-tetrafluoro-1-propanol (10 ml) to prepare a dye solution.

On a discus polycarbonate resin substrate of 120 mm in diameter and 0.6 mm in thickness having a continuous guide groove (track pitch; 0.6 μm, groove width; 0.30 μm, pitch ratio 50%, groove depth; 50 nm), this dye solution was spin-coated, and the coated substrate was dried at 70°C for 3 hr to form a recording layer of 60 nm in thickness on the groove in the substrate. On this recording layer, silver is sputtered with a sputtering apparatus (Balzars; CDI-900) to form a reflection layer of 120 nm in thickness. Argon was used as sputter gas. Sputtering was carried out under conditions including a sputtering power of 3.75 kW and a sputtering gas pressure of 1.06 Pa ($8.0 \times 10^{-3}$ Torr).

Further, after an ultraviolet curing resin SD-1700 (Dainippon Ink and Chemicals Incorporated) was spin-coated on the reflection layer, the reflection layer was irradiated with ultraviolet light to form a protection layer of 5 μm in thickness. Furthermore, after an ultraviolet curing resin DeSolite KZ-8681 (JSR) was spin-coated on the protection layer, a polycarbonate resin substrate (dummy substrate), which was the same as the above-mentioned substrate, was put on the protection layer, and these substrates were adhered together by irradiation with ultraviolet light to prepare an optical recording medium. Additional two sheets of the same optical recording medium were prepared by this method to obtain three sheets of optical recording medium of the present example. From one sheet of the prepared optical recording medium, which was unrecorded, the dummy substrate, adhesion layer, protection layer and silver reflection layer were peeled off to expose the dye layer. This sample was cut into a suitable piece and the absorbance was measured in a wavelength region of 300 nm to 500 nm on the spectrophotometer. The maximum absorption wavelength was 368 nm, which was shorter by 21.5 nm than that observed in chloroform solution. The absorbance at 405 nm was 0.18.

With one sheet of optical recording medium of the present example, evaluation test was carried out as follows.

On an optical tester (Pulstec Industrial Co. , Ltd. ; DDU-1000) equipped with a blue laser head having a wavelength of 405 nm and a numerical aperture of 0.65, recording was carried out on the groove at a recording laser power of 4.8 mW by using random signal pattern data with 1-7 modulation mode at a clock frequency of 33 MHz and a linear velocity of 3.8 m/s. A mark with large optical change and good shape was formed and high-density recording was attained. After recording, playback was carried out at a playback power of 0.4 mW on the same apparatus. The degree of signal modulation was high, the difference in jitter between recording/playback in single track and that in three contiguous tracks was less than 1%, and cross-write to adjacent tracks scarcely occurred. These results confirm very excellent multi-track characteristics. From the recorded optical recording medium, the dummy substrate, adhesion layer, protection layer and silver reflection layer were peeled off to expose the dye layer, and said recorded site was observed with the electron microscope. The image of electron microscopy (SEM) exhibited neither deformation of the substrate

nor pit formation in the dye film. Further, the amount of change in the depth direction of the recording layer was measured by using the AFM. The results indicated that the difference in film thickness between the recording layer at recorded site and the recording layer at unrecorded site was less than 15 nm and that the percentage change of the film thickness at recorded site compared to that at the unrecorded site was less than 25%. Next, the exposed dye layer was washed out with 2,2,3,3-tetrafluoro-1-propanol and the substrate was observed to confirm that there was no mechanical deformation of the substrate.

Using still another sheet of optical recording medium of the present example, recording was continuously carried out on both groove and land in a whole circle of said optical recording medium without applying on-off modulation at a constant laser power of 4.8 mW. The dummy substrate, adhesion layer, protection layer and silver reflection layer were peeled off therefrom to expose the dye layer. This sample was cut into a suitable piece, and the absorbance was measured on the spectrophotometer in a wavelength range of 300 nm to 500 nm. The maximum absorption wavelength was found to be 393 nm, which was shifted by 25 nm to the longer wavelength side, and the absorbance at 405 nm, at which recording/playback was carried out, was found to increase to 0.21. By dividing the absorbance by pre-determined dye film thickness d in groove, the absorption coefficient $\alpha$ after laser irradiation was determined, and k2 was determined using the relation between $\alpha$ and extinction coefficient k2 ($k2 = \lambda\alpha/4\pi$). The n2 value after laser irradiation was determined using Kramers-Kronig relation. At 405 nm n2 was 1.77 and k2 was 0.22. The amounts of change were $\Delta n(n2 - n1) = -0.06$ and $\Delta k(k2 - k1) = +0.04$.

[Example 1-2]

Except for using compound A-2 instead of compound A-1, three sheets of optical recording medium of the present example were prepared by the method described in Example 1-1, and each measurement was carried out in the same way as Example 1-1.

From one sheet of the prepared optical recording medium, which was unrecorded, the dummy substrate, adhesion layer, protection layer and silver reflection layer were peeled off to expose the dye layer. This sample was cut into a suitable piece and the absorbance was measured in a wavelength region of 300 nm to 500 nm on the spectrophotometer. The maximum absorption wavelength was found to be 368 nm, which was shorter by 26.5 nm than that observed in chloroform solution. The absorbance at 405 nm was found to be 0.20.

Further, using one sheet of optical recording medium of the present example, evaluation test was carried out in the same way as Example 1-1. A mark with large optical change and good shape was formed and high-density recording was attained. After recording, playback was carried out at a playback power of 0.4 mW on the same apparatus. The degree of signal modulation was high, the difference in jitter between recording/playback in single track and that in three contiguous tracks was less than 1%, and cross-write to adjacent tracks scarcely occurred. These results confirm very excellent multi-track characteristics. From one sheet of recorded optical recording medium, the dummy substrate, adhesion layer, protection layer and silver reflection layer were peeled off to expose the dye layer, and said recorded site was observed with the electron microscope. The image of electron microscopy (SEM) exhibited neither deformation of the substrate nor pit formation in the dye film. The amount of change in the depth direction of the recording layer was measured by using the AFM. The results indicated that the difference in film thickness between the recording layer at recorded site and the recording layer at unrecorded site was less than 15 nm and that the percentage change of the film thickness at recorded site compared to that at the unrecorded site was less than 25%. Next, the exposed dye layer was washed out with 2,2,3,3-tetrafluoro-1-propanol and the substrate was observed to confirm that there was no mechanical deformation of the substrate.

Using still another sheet of optical recording medium of the present example, recording was continuously carried out both on groove and land in a whole circle of said optical recording medium without applying on-off modulation at a constant laser power of 4.8 mW. The dummy substrate, adhesion layer, protection layer and silver reflection layer were peeled off therefrom to expose the dye layer. This sample was cut into a suitable piece, and the absorbance was measured on the spectrophotometer in a wavelength range of 300 nm to 500 nm. The maximum absorption wavelength was found to be 395 nm, which was shifted to the longer wavelength by 27 nm, and the absorbance at 405 nm, at which recording/playback was carried out, was found to increase to 0.22. By performing calculation in the same way as Example 1-1, it was found that n2 = 1.75 and k2 = 0.22 at 405 nm. The amounts of change were $\Delta n(n2 - n1) = -0.05$ and $\Delta k(k2 - k1) = +0.04$.

[Example 1-3]

The physical properties of compound A-3 are as follows: Color-change temperature < 300°C; Melting point > 400°C, Exothermic peak temperature on exothermic transition from an amorphous state to a crystalline state 241°C; Absorption maximum wavelength in a range of 300 nm to 900 nm in chloroform solution 397. 5 nm; Refractive index n1 = 1.80; and extinction coefficient k1 = 0.18 at 405 nm in thin film.

Except for using compound A-3 instead of compound A-1, three sheets of optical recording medium of the present example were prepared by the method described in Example 1-1, and each measurement was carried out in the same way as Example 1-1.

From one sheet of the prepared optical recording medium, which was unrecorded, the dummy substrate, adhesion layer, protection layer and silver reflection layer were peeled off to expose the dye layer. This sample was cut into a suitable piece and the absorbance was measured in a wavelength region of 300 nm to 500 nm on the spectrophotometer. The maximum absorption wavelength was found to be 372 nm, which was shorter by 25.5 nm than that observed in chloroform solution.

Further, using one sheet of optical recording medium of the present example, evaluation test was carried out in the same way as Example 1-1. A mark with large optical change and good shape was formed and high-density recording was attained. After recording, playback was carried out at a playback power of 0.4 mW on the same apparatus. The degree of signal modulation was high, the difference in jitter between recording/playback in single track and that in three contiguous tracks was less than 1%, and cross-write to adjacent tracks scarcely occurred. These results confirm very excellent multi-track characteristics. From the recorded optical recording medium, the dummy substrate, adhesion layer, protection layer and silver reflection layer were peeled off to expose the dye layer, and said recorded site was observed with the electron microscope. The image of electron microscopy (SEM) exhibited neither deformation of the substrate nor pit formation in the dye film. Measurement of the amount of change in the depth direction of the recording layer by using an AFM indicated that the difference in film thickness between the recording layer at recorded site and the recording layer at unrecorded site is less than 15 nm and that the percentage change of the film thickness at recorded site compared to that at the unrecorded site is less than 25%. Next, the exposed dye layer was washed out with 2,2,3,3-tetrafluoro-1-propanol and the substrate was observed to confirm that there was no mechanical deformation of the substrate.

Further, using still another sheet of optical recording medium of the present example, recording was continuously carried out both on groove and land in a whole circle of said optical recording medium without applying on-off modulation at a constant laser power of 4.8 mW. The dummy substrate, adhesion layer, protection layer and silver reflection layer were peeled off therefrom to expose the dye layer. This sample was cut into a suitable piece, and the absorbance was measured on the spectrophotometer in a wavelength range of 300 nm to 500 nm. The maximum absorption wavelength was found to be 391 nm, which was shifted to the longer wavelength by 19 nm, and the absorbance at 405 nm, at which recording/playback was carried out, was found to increase. By performing calculation in the same way as Example 1-1, it was found that $n2 = 1.76$ and $k2 = 0.22$ at 405 nm. The amounts of change were $\Delta n(n2 - n1) = -0.04$ and $\Delta k(k2 - k1) = +0.04$.

[Examples 1-4 to 1-6]

Except for using each of compounds A-4 to A-6 instead of compound A-1, three sheets of optical recording medium of each of the present examples were prepared by the method described in Example 1-1. For each of the present examples, from one sheet of optical recording medium prepared, which was unrecorded, the dummy substrate, adhesion layer, protection layer and silver reflection layer were peeled off to expose the dye layer. This sample was cut into a suitable piece and the absorbance was measured in a wavelength region of 300 nm to 500 nm on the spectrophotometer. The maximum absorption wavelength was found to be shorter by 10 nm or more than that observed in chloroform solution in each case.

When evaluation test was carried out in the same way as Example 1-1, a mark with large optical change and good shape was formed and high-density recording was attained. After recording, playback was carried out at a playback power of 0.4 mW on the same apparatus. The degree of signal modulation was high, the difference in jitter between recording/playback in single track and that in three contiguous tracks was less than 1%, and cross-write to adjacent tracks scarcely occurred. These results confirm very excellent multi-track characteristics.

For one sheet of recorded optical recording medium prepared in each example, said recorded site was observed with the electron microscope in the same way as Example 1-1. The image of electron microscopy (SEM) exhibited neither deformation of the substrate nor pit formation in the dye film. The amount of change in the depth direction of the recording layer was measured by using the AFNI. The results indicated that the difference in film thickness between the recording layer at recorded site and the recording layer at unrecorded site was less than 15 nm and that the percentage change of the film thickness at recorded site compared to that at the unrecorded site was less than 25%. Next, the exposed dye layer was washed out with 2,2,3,3-tetrafluoro-1-propanol and the substrate was observed to confirm that there was no mechanical deformation of the substrate.

Further, using still another sheet of optical recording medium prepared in each example, recording was continuously carried out on both groove and land in a whole circle of said optical recording medium without applying on-off modulation at a constant laser power of 4.8 mW. The dummy substrate, adhesion layer, protection layer and silver reflection layer were peeled off therefrom to expose the dye layer. This sample was cut into a suitable piece, and the absorbance was measured on the spectrophotometer in a wavelength range of 300 nm to 500 nm. The maximum

absorption wavelength was found to shift to the longer wavelength side by 10 nm or more in each case.

[Example 2-1]

Synthesis of compound B-1

A reaction solution prepared by adding the compound represented by the following formula (b-1) (10.0 parts), methyl iodide (48.6 parts) and potassium carbonate (19.7 parts) to N,N-dimethylformamide (260 parts) was stirred at room temperature for 2 hr. After completion of the reaction, the mixture was poured into water (1500 parts), and the precipitate was collected by filtration, washed with water and dried to obtain the compound represented by the following formula (B-1) (3.50 parts; yield 30%).

[Formula 30]

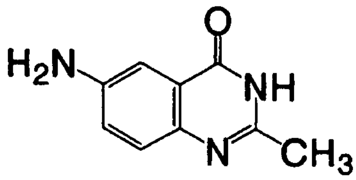

(b − 1)

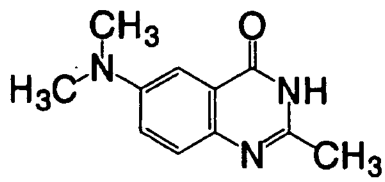

(B − 1)

- MS (m/z): 203
- $^1$H-NMR (DMSO-$d_6$, δ(ppm)) : 11.91 (1H, s, NH), 7.43 (1H, d, J=8.91, 7-H), 7.27 (1H, dd, J=2.97 , 8.91, 8-H), 7.15 (1H, d, J=2.97, 5-H), 2.97 (6H, s, $(CH_3)_2N$-), 2.27 (3H, s, -$CH_3$).

[Example 2-2]

Synthesis of compound B-2

A reaction solution prepared by adding the compound represented by formula (b-1), which was used in Example 2-1, (9.90 parts), ethyl iodide (52.8 parts) and potassium carbonate (19.5 parts) to N,N-dimethylformamide (250 parts) was stirred at room temperature for 12 hr. After completion of the reaction, the mixture was poured into water (1250 parts), and the precipitate was collected by filtration, washed with water and dried to obtain the compound represented by the following formula (B-2) (4.70 parts; yield 36%).

[Formula 31]

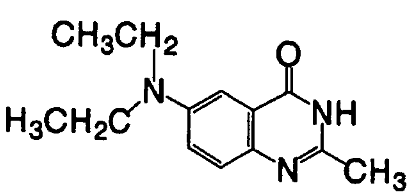

(B − 2)

- MS (m/z): 231
- $^1$H-NMR (DMSO-$d_6$, δ(ppm)): 11.88 (1H, s, NH), 7.40 (1H, d, J=8.91, 7-H), 7.20 (1H, dd, J=2.97, 9.04 , 8-H), 7.10 (1H, d, J=3.24, 5-H), 3.39 (4H, q, $CH_2$), 2.26 (3H, s, 2-$CH_3$), 1.11 (6H, t, $CH_3$).

As described in Examples 1-1 to 1-6, the optical recording medium of the present invention is capable of recording/playback at low recording laser power in the wavelength region of blue laser, and it is excellent in multi-track recording characteristics. Moreover, it provides high degree of modulation and high signal quality.

Based on these results, the recording layer containing a compound having the structure defined in the present invention is capable of signal recording with laser light having a wavelength selected from a range of 300 nm to 900 nm,

and the optical recording medium of the present invention can be used as an optical recording medium wherein recording/playback is carried out with laser light having a wavelength selected from a range of 300 nm to 900 nm.

[Industrial applicability]

According to the present invention, by using organic compound (B) relating to the present invention for recording layer (A), there is provided a truly practical optical recording medium with respect to the increase of information recording capacity, wherein high-quality recording can be carried out in a whole circle of the medium at low laser power with laser of 300 nm to 900 nm, which attracts much attention for high-density optical recording media, furthermore laser of 390 nm to 430 nm, particularly blue-violet laser of 400 nm to 410 nm.

## Claims

1. An optical recording medium containing at least one recording layer (A) capable of recording and playback with a laser light,
wherein said recording layer (A) contains at least one kind of organic compound,
wherein the percentage change ($|[a^2 - a^1] / a^1| \times 100$) of the recording layer thickness ($a^2$) at the recorded site of said recording layer (A) after recorded with a laser light compared with the recording layer thickness ($a^1$) at unrecorded site of said recording layer (A) is less than 25%, and the amount of change ($|a^2 - a^1|$) of the recording layer thickness ($a^2$) at recorded site of said recording layer (A) after recorded with laser light compared with the recording layer thickness ($a^1$) at unrecorded site of said recording layer (A) is less than 15 nm.

2. The optical recording medium according to claim 1, wherein said recording layer (A) is able to be formed by a coating method.

3. The optical recording medium according to claim 2, wherein the organic compound is organic compound (B) that comprises a six-membered ring structure composed of four carbon atoms and two nitrogen atoms and a substituted or unsubstituted amino group bonded.

4. The optical recording medium according to any of claims 1 to 3, wherein the recording laser power is 6 mW or lower.

5. The optical recording medium according to claim 3, wherein one of tautomeric structures of organic compound (B) is represented by general formula (0):

[Formula 1]

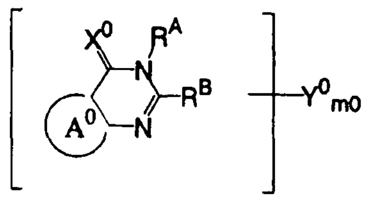

(0)

(wherein ring $A^0$ represents a substituted or unsubstituted carbocyclic aromatic ring or a substituted or unsubstituted heterocyclic aromatic ring; $R^A$ and $R^B$ represent a hydrogen atom or a substituent; $X^0$ represents a divalent substituent; $Y^0$ represents a substituted or unsubstituted amino group; and $m^0$ represents the number of $Y^0$).

6. The optical recording medium according to claim 5, wherein one of the tautomeric structures of organic compound (B) is represented by general formula (1):

[Formula 2]

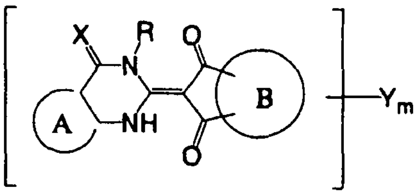

(1)

(wherein ring A and ring B represent a substituted or unsubstituted carbocyclic aromatic ring or a substituted or unsubstituted heterocyclic aromatic ring; R represents a hydrogen atom or a substituent; X represents a divalent substituent; Y represents a substituted or unsubstituted amino group; and m represents the number of Y).

**7.** The optical recording medium according to claim 6, wherein one of the tautomeric structures of organic compound (B) is represented by general formula (2):

[Formula 3]

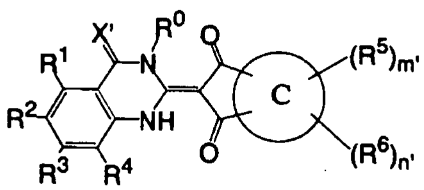

(2)

(wherein ring C represents a substituted or unsubstituted carbocyclic aromatic ring or a substituted or unsubstituted heterocyclic aromatic ring; X' represents a divalent substituent, each of $R^0$-$R^6$ represents independently a hydrogen atom or a substituent; m' represents the number of $R^5$; n' represents the number of $R^6$; at least one group selected from $R^1$-$R^4$ is a substituted or unsubstituted amino group; in a combination among $R^1$-$R^4$ and a combination of $R^5$ and $R^6$, each substituent within each combination may independently bond via a linkage group to form a ring structure together with the atom to which it bonds; and each of m' and n' represents 0 or an integer of 1 or more).

**8.** The optical recording medium according to claim 7, wherein one of the tautomeric structures of organic compound (B) is represented by general formula (3):

[Formula 4]

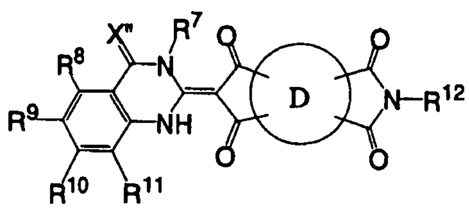

(3)

(wherein ring D represents a substituted or unsubstituted carbocyclic aromatic ring or a substituted or unsubstituted heterocyclic aromatic ring; X" represents a divalent substituent, each of $R^7$-$R^{12}$ represents independently a hydrogen atom or a substituent; at least one group selected from $R^8$-$R^{11}$ is a substituted or unsubstituted amino group; and each of $R^8$-$R^{11}$ may independently bond via a linkage group to form a ring structure together with the carbon atom to which it bonds).

9. The optical recording medium according to claim 8, wherein one of the tautomeric structures of organic compound (B) is represented by general formula (4):

[Formula 5]

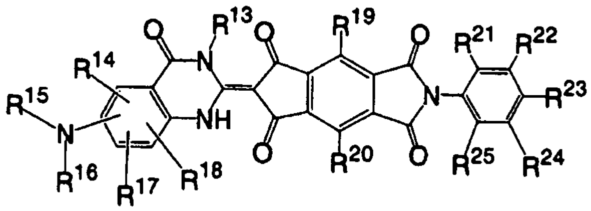

(4)

(wherein each of $R^{13}$-$R^{25}$ represents independently a hydrogen atom or a substituent; and in a combination among $R^{14}$-$R^{18}$ and a combination among $R^{21}$-$R^{25}$, each substituent within each combination may independently bond via a linkage group to form a ring structure together with the carbon atom and/or nitrogen atom to which it bonds).

10. The optical recording medium according to claim 9, wherein one of the tautomeric structures of organic compound (B) is represented by general formula (5):

[Formula 6]

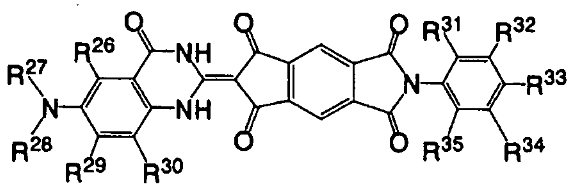

(5)

(wherein each of $R^{26}$-$R^{35}$ represents independently a hydrogen atom or a substituent; and in a combination among $R^{26}$-$R^{30}$ and a combination among $R^{31}$-$R^{35}$, each substituent within each combination may independently bond via a linkage group to form a ring structure together with the carbon atom and/or nitrogen atom to which it bonds).

11. The optical recording medium according to claim 10, wherein at least one group among $R^{31}$-$R^{35}$ is a substituted alkoxy group having a heterocyclic residue containing at least one heteroatom.

12. The optical recording medium according to claim 10, wherein the atoms constituting each substituent represented by $R^{26}$-$R^{35}$ are selected from a carbon atom, a hydrogen atom, a nitrogen atom, a sulfur atom, and an oxygen atom.

13. The optical recording medium according to claim 1, wherein the recording layer (A) contains at least one kind of organic compound that absorbs said laser light and has a temperature at which the color changes by heat.

14. The optical recording medium according to claim 1, wherein the recording layer (A) contains at least one kind of organic compound that absorbs said laser light and forms a crystalline state exothermically when an amorphous state is heated.

15. The optical recording medium according to claim 1, wherein said recording layer (A) contains at least one kind of organic compound for exhibits maximum absorption wavelength ($\lambda max^2$) in said recording layer (A) after recording that is different from that ($\lambda max^1$) before recording in said recording layer (A) by irradiation with recording laser light.

16. The optical recording medium according to claim 1, wherein said recording layer (A) contains at least one kind of organic compound that, upon irradiation with a laser light with a recording/playback wavelength of $\lambda 0$, exhibits a refraction index n2 and an extinction coefficient k2 after recording that are different from the refraction index n1 and

the extinction coefficient k1 of said recording layer (A) at λ0 before recording.

**17.** The optical recording medium according to claim 1, wherein the maximum absorption wavelength of the organic compound in solution is shifted to the shorter wavelength when it forms a thin film state of the recording layer.

**18.** A compound represented by general formula (5).

**19.** A quinazolin-4-one compound represented by general formula (6) having a disubstituted amino group at any of positions 5-8 in the quinazoline -4- ring:

[Formula 7]

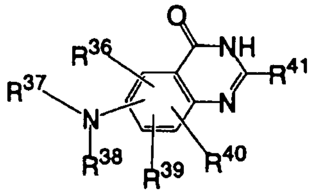

(6)

(wherein each of $R^{36}$-$R^{41}$ represents independently a hydrogen atom or a substituent).

**20.** A process for producing of the quinazolin-4-one compound represented by general formula (6) producing by reaction of a compound represented by the following general formula (7) and a compound represented by general formula (8) and/or general formula (9):

[Formula 8]

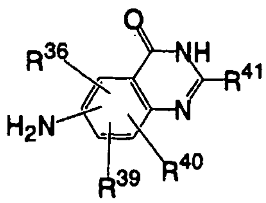

(7)

$R^{37}$-$Z^1$ (8)

$R^{38}$-$Z^2$ (9)

(wherein $R^{36}$-$R^{41}$ represent the same group as $R^{36}$-$R^{41}$ in formula (6); and $Z^1$ and $Z^2$ represent a leaving group).

**21.** A composition comprising at least one kind of compound represented by general formula (5).

**22.** An optical recording medium comprising a recording layer (A) capable of recording/playback with a laser light on a substrate,
wherein the recording layer (A) contains at least one kind of organic compound, wherein recording can be carried out by heat generated on irradiation of said recording layer (A) with recording laser light of 6 mW or lower and/or by the laser light without giving mechanical deformation to the substrate.

Fig.1

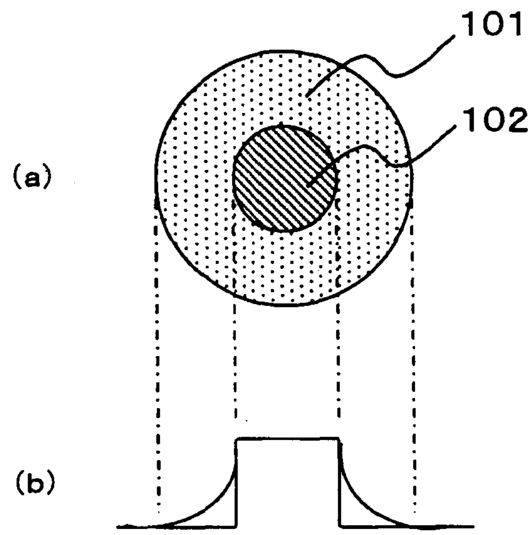


Fig.2

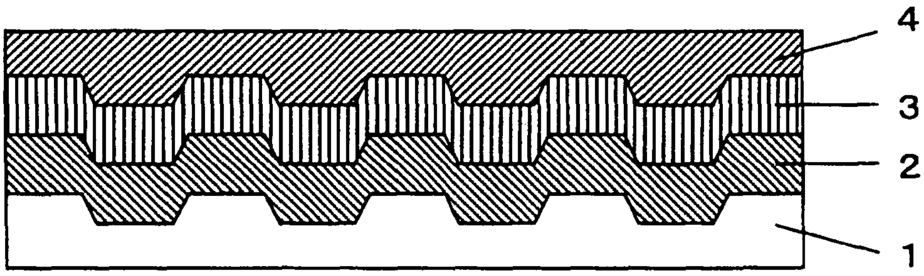


Fig.3

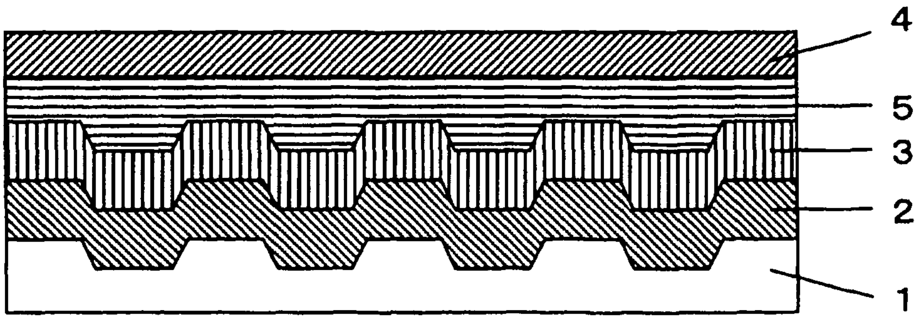

Fig.4

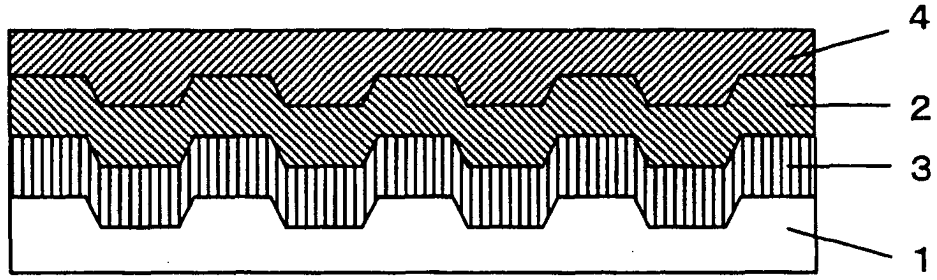


Fig.5

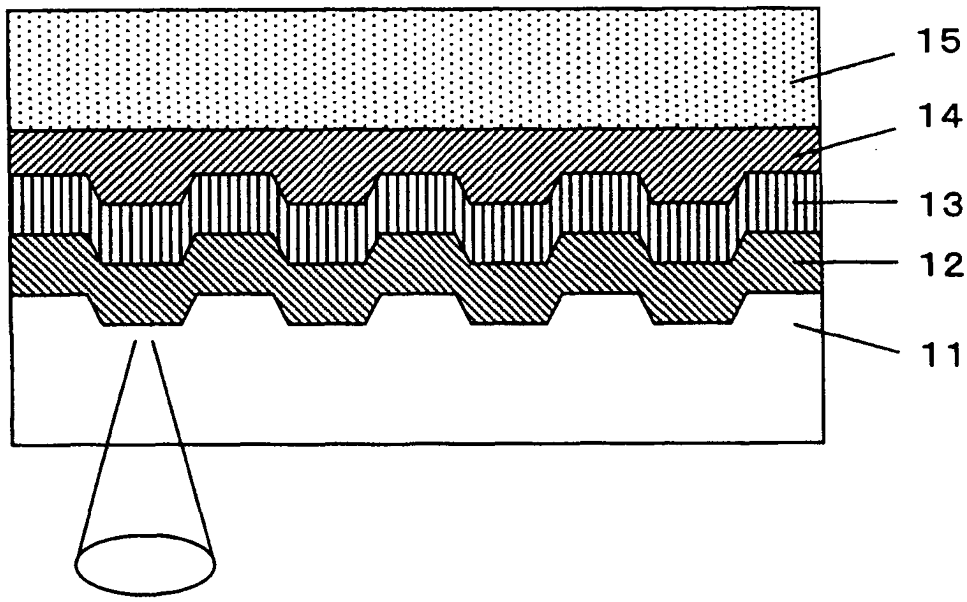

Fig.6

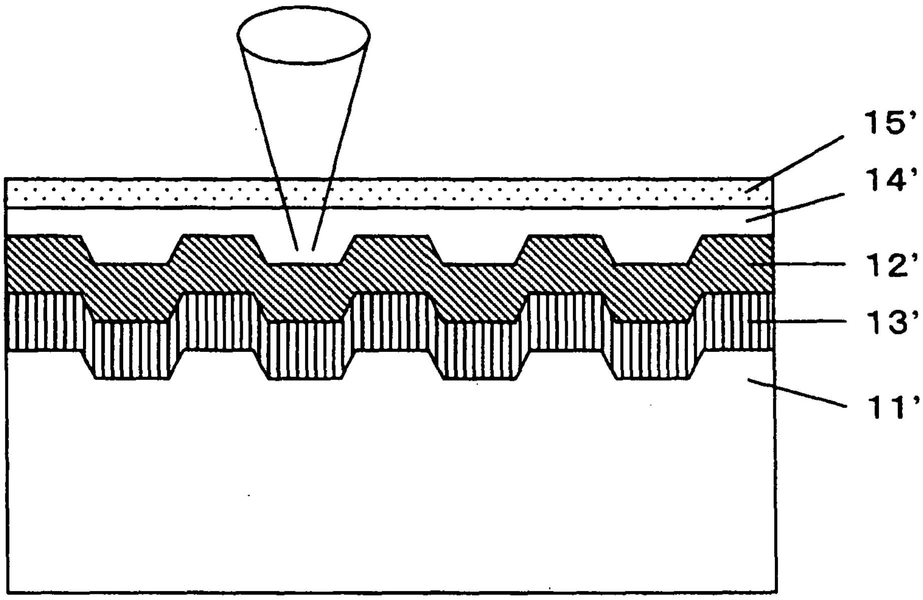


Fig.7

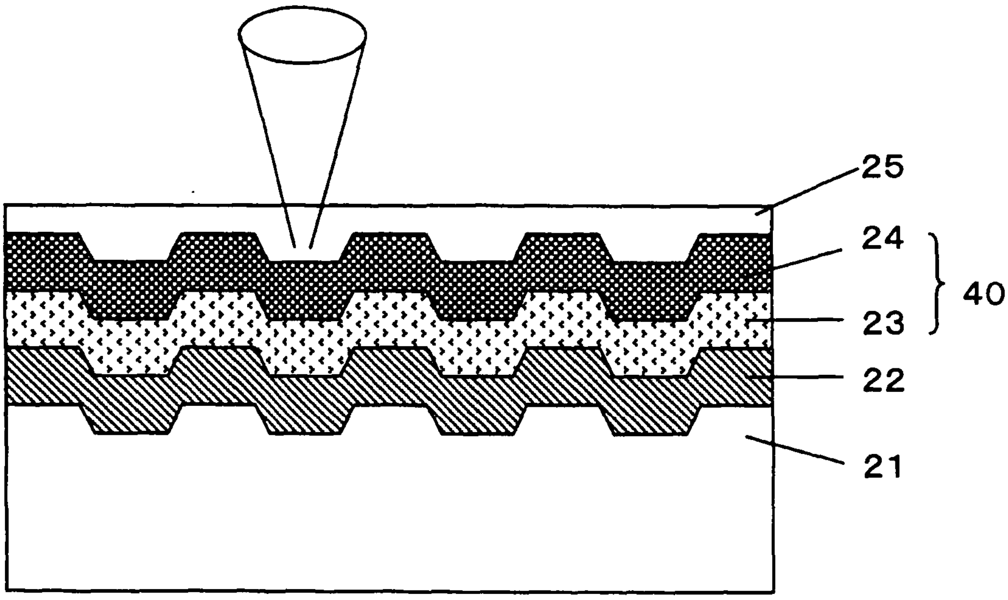

**INTERNATIONAL SEARCH REPORT**

International application No. PCT/JP2004/014969

A. CLASSIFICATION OF SUBJECT MATTER
Int.Cl[7] G11B7/24, B41M5/26

According to International Patent Classification (IPC) or to both national classification and IPC

B. FIELDS SEARCHED

Minimum documentation searched (classification system followed by classification symbols)
Int.Cl[7] G11B7/24, B41M5/264

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2004 |
| Kokai Jitsuyo Shinan Koho | 1971-2004 | Toroku Jitsuyo Shinan Koho | 1994-2004 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2002/74740 A (Kabushiki Kaisha Hayashibara Seibutsu Kagaku Kenkyujo), 15 March, 2002 (15.03.02), Full text; all drawings & US 2002-34605 A1 & EP 1191526 A2 & EP 1369861 A2 | 1-22 |

☐ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 21 December, 2004 (21.12.04) | 18 January, 2005 (18.01.05) |
| Name and mailing address of the ISA/ Japanese Patent Office | Authorized officer |
| Facsimile No. | Telephone No. |

Form PCT/ISA/210 (second sheet) (January 2004)